# EUROPEAN PATENT SPECIFICATION

(11) **EP 2 539 335 B1**
(45) Date of publication and mention of the grant of the patent: **21.12.2016**
(21) Application number: 11702823.3
(22) Date of filing: 03.02.2011
(51) Int. Cl.: C07D 413/12, C07D 413/14, C07D 453/04, A01N 43/82

(54) **PROCESS FOR THE PREPARATION OF ISOXAZOLINE DERIVATIVES**
VERFAHREN ZUR HERSTELLUNG VON ISOXAZOLIN-DERIVATEN
PROCÉDÉ DE PRÉPARATION DÉRIVÉS ISOXAZOLINES

(30) Priority: 12.10.2010 EP 10187269; 28.05.2010 EP 10164234; 28.05.2010 EP 10164231; 07.05.2010 GB 201007689; 25.02.2010 EP 10250337; 25.02.2010 EP 10250336
(43) Date of publication of application: 02.01.2013
(73) Proprietor: Syngenta Participations AG, 4058 Basel (CH); Syngenta Limited, Guildford, Surrey GU2 7YH (GB)
(72) Inventor: MULHOLLAND, Nicholas Phillip, Bracknell Berkshire RG42 6EY (GB); GODINEAU, Edouard, CH-4332 Stein (CH); CASSAYRE, Jérôme Yves, CH-4332 Stein (CH); RENOLD, Peter, CH-4332 Stein (CH); EL QACEMI, Myriem, CH-4332 Stein (CH); REVOL, Guillaume, CH-4332 Stein (CH)
(74) Representative: Syngenta International AG
(86) International application number: PCT/EP2011/051513
(87) International publication number: WO 2011/104089

(56) References cited:
- WO-A1-2009/063910
- WO-A1-2010/025998
- WO-A1-2011/067272
- WO-A2-2009/080250
- CLAUDIO GIOIA ET AL: "Organocatalytic Asymmetric Formal [3 + 2] Cycloaddition with in Situ-Generated N -Carbamoyl Nitrones", JOURNAL OF THE AMERICAN CHEMICAL SOCIETY, vol. 131, no. 28, 22 July 2009 (2009-07-22), pages 9614-9615, XP55003071, ISSN: 0002-7863, DOI: 10.1021/ja902458m
- KAZUTAKA MATOBA ET AL: "Enantioselective Synthesis of Trifluoromethyl-Substituted 2-Isoxazolines: Asymmetric Hydroxylamine/Enone Cascade Reaction", ANGEWANDTE CHEMIE INTERNATIONAL EDITION, vol. 49, no. 33, 2 August 2010 (2010-08-02), pages 5762-5766, XP55003070, ISSN: 1433-7851, DOI: 10.1002/anie.201002065
- OLIVIER MAHÉ ET AL: "Enantioselective Phase-Transfer Catalysis: Synthesis of Pyrazolines", ANGEWANDTE CHEMIE INTERNATIONAL EDITION, vol. 49, no. 39, 17 September 2010 (2010-09-17), pages 7072-7075, XP55003079, ISSN: 1433-7851, DOI: 10.1002/anie.201002485
- LYGO B ET AL: "A New Class of Asymmetric Phase-Transfer Catalysts Derived from Cinchona Alkaloids - Application in the Enantioselective Synthesis of alpha-Amino Acids", TETRAHEDRON LETTERS, ELSEVIER, AMSTERDAM, NL, vol. 38, no. 49, 8 December 1997 (1997-12-08), pages 8595-8598, XP004096008, ISSN: 0040-4039, DOI: DOI:10.1016/S0040-4039(97)10293-3
- STEFANO SANTORO ET AL: "Enantioselective organocatalytic substitution of [alpha]-cyanoacetates on imidoyl chlorides - synthesis of optically active ketimines", CHEMICAL COMMUNICATIONS, no. 48, 1 January 2007 (2007-01-01), page 5155, XP55003110, ISSN: 1359-7345, DOI: 10.1039/b715810f & "Supporting information", CHEMICAL COMMUNICATIONS, 2007, XP55003205, ISSN: 1359-7345

## Description

The present invention relates to the stereoselective synthesis of substituted isoxazolines and in particular to the stereoselective synthesis of substituted isoxazolines that have pesticidal activity.

WO2009/080250 discloses isoxazoline compounds that have insecticidal activity. The present invention provides a process for the stereoselective synthesis of isoxazoline compounds, such as those disclosed in WO2009/080250.

In first aspect, the invention provides a process for the preparation of a compound of formula (IB) 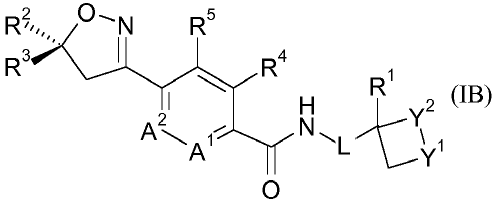 wherein
one of Y¹ and Y² is S, SO or SO₂ and the other is CH₂;
L is a direct bond or methylene;
A¹ and A² are C-H, or one of A¹ and A² is C-H and the other is N;
R¹ is hydrogen or methyl;
R² is chlorodifluoromethyl or trifluoromethyl;
R³ is 3,5-dibromo-phenyl, 3,5-dichloro-phenyl, 3,4-dichloro-phenyl, or 3,4,5-trichlorophenyl;
R⁴ is methyl;
R⁵ is hydrogen;
or R⁴ and R⁵ together form a bridging 1,3-butadiene group;
comprising reacting a compound of formula (II) 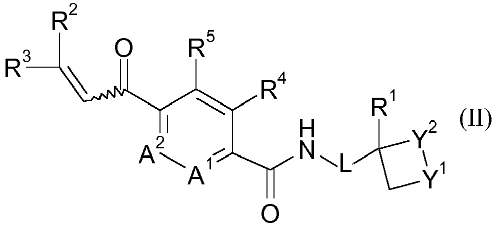 wherein Y¹, Y², L, A¹, A², R¹, R², R³, R⁴ and R⁵ are as defined for the compound of formula (IB);
with hydroxylamine in the presence of water, a base and a chiral phase transfer catalyst, which chiral phase transfer catalyst is a quinine derivative of formula (IIIa) 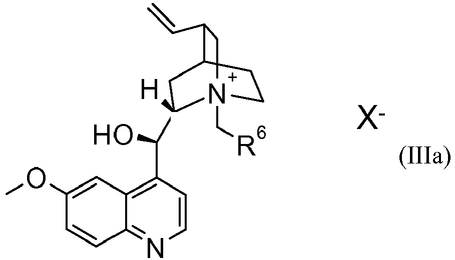 wherein X is an anion;
R⁶ is phenyl substituted by one to five R⁷, pyridyl or pyridyl substituted by one to four R⁷, pyrimidyl or pyrimidinyl substituted by one to three R⁷, or group A 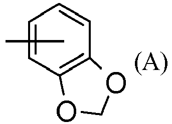 or group A substituted by one to four R⁷, and each R⁷ is independently halogen, nitro, C₁-C₄ alkyl or C₁-C₄ alkoxy.

Preferably the reaction is performed in an organic solvent.

It has been found that the enantiomer for formula IB is substantially more biologically active than the other enantiomer, e.g. it is substantially more effective at controlling invertebrate animal pests such as insects, acarines, nematodes and/or molluscs. The more biologically active enantiomer may be confirmed using the methodology described in the Examples. In some cases the less biologically active enantiomer has negligible biological activity, e.g. it provides no control of invertebrate animal pests. The present invention allows the more biologically active enantiomer to be selectively synthesised, thereby allowing production of enantiomerically enriched mixtures. This potentially allows reduced rates of application.

In enantiomerically enriched mixtures of the invention, the molar proportion of the compound of formula IB in the mixture compared to the total amount of both enantiomers is for example greater than 50%, e.g. at least 55, 60, 65, 70, 75, 80, 85, 90, 95, 96, 97, 98, or at least 99%.

In the compound of formula II the stereochemistry around the carbon-carbon double bond may be cis or trans. Both isomers lead to the desired stereochemistry of the compound of formula I. In one embodiment of the invention the compound of formula II is a compound of formula IIA 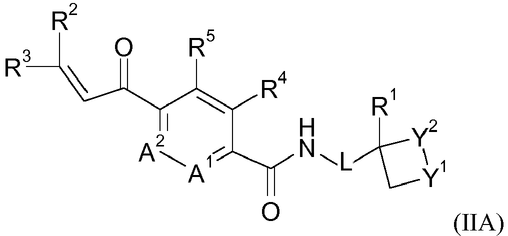 wherein Y¹, Y², L, A¹, A², R¹, R², R³, R⁴ and R⁵ are as defined for the compound of formula (IB).

Use of chiral phase transfer catalysts derived from quinine, such as compounds of formula III, may have other beneficial effects on the reaction in addition to providing stereoselectivity. For example, they may increase the rate of the reaction and/or allow the reaction to be run under milder conditions, for example at lower temperature.

Each alkyl moiety either alone or as part of a larger group (such as alkoxy, alkylcarbonyl, or alkoxycarbonyl) is a straight or branched chain and is, for example, methyl, ethyl, *n*-propyl, prop-2-yl, *n*-butyl, but-2-yl, 2-methyl-prop-1-yl or 2-methyl-prop-2-yl. The alkyl groups are preferably C₁ to C₆ alkyl groups, more preferably C₁-C₄ and most preferably C₁-C₃ alkyl groups.

Halogen is fluorine, chlorine, bromine or iodine.

Haloalkyl groups (either alone or as part of a larger group, such as haloalkoxy) are alkyl groups which are substituted by one or more of the same or different halogen atoms and are, for example, trifluoromethyl, chlorodifluoromethyl, 2,2,2-trifluoro-ethyl or 2,2-difluoro-ethyl.

In the context of the present specification the term "aryl" refers to a ring system which may be mono-, bi- or tricyclic. Examples of such rings include phenyl, naphthalenyl, anthracenyl, indenyl or phenanthrenyl. A preferred aryl group is phenyl.

The term "heteroaryl" refers to an aromatic ring system containing at least one heteroatom and consisting either of a single ring or of two or more fused rings. Preferably, single rings will contain up to three heteroatoms and bicyclic systems up to four heteroatoms which will preferably be chosen from nitrogen, oxygen and sulfur. Examples of monocyclic groups include pyridyl, pyridazinyl, pyrimidinyl, pyrazinyl, pyrrolyl, pyrazolyl, imidazolyl, triazolyl, tetrazolyl, furanyl, thiophenyl, oxazolyl, isoxazolyl, oxadiazolyl, thiazolyl, isothiazolyl, and thiadiazolyl. Examples of bicyclic groups include quinolinyl, cinnolinyl, quinoxalinyl, benzimidazolyl, benzothiophenyl, and benzothiadiazolyl. Monocyclic heteroaryl groups are preferred, pyridyl being most preferred.

Preferably compound of formula IIIa 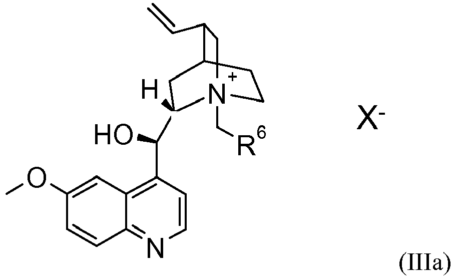 wherein R⁶ is phenyl substituted by one to five substituents independently selected from halogen, methyl and methoxy, or pyridyl or pyridyl substituted by one to four halogen atoms.

In yet another preferred group of compounds of formula III R⁶ is phenyl substituted by one to five substituents independently selected from fluorine, methyl and methoxy.

In yet another preferred group of compounds of formula III R⁶ is phenyl substituted by three to five substituents independently selected from fluorine, methyl and methoxy.

In yet another preferred group of compounds of formula III R⁶ is phenyl substituted by three to five methoxy groups.

In yet another preferred group of compounds of formula III R⁶ is phenyl substituted by three to five substituents independently selected from methyl and fluorine.

In yet another preferred group of compounds of formula III R⁶ is phenyl substituted by three to five substituents independently selected from methyl and fluorine, and no more than one substituent is methyl.

In yet another preferred group of compounds of formula III R⁶ is phenyl substituted by three to five fluorine atoms.

In yet another preferred group of compounds of formula III R⁶ is pyridyl or pyridyl substituted by two to four halogen atoms.

Most preferably the compound of formula IIIa is a compound of formula IIIB, IIIC, IIIE, IIIF, IIIG, IIIH, IIIJ, IIIK, IIIL, IIIM, IIIN and IIIO, wherein X is an anion, preferably a halogen anion, more preferably chloride or bromide.

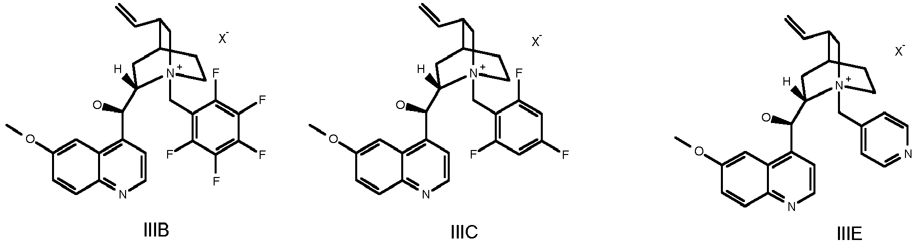

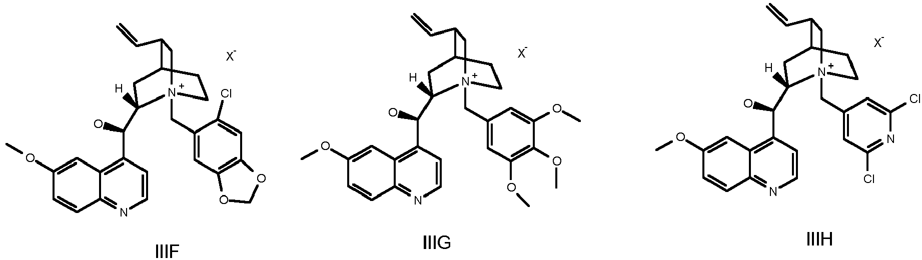

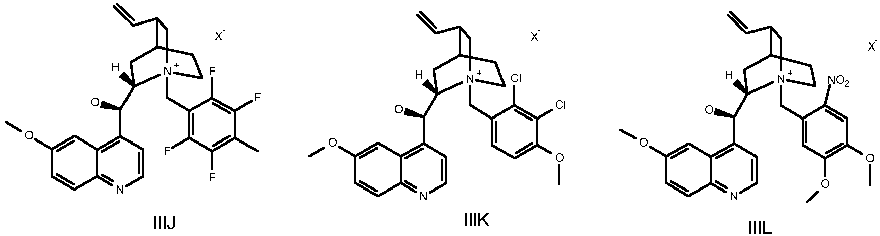

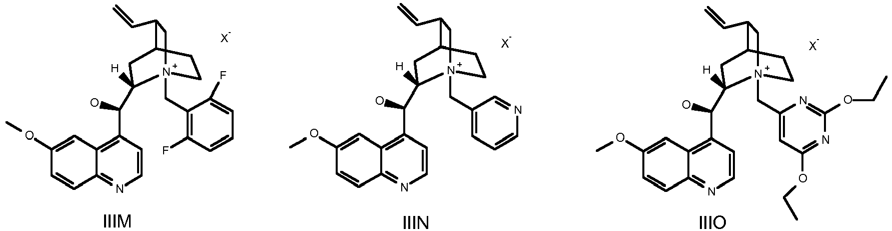

Compounds of formula IIIB, IIIG and IIIJ are preferred.

Some compounds of formula III are novel. Accordingly, in a further aspect the invention provides a compound of formula (III*) 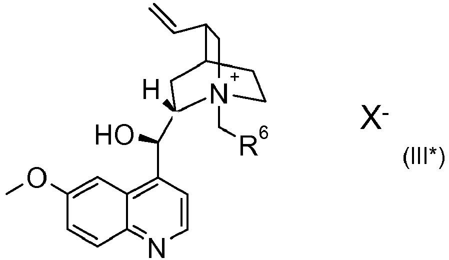 wherein R⁶.
is 2,4,6-trifluorophenyl, phenyl substituted by three to five groups independently selected from methyl and fluorine, providing that the phenyl is substituted by at least one methyl and one fluorine; or pyridyl or pyridyl substituted by two to four halogen, preferably phenyl substituted by methyl and two to four fluorine atoms.

The invention includes the dissociated cation and N-oxides of the compounds of formula (IIIa).

Preferably the compound of formula III* is a compound of formula IIIC, IIIE, IIIH or IIIJ more preferably a compound of formula IIIJ.

The preparation of a compound of formula (IIIa) 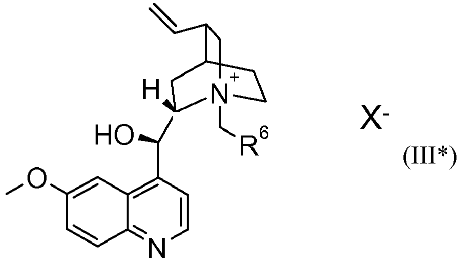 wherein R⁶
phenyl substituted by one to five R⁷, pyridyl or pyridyl substituted by one to four R⁷, pyrimidyl or pyrimidinyl substituted by one to three R⁷, or group A 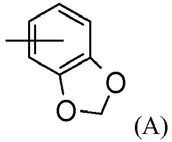 or group A substituted by one to four R⁷, and each R⁷ is independently halogen, nitro, C₁-C₄ alkyl or C₁-C₄ alkoxy. ;
comprising reacting a compound of formula IV 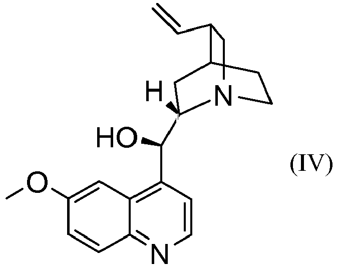 with a compound of formula V 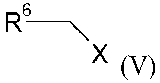 wherein R⁶ and X are as defined for the compound of formula (IIIa);
wherein the compound of formula (IIIa) is for use in a process for the preparation of the compound of formula (IB) or enriched mixtures thereof. Preferably said process includes the preparation of said compound or mixture.

Each substituent definition in each preferred group of compounds of formula I may be juxtaposed with any substituent definition in any other preferred group of compounds, in any combination. Preferred compounds of formula II correspond to the preferred compounds of formula I.

Preferred compounds of formula( IB) are shown in the Table below.

**Table A: Compounds of formula (Ia)**

| | | | | |
|---|---|---|---|---|
| 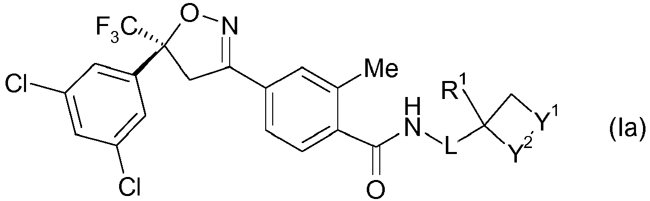 | | | | |

| Comp No. | L | R¹ | Y¹ | Y² |
|---|---|---|---|---|
| 1 | bond | CH₃ | S | CH₂ |
| 2 | bond | CH₃ | SO (cis) | CH₂ |
| 3 | bond | CH₃ | SO (trans) | CH₂ |
| 4 | bond | CH₃ | SO₂ | CH₂ |
| 5 | bond | H | S | CH₂ |
| 6 | bond | H | SO (cis) | CH₂ |
| 7 | bond | H | SO (trans) | CH₂ |
| 8 | bond | H | SO₂ | CH₂ |
| 9 | CH₂ | CH₃ | CH₂ | S |
| 10 | CH₂ | CH₃ | CH₂ | SO (cis) |
| 11 | CH₂ | CH₃ | CH₂ | SO (trans) |
| 12 | CH₂ | CH₃ | CH₂ | SO₂ |
| 13 | CH₂ | H | CH₂ | S |
| 14 | CH₂ | H | CH₂ | SO (cis) |
| 15 | CH₂ | H | CH₂ | SO (trans) |
| 16 | CH₂ | H | CH₂ | SO₂ |

Bearing in mind the stereocentre which is the subject of the invention, the invention otherwise includes all isomers of compounds of formula I (including formula IA and IB) and salts and N-oxides thereof, including enantiomers, diastereomers and tautomers. The more biologically active enantiomer (i.e. the compound of formula IB relative to the compound of formula IA) may be a mixture of any type of isomer of a compound of formula I, or may be substantially a single type of isomer. For example, where Y¹ or Y² is SO, the more biologically active enantiomer may be a mixture of the cis and trans isomer in any ratio, e.g. in a molar ratio of 1:99 to 99:1, e.g. 10:1 to 1:10, e.g. a substantially 50:50 molar ratio, e.g. in some cases the mixture may be cis enriched, in other cases the mixture may be trans enriched. For example, in trans enriched mixtures of the more biologically active enantiomer, e.g. when Y¹ or Y² is SO, the molar proportion of the trans compound in the mixture compared to the total amount of both cis and trans is for example greater than 50%, e.g. at least 55, 60, 65, 70, 75, 80, 85, 90, 95, 96, 97, 98, or at least 99%., e.g. mixtures may be at least 60% enantiomerically enriched for formula IB and at least 60, 70, 80, or at least 90% enriched for trans SO, e.g. at least 70% enantiomerically enriched for formula IB and at least 60, 70, 80, or at least 90% enriched for trans SO, e.g. at least 80% enantiomerically enriched for formula IB and at least 60, 70, 80, or at least 90% enriched for trans SO, e.g. at least 90% enantiomerically enriched for formula IB and at least 60, 70, 80, or at least 90% enriched for trans SO. Likewise, in cis enriched mixtures (preferred) of the more biologically active enantiomer, e.g. when Y¹ or Y² is SO, the molar proportion of the cis compound in the mixture compared to the total amount of both cis and trans is for example greater than 50%, e.g. at least 55, 60, 65, 70, 75, 80, 85, 90, 95, 96, 97, 98, or at least 99%, e.g. at least 60% enantiomerically enriched for formula IB and at least 60, 70, 80, or at least 90% enriched for cis SO, e.g. at least 70% enantiomerically enriched for formula IB and at least 60, 70, 80, or at least 90% enriched for cis SO, e.g. at least 80% enantiomerically enriched for formula IB and at least 60, 70, 80, or at least 90% enriched for cis SO, e.g. at least 90% enantiomerically enriched for formula IB and at least 60, 70, 80, or at least 90% enriched for cis SO). Y¹ or Y² is SO for compounds 2, 3, 6, 7, 10, 11, 14 and 15 in Table A.

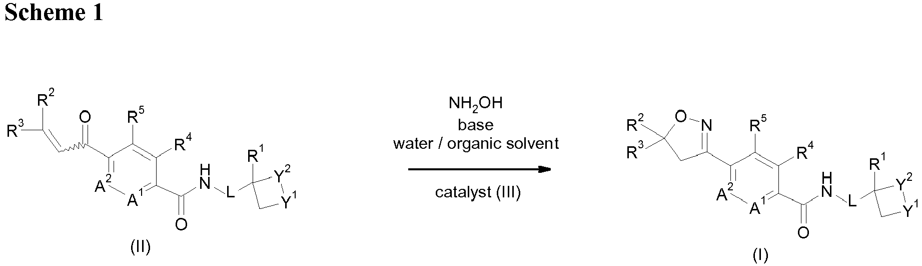

Enantioenriched compounds of formula (I) can be prepared by reacting a compound of formula (II) with hydroxylamine in the presence of a chiral phase transfer catalyst derived from quinine in a mixture of water and an organic solvent, as shown in **Scheme 1**. In some cases it should be possible to perform the reaction in the absence of an organic solvent, e.g. when the compound of formula II forms an immiscible liquid phase with water. However such reactions are preferably carried out in a suitable organic solvent, for example dichloromethane, 1,2-dichloroethane, toluene, preferably 1,2-dichloroethane at a temperature of between -78°C to 60°C, preferably between -20°C and +20°C, and at a dilution of e.g. between 0.1 M to 1 M. The reaction time is usually between 30 minutes and 48 hours, preferably between 1 and 4 hours. The amount of catalyst is usually between 0.1 and 0.4 molar equivalents, preferably between 0.1 and 0.2 molar equivalents. The amount of hydroxylamine is usually between 1 and 20 equivalents, preferably between 2 and 6 equivalents. Such reactions are usually carried out in the presence of a base. Suitable bases include alkali hydroxides such as lithium hydroxide, sodium hydroxide or potassium hydroxide, preferably sodium hydroxide, in usual amounts of between 1 and 10 equivalents, although sub-stochiometric amounts can be used. Preferably the amount of base used is between 2 and 6 equivalents.

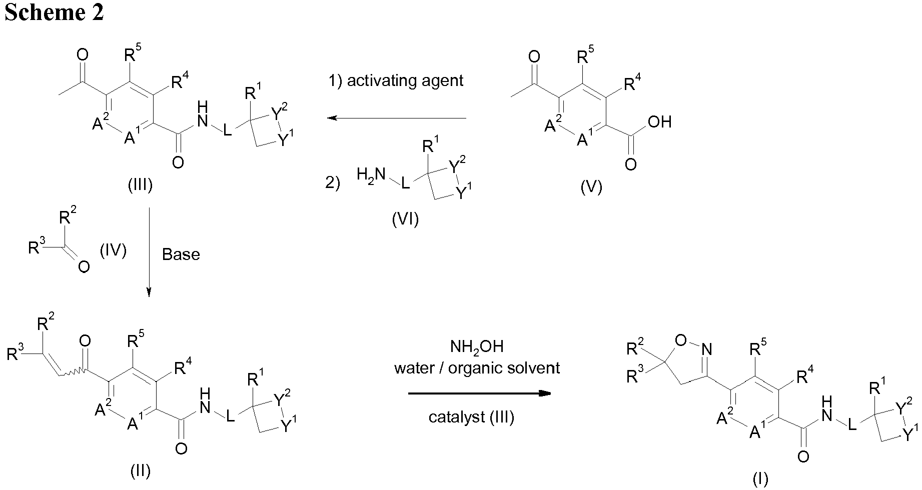

The compound of formula II may be prepared according to scheme 2, e.g. as described in WO2009/080250.

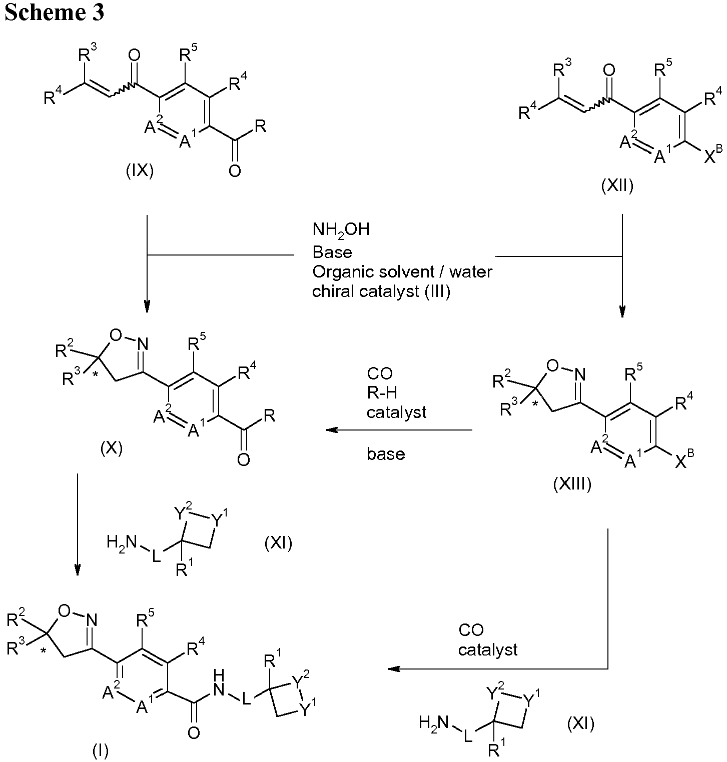

Alternatively, the more biologically active enantiomer of the compound of formula I can be prepared selectively from compounds of formula (IX) wherein R is OH or C₁-C₆alkoxy and (XII) wherein X^{B} is a leaving group, for example a halogen, such as bromo,. using an asymmetric reaction similar to that used for conversion of (II) to the more biologically active enantiomer of (I) as shown on **Scheme 3.** In that case, compounds of formula (X) and (XIII) are obtained enantioselectively and can be converted to compounds of formula (I) using the following methods:
1) Compounds of formula (I) wherein G¹ is oxygen, can be prepared by reacting a compound of formula (X) wherein R is OH, C₁-C₆alkoxy or Cl, F or Br, with an amine of formula (XI) as shown in **Scheme 3.** When R is OH such reactions are usually carried out in the presence of a coupling reagent, such as N,N'-dicyclohexylcarbodiimide ("DCC"), 1-ethyl-3-(3-dimethylamino-propyl)carbodiimide hydrochloride ("EDC") or bis(2-oxo-3-oxazolidinyl)phosphonic chloride ("BOP-Cl"), in the presence of a base, and optionally in the presence of a nucleophilic catalyst, such as hydroxybenzotriazole ("HOBT"). When R is Cl, such reactions are usually carried out in the presence of a base, and optionally in the presence of a nucleophilic catalyst. Alternatively, it is possible to conduct the reaction in a biphasic system comprising an organic solvent, preferably ethyl acetate, and an aqueous solvent, preferably a solution of sodium hydrogen carbonate. When R is C₁-C₆alkoxy it is sometimes possible to convert the ester directly to the amide by heating the ester and amine together in a thermal process. Suitable bases include pyridine, triethylamine, 4-(dimethylamino)-pyridine ("DMAP") or diisopropylethylamine (Hunig's base). Preferred solvents are *N*,*N*-dimethylacetamide, tetrahydrofuran, dioxane, 1,2-dimethoxyethane, ethyl acetate and toluene. The reaction is carried out at a temperature of from 0°C to 100°C, preferably from 15°C to 30°C, in particular at ambient temperature. Amines of formula (XII) are known in the literature or can be prepared using methods known to a person skilled in the art.
2) Acid halides of formula (X), wherein R is Cl, F or Br, may be made from carboxylic acids of formula (X), wherein R is OH, under standard conditions, such as treatment with thionyl chloride or oxalyl chloride. A preferred solvent is dichloromethane. The reaction is carried out at a temperature of from 0°C to 100°C, preferably from 15°C to 30°C, in particular at ambient temperature.
3) Carboxylic acids of formula (X), wherein R is OH, may be formed from esters of formula (X), wherein G¹ is oxygen and R is C₁-C₆alkoxy. It is known to a person skilled in the art that there are many methods for the hydrolysis of such esters depending on the nature of the alkoxy group. One widely used method to achieve such a transformation is the treatment of the ester with an alkali hydroxide, such as lithium hydroxide, sodium hydroxide or potassium hydroxide, in a solvent, such as ethanol or tetrahydrofuran, in the presence of water. Another is the treatment of the ester with an acid, such as trifluoroacetic acid, in a solvent, such as dichloromethane, followed by addition of water. The reaction is carried out at a temperature of from 0°C to 150°C, preferably from 15°C to 100°C, in particular at 50°C.
4) Compounds of formula (X) wherein R is C₁-C₆alkoxy, can be prepared by reacting a compound of formula (XII) wherein X^{B} is a leaving group, for example a halogen, such as bromo, with carbon monoxide and an alcohol of formula R-OH, such as ethanol, in the presence of a catalyst, such as bis(triphenylphosphine)palladium(II) dichloride, and a base, such as pyridine, triethylamine, 4-(dimethylamino)-pyridine ("DMAP") or diisopropylethylamine (Hunig's base). The reaction is carried out at a temperature of from 50°C to 200°C, preferably from 100°C to 150°C, in particular at 115°C. The reaction is carried out at a pressure of from 50 to 200 bar, preferably from 100 to 150 bar, in particular at 120 bar.
5) Alternatively, compounds of formula (I) can be prepared by reacting a compound of formula (XIII) wherein X^{B} is a leaving group, for example a halogen, such as bromo, with carbon monoxide and an amine of formula (XI), in the presence of a catalyst, such as palladium(II) acetate or bis(triphenylphosphine)palladium(II) dichloride, optionally in the presence of a ligand, such as triphenylphosphine, and a base, such as sodium carbonate, pyridine, triethylamine, 4-(dimethylamino)-pyridine ("DMAP") or diisopropylethylamine (Hunig's base), in a solvent, such as water, *N*,*N*-dimethylformamide or tetrahydrofuran. The reaction is carried out at a temperature of from 50°C to 200°C, preferably from 100°C to 150°C. The reaction is carried out at a pressure of from 50 to 200 bar, preferably from 100 to 150 bar.

The biologically active enantiomers of compounds of formula (IB) and enantiomerically enriched mixtures thereof can be used to combat and control infestations of insect pests such as Lepidoptera, Diptera, Hemiptera, Thysanoptera, Orthoptera, Dictyoptera, Coleoptera, Siphonaptera, Hymenoptera and Isoptera and also other invertebrate pests, for example, acarine, nematode and mollusc pests. Insects, acarines, nematodes and molluscs are hereinafter collectively referred to as pests. The pests which may be combated and controlled by the use of the invention compounds include those pests associated with agriculture (which term includes the growing of crops for food and fiber products), horticulture and forestry and the storage of products of vegetable origin (such as fruit, grain and timber); those pests associated with the damage of man-made structures and the transmission of diseases of man and animals; and also nuisance pests (such as flies).

### Examples

The following Examples illustrate, but do not limit, the invention.

The following abbreviations were used in this section: DCE=1,2-dichloroethane , s = singlet; bs = broad singlet; d = doublet; dd = double doublet; dt = double triplet; t = triplet, tt = triple triplet, q = quartet, sept = septet; m = multiplet; Me = methyl; Et = ethyl; Pr = propyl; Bu = butyl; M.p. = melting point; RT = retention time, [M+H]⁺ = molecular mass of the molecular cation, [M-H]⁻ = molecular mass of the molecular anion, ee = enantiomeric excess.

The following LC-MS methods were used to characterize the compounds:

**Method A**

| | | | | |
|---|---|---|---|---|
| MS | ZQ Mass Spectrometer from Waters (single quadrupole mass spectrometer), ionization method: electrospray, polarity: positive ionization, capillary (kV) 3.00, cone (V) 30.00, source temperature (°C) 100, desolvation temperature (°C) 250, cone gas flow (L/Hr) 50, desolvation gas flow (L/Hr) 400, mass range: 150 to 1000 Da. | | | |
| LC | HP 1100 HPLC from Agilent: solvent degasser, quaternary pump, heated column compartment and diode-array detector. | | | |
| | Column: Phenomenex Gemini C18, length (mm) 30, internal diameter (mm) 3, particle size (µm) 3, temperature (°C) 60, DAD wavelength range (nm): 200 to 500, solvent gradient: A = 0.05% v/v formic acid in water and B = 0.04% v/v formic acid in acetonitrile / methanol (4:1). | | | |

| | Time (min) | A% | B% | Flow (ml/min) |
|---|---|---|---|---|
| | 0.0 | 95 | 5.0 | 1.7 |
| | 2.0 | 0.0 | 100 | 1.7 |
| | 2.8 | 0.0 | 100 | 1.7 |
| | 2.9 | 95 | 5.0 | 1.7 |

**Method B**

| | | | | |
|---|---|---|---|---|
| MS | ZMD Mass Spectrometer from Waters (single quadrupole mass spectrometer), ionization method: electrospray, polarity: positive ionization, capillary (kV) 3.00, cone (V) 30.00, extractor (V) 3.00, source temperature (°C) 150, desolvation temperature (°C) 320, cone gas flow (L/Hr) 50, desolvation gas flow (L/Hr) 400, mass range: 150 to 800 Da. | | | |
| LC | Alliance 2795 LC HPLC from Waters: quaternary pump, heated column compartment and diode-array detector. | | | |
| | Column: Waters Atlantis dc18, length (mm) 20, internal diameter (mm) 3, particle size (µm) 3, temperature (°C) 40, DAD wavelength range (nm): 200 to 500, solvent gradient: A = 0.1 % v/v formic acid in water and B = 0.1 % v/v formic acid in acetonitrile. | | | |

| | Time (min) | A% | B% | Flow (ml/min) |
|---|---|---|---|---|
| | 0.0 | 80 | 20 | 1.7 |
| | 5.0 | 0.0 | 100 | 1.7 |
| | 5.6 | 0.0 | 100 | 1.7 |
| | 6.0 | 80 | 20 | 1.7 |

**Method C**

| | | | | |
|---|---|---|---|---|
| MS | ZQ Mass Spectrometer from Waters (single quadrupole mass spectrometer), ionization method: electrospray, polarity: positive ionization, capillary (kV) 3.00, cone (V) 30.00, extractor (V) 3.00, source temperature (°C) 100, desolvation temperature (°C) 200, cone gas flow (L/Hr) 200, desolvation gas flow (L/Hr) 250, mass range: 150 to 800 Da. | | | |
| LC | 1100er Series HPLC from Agilent: quaternary pump, heated column compartment and diode-array detector. | | | |
| | Column: Waters Atlantis dc18, length (mm) 20, internal diameter (mm) 3, particle size (µm) 3, temperature (°C) 40, DAD wavelength range (nm): 200 to 500, solvent gradient: A = 0.1 % v/v formic acid in water and B = 0.1 % v/v formic acid in acetonitrile. | | | |

| | Time (min) | A% | B% | Flow (ml/min) |
|---|---|---|---|---|
| | 0.0 | 90 | 10 | 1.7 |
| | 5.5 | 0.0 | 100 | 1.7 |
| | 5.8 | 0.0 | 100 | 1.7 |
| | 5.9 | 90 | 10 | 1.7 |

**Method D**

| | | | | |
|---|---|---|---|---|
| MS | ZMD Mass Spectrometer from Waters (single quadrupole mass spectrometer), ionization method: electrospray, polarity: positive ionization, capillary (kV) 3.00, cone (V) 30.00, extractor (V) 3.00, source temperature (°C) 150, desolvation temperature (°C) 320, cone gas flow (L/Hr) 50, desolvation gas flow (L/Hr) 400, mass range: 150 to 800 Da. | | | |
| LC | Alliance 2795 LC HPLC from Waters: quaternary pump, heated column compartment and diode-array detector. | | | |
| | Column: Waters Atlantis dc18, length (mm) 20, internal diameter (mm) 3, particle size (µm) 3, temperature (°C) 40, DAD wavelength range (nm): 200 to 500, solvent gradient: A = 0.1 % v/v formic acid in water and B = 0.1 % v/v formic acid in acetonitrile. | | | |

| | Time (min) | A% | B% | Flow (ml/min) |
|---|---|---|---|---|
| | 0.0 | 80 | 20 | 1.7 |
| | 2.5 | 0.0 | 100 | 1.7 |
| | 2.8 | 0.0 | 100 | 1.7 |
| | 2.9 | 80 | 20 | 1.7 |

**Method E**

| | | | | |
|---|---|---|---|---|
| MS | ZQ Mass Spectrometer from Waters (single quadrupole mass spectrometer), ionization method: electrospray, polarity: positive ionization, capillary (kV) 3.00, cone (V) 30.00, extractor (V) 3.00, source temperature (°C) 100, desolvation temperature (°C) 200, cone gas flow (L/Hr) 200, desolvation gas flow (L/Hr) 250, mass range: 150 to 800 Da. | | | |
| LC | 1100er Series HPLC from Agilent: quaternary pump, heated column compartment and diode-array detector. | | | |
| | Column: Waters Atlantis dc18, length (mm) 20, internal diameter (mm) 3, particle size (µm) 3, temperature (°C) 40, DAD wavelength range (nm): 200 to 500, solvent gradient: A = 0.1 % v/v formic acid in water and B = 0.1 % v/v formic acid in acetonitrile. | | | |

| | Time (min) | A% | B% | Flow (ml/min) |
|---|---|---|---|---|
| | 0.0 | 80 | 20 | 1.7 |
| | 2.5 | 0.0 | 100 | 1.7 |
| | 2.8 | 0.0 | 100 | 1.7 |
| | 2.9 | 80 | 20 | 1.7 |

**Method F**

| | | | | |
|---|---|---|---|---|
| MS | ZQ Mass Spectrometer from Waters (single quadrupole mass spectrometer), ionization method: electrospray, polarity: negative ionization, capillary (kV) 3.00, cone (V) 45.00, source temperature (°C) 100, desolvation temperature (°C) 250, cone gas flow (L/Hr) 50, desolvation gas flow (L/Hr) 400, mass range: 150 to 1000 Da. | | | |
| LC | HP 1100 HPLC from Agilent: solvent degasser, binary pump, heated column compartment and diode-array detector. | | | |
| | Column: Phenomenex Gemini C18, length (mm) 30, internal diameter (mm) 3, particle size (µm) 3, temperature (°C) 60, DAD wavelength range (nm): 200 to 500, solvent gradient: A = 0.05% v/v formic acid in water and B = 0.04% v/v formic acid in acetonitrile / methanol (4:1). | | | |

| | Time (min) | A% | B% | Flow (ml/min) |
|---|---|---|---|---|
| | 0.0 | 95 | 5.0 | 1.7 |
| | 2.0 | 0.0 | 100 | 1.7 |
| | 2.8 | 0.0 | 100 | 1.7 |
| | 2.9 | 95 | 5.0 | 1.7 |
| | 3.1 | 95 | 5 | 1.7 |

**Methode G**

| | | | | |
|---|---|---|---|---|
| MS | ZQ Mass Spectrometer from Waters (single quadrupole mass spectrometer), ionization method: electrospray, polarity: negative ionization, capillary (kV) 3.00, cone (V) 30.00, source temperature (°C) 100, desolvation temperature (°C) 250, cone gas flow (L/Hr) 50, desolvation gas flow (L/Hr) 400, mass range: 100 to 900 Da. | | | |
| LC | HP 1100 HPLC from Agilent: solvent degasser, quaternary pump (ZDQ), heated column compartment and diode-array detector. | | | |
| | Column: Phenomenex Gemini C18, length (mm) 30, internal diameter (mm) 3, particle size (µm) 3, temperature (°C) 60, DAD wavelength range (nm): 200 to 500, solvent gradient: A = 0.05% v/v formic acid in water and B = 0.04% v/v formic acid in acetonitrile / methanol (4:1). | | | |

| | Time (min) | A% | B% | Flow (ml/min) |
|---|---|---|---|---|
| | 0.0 | 95 | 5.0 | 1.7 |
| | 2.0 | 0.0 | 100 | 1.7 |
| | 2.8 | 0.0 | 100 | 1.7 |
| | 2.9 | 95 | 5.0 | 1.7 |
| | 3.0 | 95 | 5 | 1.7 |

**Method H**

| | | | | |
|---|---|---|---|---|
| MS | ZMD Mass Spectrometer from Waters (single quadrupole mass spectrometer), ionization method: electrospray, polarity: positive ionization, capillary (kV) 3.80, cone (V) 30.00, extractor (V) 3.00, source temperature (°C) 150, desolvation temperature (°C) 350, cone gas flow (L/Hr) OFF, desolvation gas flow (L/Hr) 600, mass range: 100 to 900 Da. | | | |
| LC | HP 1100 HPLC from Agilent: solvent degasser, binary pump, heated column compartment and diode-array detector. | | | |
| | Column: Phenomenex Gemini C18, length (mm) 30, internal diameter (mm) 3, particle size (µm) 3, temperature (°C) 60, DAD wavelength range (nm): 200 to 500, solvent gradient: A = 0.05% v/v formic acid in water and B = 0.04% v/v formic acid in | | | |
| | acetonitrile / methanol (4:1). | | | |

| | Time (min) | A% | B% | Flow (ml/min) |
|---|---|---|---|---|
| | 0.0 | 95 | 5.0 | 1.7 |
| | 2.0 | 0.0 | 100 | 1.7 |
| | 2.8 | 0.0 | 100 | 1.7 |
| | 2.9 | 95 | 5.0 | 1.7 |
| | 3.0 | 95 | 5 | 1.7 |

The following Chiral HPLC method was used to characterize the compounds:

**Method I**

| | |
|---|---|
| CHIRAL HPLC | Alliance 2695 HPLC from Waters: solvent degasser, binary pump, heated column compartment and diode-array detector |
| | Column: Chiralpak IC, length (mm) 250, internal diameter (mm) 4.6, particle size (µ) 5, wavelength (nm): 220 nm, temperature (°C) 30, solvent: Isocratic isopropyl alcohol: heptane 20:80, injection volume 25 uL, flow (ml/min) 1. |

**Method J**

| | |
|---|---|
| CHIRAL HPLC | Alliance 2695 HPLC from Waters: solvent degasser, binary pump, heated column compartment and diode-array detector |
| | Column: Chiralpak IC, length (mm) 250, internal diameter (mm) 4.6, particle size (µ) 5, wavelength (nm): 220 nm, temperature (°C) 30, solvent: Isocratic isopropyl alcohol: heptane: diethylamine 30:70:0.1, injection volume 25 uL, flow (ml/min) 1. |

### Example 1A: Catalyst preparation: Anthracenyl-methyl quininium chloride (not according to the invention)

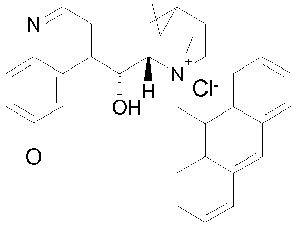

A solution of 9-chloromethyl-anthracene (0.91 g) and quinine (1 g) in toluene (7 ml) was heated at 90°C for 18 hours. The reaction mixture was filtered, washed with n-heptane. The solid was recrystallised from chloroform and n-heptane to afford the title product (1.69 g) as a yellow solid. M.p. 150-152°C (decomposed). LCMS (method H) 1.31 min, M⁺ 515; ¹H NMR (400 MHz, CDCl₃) 9.15 (d, 1H), 8.65 (d, 1H), 8.53 (d, 1H), 8.40 (s, 1H), 8.00 (m, 3H), 7.88 (d, 1H), 7.71 (s, 1H), 7.68 (t, 1H), 7.53 (t, 1H), 7.45 (m, 2H), 7.33 (dd, 1H), 7.11 (d, 1H), 7.02 (d, 1H), 6.22 (d, 1H), 5.51 (m, 1H), 5.15 (m, 1H), 4.98 (m, 2H), 4.38 (m, 1H), 3.98 (s, 3H), 3.48 (m, 1H), 2.90 (m, 1H), 2.63 (t, 1H), 2.20 (m, 2H), 1.85 (m, 2H), 1.45 (m, 2H).

### Example 1B: Catalyst preparation: Anthracenyl-methyl dihydroquininium chloride (not according to the invention)

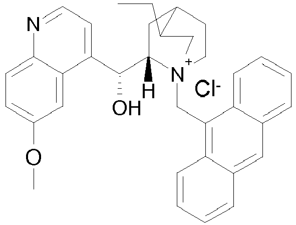

A solution of 9-chloromethylanthracenyl (0.45 g) and hydroquinine (0.5 g) in toluene (9 ml) was heated at 80°C for 18 hours. The reaction mixture was poured in diethyl ether and then filtrated to afford the title product as a yellow solid (0.60 g). ¹H NMR (400 MHz, CDCl₃) 9.33 (d, 1H), 8.72 (d, 1H), 8.54 (bs, 1H), 8.34 (d 1H), 8.03-8.09 (m, 2H), 7.95-8.01 (m, 3H), 7.75-7.80 (m, 1H), 7.65 (d, 1H), 7.56-7.62 (m, 1H), 7.47-7.54 (m, 2H), 7.37-7.41 (m, 1H), 7.08-7.19 (m, 2H), 5.93 (d, 1H), 5.23-5.32 (m, 1H), 4.15 (t, 1H), 3.97 (s, 3H), 2.90-2.99 (m, 2H), 2.67 (t, 1H), 2.29-2.39 (m, 2H), 1.87 (bs, 1H), 1.33-1.50 (m, 3H), 1.10-1.19 (m, 2H), 0.55 (t, 3H).

### Example 2: Catalyst preparation: 2,4,6-pentafluorophenyl-methyl quininium bromide

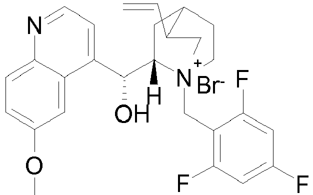

A solution of 1-bromomethyl-2,4,6-pentafluorobenzene (0.45 g) and quinine (0.5 g) in toluene (9 ml) was heated at 80°C for 18 hours. The reaction mixture was poured in diethyl ether and then filtrate to afford the title product as a white solid (0.80 g). LCMS (method H) 1.02 min, M⁺ 469; ¹H NMR (400 MHz, DMSO-*d6*) 8.82 (d, 1H), 8.05 (d, 1H), 7.78 (d, 1H), 7.54 (q, 2H), 7.41 (m, 1H), 7.21 (m, 1H), 6.79 (m, 1H), 6.57 (m, 1H), 5.77 (m, 1H), 5.42 (d, 1H), 5.09 (d, 1H), 5.04 (d, 1H), 4.63 (d, 1H), 4.18 (m, 2H), 3.98 (s, 3H), 3.59 (m, 2H), 2.71 (m, 1H), 2.20 (m, 2H), 2.05 (m, 1H), 1.87 (m, 1H), 1.42 (m, 1H).

### Example 3: Catalyst preparation: 2,3,4,5,6-pentafluorophenyl-methyl quininium bromide

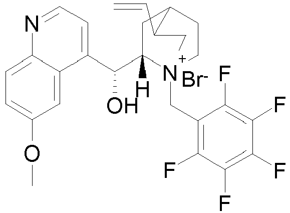

A solution of 1-bromomethyl-2,3,4,5,6-pentafluorobenzene (0.52 g) and quinine (0.5 g) in toluene (9 ml) was heated at 80°C for 18 hours. The reaction mixture was poured in diethyl ether and then filtrate to afford the title product as a white solid (0.90g). M.p. 162-165°C (decomposed). LCMS (method G) 1.08 min, M⁺ 505; ¹H NMR (400 MHz, CDCl₃) 8.78 (d, 1H), 8.05 (d, 1H), 7.78 (d, 1H), 7.39 (dd, 1H), 7.18 (d, 1H), 6.73 (m, 1H), 6.41 (d, 1H), 6.09 (d, 1H), 5.50 (m, 1H), 5.04(d, 1H), 4.98 (d, 1H), 4.70 (m, 1H), 4.63 (d, 1H), 3.98 (s, 3H),3.97 (m, 1H), 3.74 (m, 2H), 3.10 (m, 1H), 2.81 (m, 1H), 2.30 (m, 2H), 2.05 (m, 2H), 1.41 (m, 1H). ¹⁹F NMR (376 MHz, CDCl₃)-132.67 (s, 1F), -146.60(s, 2F), -158.28(s, 2F).

### Example 4: Catalyst preparation: 2,3,4,5,6-pentafluorophenyl-methyl quininium chloride

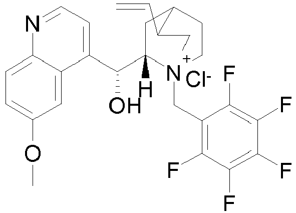

A solution of 1-chloromethyl-2,3,4,5,6-pentafluorobenzene (0.42 g) and quinine (0.50 g) in toluene (9 ml) was heated at 80°C for 18 hours. The reaction mixture was poured in diethyl ether and then filtrate to afford the title product as a pale yellow solid (0.3g). LCMS (method G) 1.10 min, M⁺ 505; ¹H NMR (400 MHz, CDCl₃) 8.78 (d, 1H), 7.95 (d, 1H), 7.78 (d, 1H), 7.31 (dd, 1H), 7.21 (d, 1H), 6.75 (s, 1H), 6.25 (d, 1H), 5.50 (m, 1H), 5.00(m, 2H), 4.88 (d, 1H), 3.98 (s, 3H). ¹⁹F NMR (376 MHz, CDCl₃) -132.67 (s, 1F), -146.60(s, 2F), -158.28(s, 2F).

### Example 5: Catalyst preparation: 6-chloropiperonyl quininium chloride

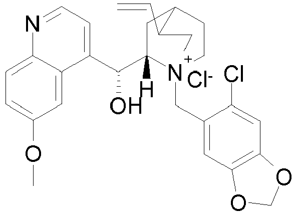

A solution of 6-chloropiperonyl chloride (0.41 g) and quinine (0.50 g) in toluene (9 ml) was heated at 80°C for 18 hours. The reaction mixture was poured in diethyl ether and then filtrate to afford the title product as a pale yellow solid (0.75 g). LCMS (method H) 1.12 min, M⁺ 494; ¹H NMR (400 MHz, CDCl₃) 8.83 (d, 1H), 8.11 (d, 1H), 7.86 (m, 2H), 7.41 (m, 2H), 7.09 (d, 1H), 6.98 (s, 1H), 6.72 (m, 2H), 6.10 (s, 2H), 5.61 (m, 1H), 5.12 (d, 1H), 5.05 (d, 1H), 4.62 (d, 1H), 3.98 (s, 3H), 3.67 (m, 1H), 3.38 (dd, 1H), 3.19 (m, 2H), 2.58 (m, 2H), 2.33 (m, 1H), 2.10 (m, 1H), 1.83 (m, 1H), 1.48 (m, 1H).

### Example 6: Catalyst preparation: 3,4,5-trimethoxybenzyl quininium chloride

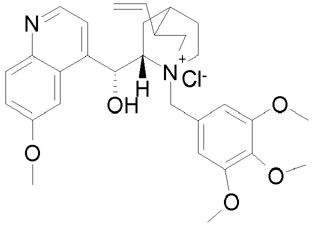

A solution of 3,4,5-trimethoxybenzyl chloride (0.42 g) and quinine (0.50 g) in toluene (9 ml) was heated at 80°C for 18 hours. The reaction mixture was poured in diethyl ether and then filtrate to afford the title product as a white solid (0.848g). LCMS (method G) 1.06 min, M⁺ 505; ¹H NMR (400 MHz, CDCl₃) 8.75 (d, 1H), 8.05 (d, 1H), 7.71 (d, 1H), 7.37 (m, 2H), 7.08 (s, 2H), 6.67 (d, 1H), 6.11 (d, 1H), 5.61 (m, 1H), 5.12 (m, 2H), 4.61 (d, 1H), 3.98 (s, 3H), 3.87 (s, 3H), 3.81 (s, 6H), 3.70 (t, 1H), 3.15 (m, 2H), 2.65 (m, 1H), 2.37 (m, 2H), 2.09 (m, 1H), 1.79 (m, 3H), 1.58 (t, 1H).

### Example 7: Catalyst preparation: 4-methylpyridine quininium chloride

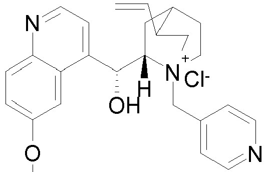

A solution of 4-chloromethyl-pyridine hydrochloride (0.41 g) and potassium bicarbonate (0.22 g) in toluene (9 ml) was stirred for 1 hour. Then quinine (0.50 g) was added to the suspension and the reaction mixture was heated at 80°C for 18 hours. The reaction mixture was poured in a mixture of water and diethyl ether, then filtrate, dried *in vacuo* at 35°C to afford the title product as a red solid (0.30 g). LCMS (method G) 0.76 min, M⁺ 516; ¹H NMR (400 MHz, CDCl₃) 8.71 (d, 1H), 8.65 (d, 2H), 7.95 (d, 1H), 7.74 (m, 3H), 7.29 (m, 1H), 6.58 (m, 1H), 6.28 (d, 1H), 5.55 (m, 1H), 5.10 (m, 3H), 3.94 (s, 3H), 3.54 (m, 1H), 3.38 (m, 2H), 3.00 (m, 2H), 2.61 (m, 1H), 2.31 (m, 1H), 2.05 (m, 2H), 1.74 (m, 2H), 1.41 (m, 1H).

### Example 8: Catalyst preparation: 2,6-dichloro-4-methyl-pyridine quininium chloride

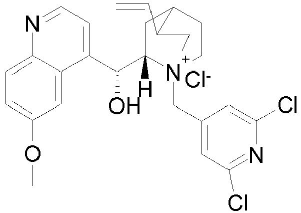

A solution of 2,6-dichloro-4-chloromethyl-pyridine (0.14 g) and quinine (0.18 g) in toluene (3 ml) was heated at 80°C for 18 hours. The reaction mixture was poured in diethyl ether and then filtrate to afford the title product as a white solid (0.10 g). LCMS (method G) 1.07 min, M⁺ 485; ¹H NMR (400 MHz, DMSO-*d6*) 8.81 (d, 1H), 8.05 (s, 2H), 8.03 (d, 1H), 7.73 (d, 1H), 7.48 (dd, 1H), 7.21 (d, 1H), 6.48 (s, 1H), 5.75 (m, 1H), 5.58 (m, 1H), 5.15 (d, 1H), 5.03 (d, 1H), 4.72 (d, 1H), 4.42 (m, 1H), 4.01 (s, 3H), 3.78 (m, 1H), 3.68 (m, 1H), 3.51 (t, 1H), 3.27 (m, 1H), 2.63 (m, 1H), 2.18 (m, 2H), 2.05 (m, 1H), 1.78 (m, 1H), 1.45 (m, 1H).

### Example 9: Chiral separation

### 4-[5-(3,5-Dichloro-phenyl)-5-trifluoromethyl-4,5-dihydro-isoxazol-3-yl]-2-methyl-N-thietan-3-yl-benzamide

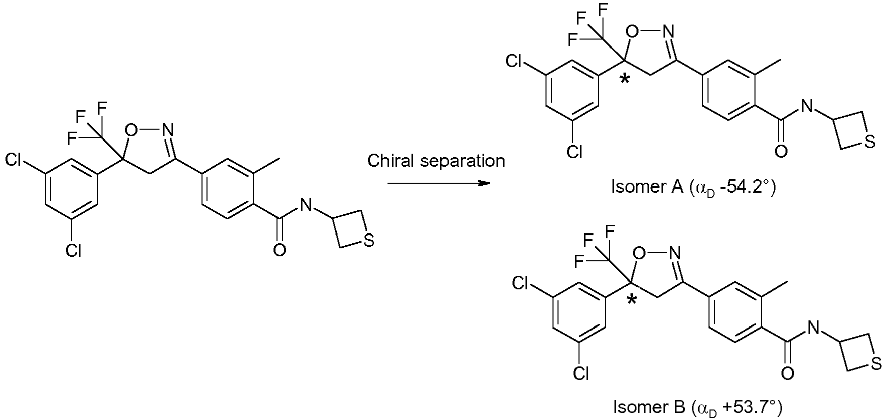

4-[5-(3,5-Dichloro-phenyl)-5-trifluoromethyl-4,5-dihydro-isoxazol-3-yl]-2-methyl-N-thietan-3-yl-benzamide (0.97 g; prepared as described in WO09080250) was separated through chiral phase preparative HPLC (Column: CHIRALPAK® IC 5 µm; Mobile Phase: 90/10 Carbon Dioxide/Ethanol; Flow Rate: 120 ml/min; Detection: 230 nm; Temperature: 25°C; Outlet Pressure: 150 bars) to afford 0.38 g of 4-[5-(3,5-dichloro-phenyl)-5-trifluoromethyl-4,5-dihydro-isoxazol-3-yl]-2-methyl-N-thietan-3-yl-benzamide ISOMER A (α_{D} -54.2°), and 0.35 g 4-[5-(3,5-dichloro-phenyl)-5-trifluoromethyl-4,5-dihydro-isoxazol-3-yl]-2-methyl-N-thietan-3-yl-benzamide ISOMER B (α_{D} +53.7°).

Similarly 4-[5-(3,5-dichloro-phenyl)-5-trifluoromethyl-4,5-dihydro-isoxazol-3-yl]-2-methyl-N-(cis-1-oxide-thietan-3-yl)-benzamide (1.98 g; prepared as described in WO09080250) was resolved to afford 0.81 g of 4-[5-(3,5-dichloro-phenyl)-5-trifluoromethyl-4,5-dihydro-isoxazol-3-yl]-2-methyl-N-(cis-1-oxide-thietan-3-yl)-benzamide ISOMER A (α_{D} -46.2°) and 0.83 g of ISOMER B (α_{D} +48.4°). Similarly 4-[5-(3,5-dichloro-phenyl)-5-trifluoromethyl-4,5-dihydro-isoxazol-3-yl]-2-methyl-N-(1,1-dioxo-thietan-3-yl)-benzamide (0.94 g ; prepared as described in WO09080250) was resolved to afford 0.44 g of 4-[5-(3,5-dichloro-phenyl)-5-trifluoromethyl-4,5-dihydro-isoxazol-3-yl]-2-methyl-N-(1,1-dioxo-thietan-3-yl)-benzamide ISOMER A (α_{D} -50.2°); and 0.45g of ISOMER B (α_{D} +51.1°).

### Example 10: Intermediate preparation

### 4-[(E)-3-(3,5-dichloro-phenyl)-4,4,4-trifluoro-but-2-enoyl]-2-methyl-N-thietan-3-yl-benzamide

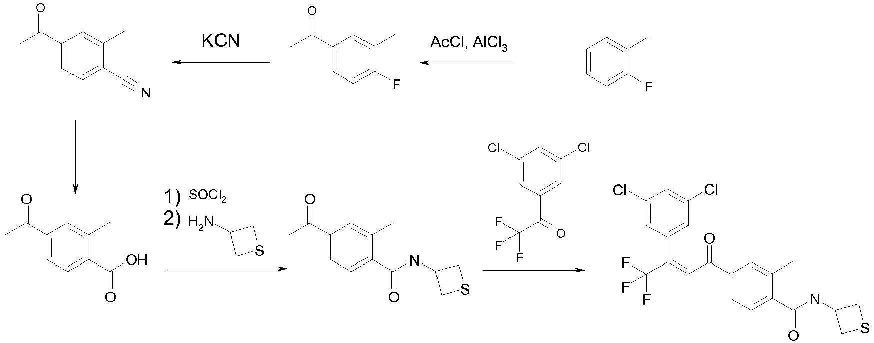

### Step A: 1-(4-Fluoro-3-methyl-phenyl)-ethanone

To a solution of 2-fluorotoluene (10.0 g, 90.8 mmol) and acetyl chloride (6.64 ml, 93.4 mmol) in dry CH₂Cl₂ (50 ml) was added portion-wise AlCl₃ (15.3 g, 115 mmol) (caution: vigorous exothermic reaction). The reaction was stirred at room temperature for 24 h. To complete the reaction, the mixture was heated to reflux for 2 h. The reaction mixture was quenched with saturated aqueous Na₂CO₃, diluted with Et₂O and filtered through a pad of Celite®. The organic phase was then separated, washed with saturated aqueous NaCl, dried over Na₂SO₄ and concentrated in vacuo. Purification by flash chromatography (SiO₂, Heptane: Ethylacetate 97:3 to 90:10) gave the title product as a pale brown oil (13.6 g). ¹H NMR (400 MHz, CDCl₃) 7.83 (d, 1H), 7.77-7.81 (m, 1H), 7.06 (t, 1H), 2.58 (s, 3H), 2.32 (d, 3H).

### Step B: 1-(4-Cyano-3-methyl-phenyl)-ethanone

1-(4-Fluoro-3-methyl-phenyl)-ethanone (2.02 g, 13.3 mmol) was dissolved in DMSO (4.5 ml) and then NaCN (0.81g, 16.6 mmol) was added. The resulting mixture was heated for 12h at 120 °C. The cooled reaction mixture was then diluted with CH₂Cl₂ and washed with water. The water phase was back-extracted with CH₂Cl₂ (3x). The combined organics were washed with H₂O (3x), saturated aqueous NaCl (1x), dried over Na₂SO₄, filtrated and concentrated under reduced pressure to afford an orange oil (2.1 g). ¹H NMR (400 MHz, CDCl₃): 7.89 (s, 1H), 7.83 (d, 1H), 7.71 (d, 1H), 2.63 (s, 3H), 2.62 (s, 3H).

### Step C: 4-Acetyl-2-methyl-benzoic acid

A pale yellow solution of 1-(4-Cyano-3-methyl-phenyl)-ethanone (4.54 g, 28.5 mmol) in AcOH (23 ml), H₂O (23 ml) and concentrated H₂SO₄ (23 ml) was refluxed for 5 h and stirred at room temperature overnight. The resulting orange slurry was adjusted to pH≈10 with 175ml NaOH 30%. The resulting orange solution was washed with AcOEt (2x250mL). The aqueous layer was acidified with concentrated HCI (100 ml) to CH=1 and then extracted with AcOEt (2x 200 ml). The organic phase was washed with H₂O (1x200mL), dried over Na₂SO₄, filtrated and evaporated under reduced pressure to give an orange solid (4.94g). The orange solid was dissolved in CH₂Cl₂ (150 ml) and water (100 ml) followed by 30% NaOH (50 ml) were successively added. The water phase was washed with CH₂Cl₂ (2x50mL). To the aqueous phase HCl conc. (90 ml) was added. The resulting precipitate was then filtered off and washed with water. The residue was then dissolved in CH₂Cl₂. The organic phase was dried over Na₂SO₄, filtrated and concentrated under reduced pressure to give a pale orange solid (3.3 g). LCMS (method F) RT = 1.21min, [M+H⁺]= 179; ¹H NMR (400 MHz, DMSO-d₆): 13.2 (bs, 1H), 7.82-7.91 (m, 3H), 2.61 (s, 3H), 2.58 (s, 3H).

### Step D: 4-Acetyl-2-methyl-N-thietan-3-yl-benzamide

4-Acetyl-2-methyl-benzoic acid (70 g, 39.3 mmol) was dissolved in CH₂Cl₂ (700 ml) and oxalyl chloride (109 g, 86 mmol) DMF (1 mL) were successively added. The reaction mixture was stirred overnight at room temperature. The mixture was then concentrated in vacuo to afford a brown oil (82 g). The acid chloride was redissolved in CH₂Cl₂ (500 ml) and cooled to 0 °C. A solution of hydro thietane-3-yl amine trifluoroacetate (prepared as described in WO09080250, 92 g, 34 mmol) and Et₃N (200 g, 200 mmol) in CH₂Cl₂ (400mL) was then added dropwise. The resulting mixture was stirred overnight at room temperature. NaOH 10% was added. The mixture was washed successively with water and with 2N HCl (300 ml). The organic layer was dried over Na₂SO₄ and evaporated in vacuo to afford brown oil (132 g). Purification by silica gel chromatography (CH₂Cl₂: Ethyl acetate 2:1) afforded the title compound as a yellow solid (34 g). LCMS (method F) RT = 1.21min, [M+H⁺]= 250. ¹H NMR (400 MHz, CDCl₃): 7.82 (s, 1H), 7.79 (d, 1H), 7.43 (d, 1H), 6.21 (bs, 1H), 5.43 (q, 1H), 3.50 (t, 2H), 3.39 (t, 2H), 2.61 (s, 3H), 2.49 (s, 3H).

### Step E: 4-[(E)-3-(3,5-dichloro-phenyl)-4,4,4-trifluoro-but-2-enoyl]-2-methyl-N-thietan-3-yl-benzamide

4-Acetyl-2-methyl-N-thietan-3-yl-benzamide (0.25 g, 1 mmol), 3,5 dichloro 2,2,2 trifluoroacetophenone (0.24 g, 1 mmol) potassium carbonate (138 mg, 1 mmol) and triethylamine (10 mg, 0.1 mmol) were dissolved in 1,2 dichloroethane (2.5 ml). The resulting mixture was refluxed for 3h. The reaction mixture was cooled to room temperature and saturated aqueous NH₄Cl was added. The mixture was extracted with CH₂Cl₂ (3x50 ml). The combined organic extracts were dried over Na₂SO₄ and concentrated in vacuo to afford a brown solid (0.51 g). Purification by silica gel chromatography (Heptane: Ethyl acetate 2:1) afforded the title compound as a yellow solid (0.33 g). LCMS (method F) RT = 2.09 min, [M+H⁺]= 474, 476, 477. ¹H NMR (400 MHz, CDCl₃): 7.65-7.67 (m, 2H), 7.40-7.43 (m, 1H), 7.36-7.37 (m, 1H), 7.34 (t, 1H), 7.15 (s, 2H), 6.17 (d, 1H), 5.42 (q, 1H), 3.50 (t, 2H), 3.38 (t, 2H), 2.46 (s, 3H).

### Example 11: Intermediate preparation

### 4-[(E)-3-(3,5-dichloro-phenyl)-4,4,4-trifluoro-but-2-enoyl]-2-methyl-N-(cis-1-oxide-thietan-3-yl)-benzamide

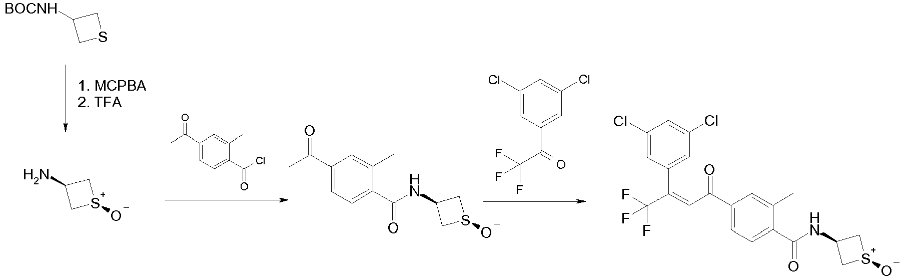

### Step A: cis-1-oxide-thietan-3ylamine

Thietan-3-yl-carbamic acid tert-butyl ester (prepared as described in WO09080250, 5.0 g, 26.4 mmol) was dissolved in dichloromethane (400 ml), followed by the addition of NaHCO₃ as a solution in water (150 ml). The reaction mixture was cooled to 0°C and under strong stirring, 3-chloroperbenzoic acid (6.5 g, 264.4 mmol) was added dropwise as a solution in dichloromethane (100 ml) within 30 minutes. The reaction mixture temperature did not go over 4°C during the addition. Dichloromethane (50 ml) was added for washing, and the reaction mixture was stirred at around 2-3 °C during 2 hours. Immiscible phases were separated, and the aqueous phase was extracted with dichloromethane (3x). The combined organic phases were collected, dried over Na₂SO₄, filtered and concentrated in vacuo to give a white solid (5.3 g). This crude product was purified by silica gel chromatography (ethyl acetate / heptane / methanol 75:25:0 - 100:0:0 - 80:0:20) and first afforded *cis*-1-oxide-thietan-3-yl carbamic acid tert-butyl ester (2.09 g), then a mixture of both *cis*-1-oxide-thietan-3-yl carbamic acid tert-butyl ester and *trans*-1-oxide-thietan-3yl carbamic acid tert-butyl ester (1.15 g), and finally 1,1-dioxide-thietan-3-yl carbamic acid tert-butyl ester (0.92 g).

To a solution of *cis*-1-oxide-thietan-3-yl carbamic acid tert-butyl ester (10 g, 5.3 mmol) at 0 °C was added trifluoroacetic acid (6.5 g, 5.7 mmol). The resulting mixture was stirred at 0 °C overnight and was then concentrated in vacuo to afford the title salt as a yellow oil (12.1 g), which was used without further purification in the next step. ¹H NMR (400 MHz, DMSO-d₆): 12.1 (bs, 1H), 8.73 (bs, 2H), 4.50-4.57 (m, 2H), 4.35-4.43 (m, 2H), 4.07-4.16 (m, 1H).

### Step B: 4-Acetyl-2-methyl-N-(cis-I-oxide-thietan-3-yl)-benzamide

To a solution of *cis*-1-oxide-thietan-3ylamine (2.2 g, 10 mmol) and 4-Acetyl-2-methylbenzoic acid chloride (2 g, 10 mmol) in CH₂Cl₂ (21 ml) was added triethylamine (5.05 g, 50 mmol) at 0 °C. The resulting mixture was stirred at room temperature overnight. The mixture was quenched with saturated aqueous NH₄Cl. The aqueous phase was extracted with CH₂Cl₂ (3x50mL). The combined organic phases were washed with saturated aqueous Na₂CO₃, dried over Na₂SO₄ and concentrated in vacuo to afford the title compound as an amber solid (2.2 g). ¹H NMR (400 MHz, CDCl₃): 7.80 (s, 1H), 7.77 (d, 1H), 7.44 (d, 1H), 6.66 (d, 1H), 4.69 (q, 1H), 4.17-4.32 (m, 2H), 3.29 (dt, 2H), 2.61 (s, 3H), 2.48 (s,3H).

### Step C: 4-[('E)-3-(3,5-dichloro-phenyl)-4,4,4-trifluoro-but-2-enoyl]-2-methyl-N-(cis-1-oxide-thietan-3-yl-benzamide

4-Acetyl-2-methyl-N-(cis-1-dioxide-thietan-3-yl)-benzamide (1.0 g, 3.7 mmol), (3,5 dichloro 2,2,2 trifluoroacetophenone (0.9 g, 3.7 mmol) potassium carbonate (0.52 g, 3.7 mmol) and triethylamine (40 mg, 0.37 mmol) were dissolved in 1,2 dichloroethane (11 ml). The resulting mixture was refluxed overnight. The reaction mixture was cooled to room temperature and was diluted with warm (40 °C) ethyl acetate, water was added, and the separated aqueous layer was extacted with warm EtOAc, the organic layer was washed brine with dried with Na₂SO₄ and concentrated in vacuo to afford an off white solid (1.8 g). LCMS (method F) RT = 1.89 min, [M+H⁺]= 490, 492. ¹H NMR (400 MHz, CDCl₃): 7.66-7.68 (m, 2H), 7.42-7.45 (m, 2H), 7.36-7.38 (m, 1H), 7.34 (t, 1H), 7.15 (d, 2H), 6.54 (d, 1H), 4.64-4.75 (m, 1H), 4.17-4.24 (m, 2H), 3.25 (dt, 2H), 2.46 (s, 3H).

### Example 12: Intermediate preparation

### 4-[(E)-3-(3,5-dichloro-phenyl)-4,4,4-trifluoro-but-2-enoyl]-2-methyl-N-(1,1-dioxide-thietan-3-yl)-benzamide

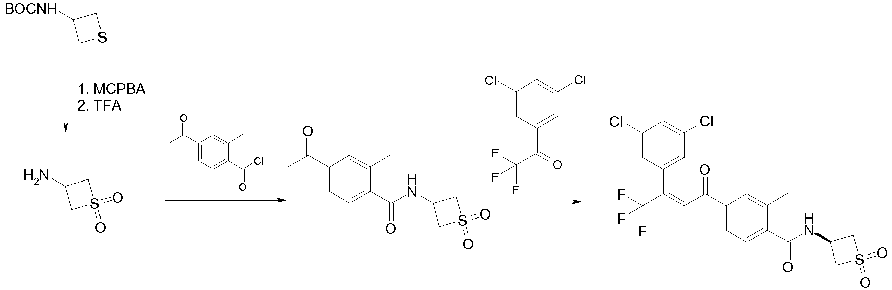

### Step A: 1,1-dioxide-thietan-3ylamine

Thietan-3-yl-carbamic acid tert-butyl ester (prepared as described in WO09080250, 5.0 g, 26.4 mmol) was dissolved in dichloromethane (400 ml), followed by the addition of NaHCO₃ as a solution in water (150 ml). The reaction mixture was cooled to 0°C and under a strong stirring, 3-chloroperbenzoic acid (6.5 g, 264.4 mmol) was added dropwise as a solution in dichloromethane (100 ml) within 30 minutes. The reaction mixture temperature did not go over 4°C during the addition. Dichloromethane (50 ml) was added for washing, and the reaction mixture was stirred at around 2-3 °C during 2 hours. Immiscible phases were separated, and the aqueous phase was extracted with dichloromethane (3x). The combined organic phases were collected, dried over Na₂SO₄, filtered and concentrated in vacuo to give a white solid (5.3 g). This crude product was purified by silica gel chromatography (ethyl acetate / heptane / methanol 75:25:0 - 100:0:0 - 80:0:20) and first afforded *cis*-1-oxide-thietan-3-yl carbamic acid tert-butyl ester (2.09 g), then a mixture of both *cis*-1-oxide-thietan-3-yl carbamic acid tert-butyl ester and *trans*-1-oxide-thietan-3yl carbamic acid tert-butyl ester (1.15 g), and finally 1,1-dioxide-thietan-3-yl carbamic acid tert-butyl ester (0.92 g).

To a solution of 1,1-dioxide-thietan-3-yl carbamic acid tert-butyl ester (1.0 g, 4.5 mmol) was added trifluoroacetic acid (1 ml, 12.9 mmol). The resulting mixture was stirred for 2h at room temperature. Another injection of trifluoroacetic acid (3 ml) was performed after 2h followed by a last addition of trifluoroacetic acid (5 ml) after 5h. Solvents were then removed in vacuo. The resulting gum was washed with Et₂O (3x) and the resulting solid was dried in vacuo to afford the title compound as a white powder (0.92 g). ¹H NMR (400 MHz, DMSO-d₆): 8.31 (bs, 2H), 4.01 (t, 2H), 3.70-3.81 (bs, 1H), 3.27 (t, 2H).

### Step B: 4-Acetyl-2-methyl-N-(1,1-dioxide-thietan-3-yl)-benzamide

To a solution of 4-acetyl-2-methyl benzoylchloride (4.6 g, 23 mmol) and Et₃N (2.53 g, 25mmol) in CH₂Cl₂ (100 ml) cooled to 0 °C was added hydro thietane-3-yl amine1,1-dioxide trifluoroacetate (5.0 g, 21 mmol). The resulting mixture was stirred overnight at room temperature. The mixture was washed successively with water and extracted with CH₂Cl₂ (2x100 ml). The combined organic phases were washed with 2N HCl. The organic layer was dried over Na₂SO₄ and evaporated in vacuo to afford yellow oil (5.9 g). LCMS (method F) RT = 1.05 min, [M+H⁺]=282. ¹H NMR (400 MHz, CDCl₃): 7.82 (s, 1H), 7.78 (d, 1H), 7.46 (d, 1H), 6.63 (d, 1H), 4.86-4.94 (m, 1H), 4.59-4.67 (m, 2H), 4.03-4.08 (m, 2H), 2.62 (s, 3H), 2.50 (s, 3H).

### Step C: 4-[(E)-3-(3,5-dichloro-phenyl)-4,4,4-trifluoro-but-2-enoyl]-2-methyl-N-(1,1-dioxide-thietan-3-yl-benzamide

4-Acetyl-2-methyl-N-(1,1-dioxide-thietan-3-yl)-benzamide (0.28 g, 1 mmol), (3,5 dichloro 2,2,2 trifuloroacetophenone (0.24 g, 1 mmol) potassium carbonate (138 mg, 1 mmol) and triethylamine (10 mg, 0.1 mmol) were dissolved in 1,2 dichloroethane (2.5 ml). The resulting mixture was refluxed for 3h. The reaction mixture was cooled to room temperature and saturated aqueous NH₄Cl was added. The mixture was extracted with CH₂Cl₂ (3x50 ml). The combined organic extracts were dried over Na₂SO₄ and concentrated in vacuo to afford a brown solid (0.52 g). Purification by silica gel chromatography (heptane: Ethyl acetate 2:1) afforded the title compound as a yellow solid (0.33 g). The solid was suspended in heptane (10 ml) and stirred for 1h at 40 °C. It was then cooled to 0 °C, filtered, washed with 1 ml cold TBME. After being dried under vacuum the title compound was obtained as a white solid (0.50 g). ¹H NMR (400 MHz, CDCl₃): 7.65-7.69 (m, 2H), 7.45-7.47 (m, 2H), 7.43-7.45 (m, 2H), 7.36-7.37 (m, 1H), 7.35 (t, 1H), 7.15 (d, 2H), 6.49 (d, 1H), 4.86-4.94 (m, 1H), 4.59-4.67 (m, 2H), 3.99-4.05 (m, 2H), 2.48 (s, 3H).

### Example 13: (not according to the invention)

### 4-[5-(3,5-Dichloro-phenyl)-5-trifluoromethyl-4,5-dihydro-isoxazol-3-yl]-2-methyl-N-thietan-3-yl-benzamide

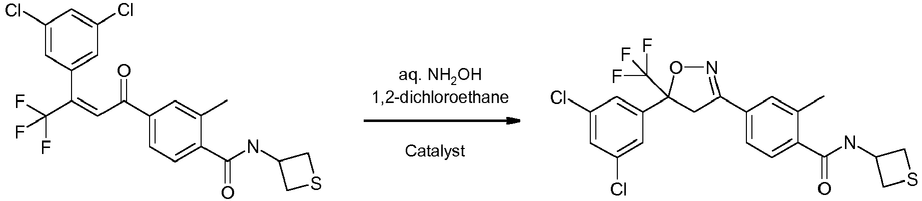

### Catalyst:

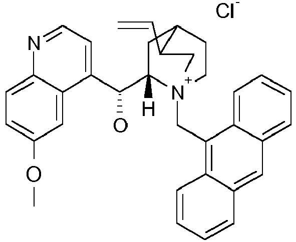

A pre-cooled solution of 5M sodium hydroxide (0.25 ml) was added to a solution of hydroxylamine (50% in water, 37 mg) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and anthracenyl-methyl quininium chloride (20 mg) (catalyst preparation Example 1) in 1,2-dichloroethane (1.5 ml) cooled in an ice-acetone bath. The mixture was stirred rapidly at ca -25 °C for 5 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 5%) to give the title compound (90 mg) as a white solid. ¹H NMR (400 MHz, CDCl₃) 7.52 (m, 4H), 7.42 (m, 2H), 6.18 (d, 1H), 5.41 (q, 1H), 4.09 (d, 1H), 3.67 (d, 1H), 3.50 (t, 2H), 3.39 (t, 2H), 2.47 (s, 3H). ¹⁹F NMR (376 MHz, CDCl₃) -79.51 (s, 3F). The product was analysed by chiral HPLC (method I) and compared to reference samples of ISOMERS A and B of the title compound (chiral separation Example): the product contained ISOMER B (retention time 8.60 minutes) as the major product (40%ee).

### Example 14

### 4-[5-(3,5-Dichloro-phenyl)-5-trifluoromethyl-4,5-dihydro-isoxazol-3-yl]-2-methyl-N-thietan-3-yl-benzamide

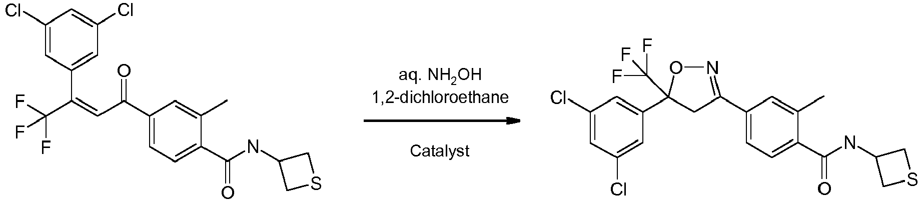

### Catalyst:

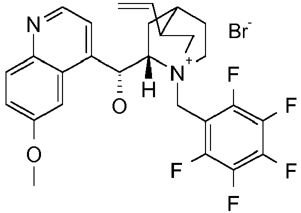

A pre-cooled solution of 5M sodium hydroxide (0.25 ml) was added to a solution of hydroxylamine (50% in water, 37 mg) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 2,3,4,5,6-pentafluorophenyl-methyl quininium bromide (20 mg) (catalyst preparation Example 3) in 1,2-dichloroethane (1.5 ml) cooled by a cryostat in an acetone bath. The mixture was stirred rapidly at ca -15 °C for 3 hours. The reaction mixture was diluted with dichloromethane, passed through an isolute phase separating cartridge and concentrated *in vacuo* to leave a yellow oil. This residue was purified by chromatography on silica gel (eluent: heptane / ethyl acetate 20%) to give the title compound (100 mg) as a white solid. ¹H NMR (400 MHz, CDCl₃) 7.52 (m, 4H), 7.42 (m, 2H), 6.18 (d, 1H), 5.41 (q, 1H), 4.09 (d, 1H), 3.67 (d, 1H), 3.50 (t, 2H), 3.39 (t, 2H), 2.47 (s, 3H). ¹⁹F NMR (376 MHz, CDCl₃) - 79.51 (s, 3F). The product was analysed by chiral HPLC (method I) and compared to reference samples of ISOMERS A and B of the title compound (Chiral separation Example): the product contained ISOMER B (retention time 8.80 min) as the major product (63%ee).

### Example 15

### 4-[5-(3,5-Dichloro-phenyl)-5-trifluoromethyl-4,5-dihydro-isoxazol-3-yl]-2-methyl-N-thietan-3-yl-benzamide

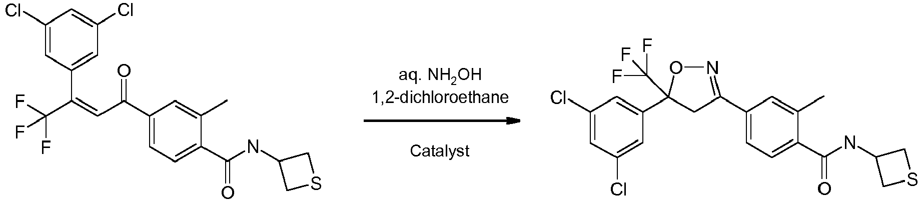

### Catalyst:

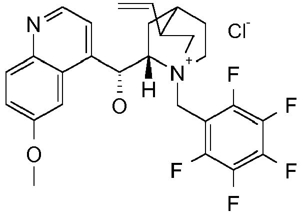

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 2,3,4,5,6-pentafluorophenyl-methyl quininium chloride (21 mg) (catalyst preparation Example 4) in 1,2-dichloroethane (1.5 ml) cooled in an ice bath. The mixture was stirred rapidly at ca 0°C for 2 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (75 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (75%ee).

### Example 16

### 4-[5-(3,5-Dichloro-phenyl)-5-trifluoromethyl-4,5-dihydro-isoxazol-3-yl]-2-methyl-N-thietan-3-yl-benzamide

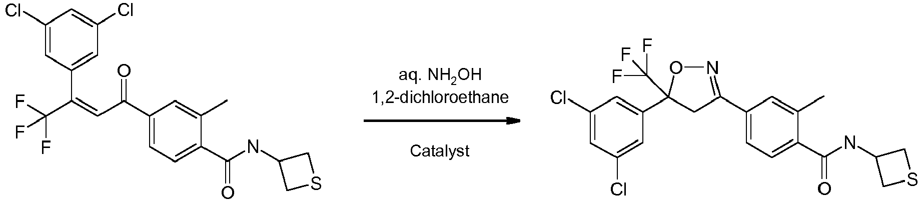

### Catalyst:

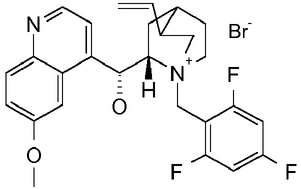

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 2,4,6-trifluorophenyl-methyl quininium bromide (19 mg) (catalyst preparation Example 2) in 1,2-dichloroethane (1.5 ml) cooled by a cryostat in an acetone bath. The mixture was stirred rapidly at ca -15°C for 3 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (100 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) - 79.51 (s, 3F).The product was analysed by chiral HPLC (method I) and compared to reference samples of ISOMERS A and B of the title compound (chiral separation Example): the product contained ISOMER B (retention time 8.40 min) as the major product (49%ee).

### Example 17

### 4-[5-(3,5-Dichloro-phenyl)-5-trifluoromethyl-4,5-dihydro-isoxazol-3-yl]-2-methyl-N-thietan-3-yl-benzamide

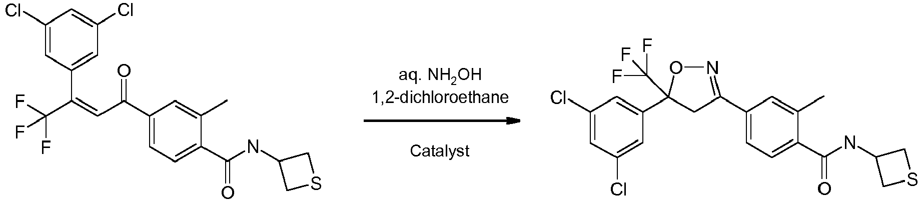

### Catalyst:

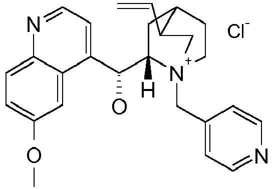

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 4-pyridine-methyl-quininium chloride (15 mg) (catalyst preparation Example 7) in 1,2-dichloroethane (1.5 mL) cooled by a cryostat in an acetone bath. The mixture was stirred rapidly at ca - 15°C for 3 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (54 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (51%ee).

### Example 18

### 4-[5-(3,5-Dichloro-phenyl)-5-trifluoromethyl-4,5-dihydro-isoxazol-3-yl]-2-methyl-N-thietan-3-yl-benzamide

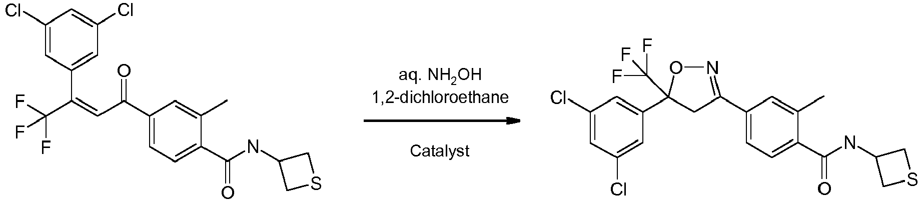

### Catalyst:

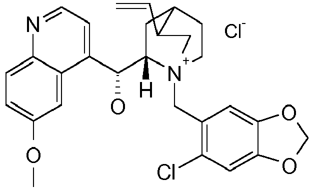

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 6-piperonyl quininium chloride (18 mg) (catalyst preparation Example 5) in 1,2-dichloroethane (1.5 ml) cooled by a cryostat in an acetone bath. The mixture was stirred rapidly at ca -15°C for 3 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (100 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (51%ee).

### Example 19

### 4-[5-(3,5-Dichloro-phenyl)-5-trifluoromethyl-4,5-dihydro-isoxazol-3-yl]-2-methyl-N-thietan-3-yl-benzamide

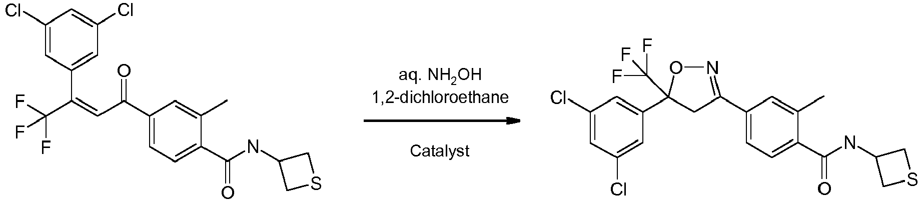

### Catalyst:

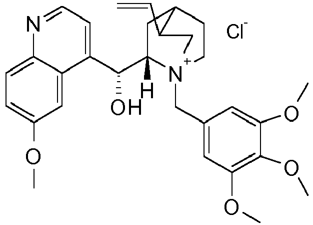

A pre-cooled solution of 5M sodium hydroxide (0.25 ml) was added to a solution of hydroxylamine (50% in water, 0.075 mL) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 3,4,5-trimethoxyphenyl-methyl quininium chloride (22 mg) (catalyst preparation Example 6) in 1,2-dichloroethane (1.5 ml) cooled in an ice bath. The mixture was stirred rapidly at ca 0°C for 2 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (67 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (77%ee).

### Example 20

### 4-[5-(3,5-Dichloro-phenyl)-5-trifluoromethyl-4,5-dihydro-isoxazol-3-yl]-2-methyl-N-thietan-3-yl-benzamide

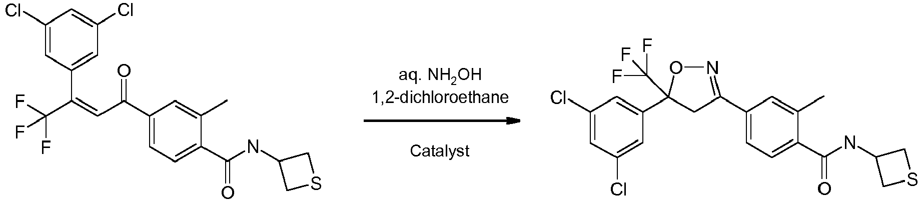

### Catalyst:

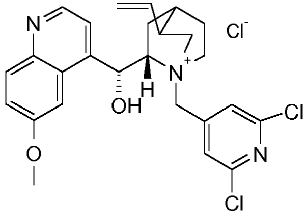

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 2,6-dichloropyridine-methyl quininium chloride (21 mg) (catalyst preparation Example 8) in 1,2-dichloroethane (1.5 ml) cooled in an ice bath. The mixture was stirred rapidly at ca 0°C for 2 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (70 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (66%ee).

### Example 21: (not according to the invention)

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

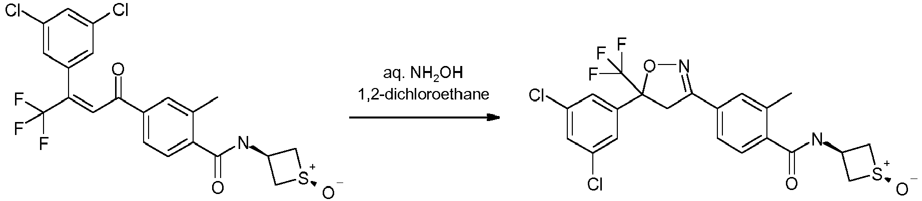

### Catalyst:

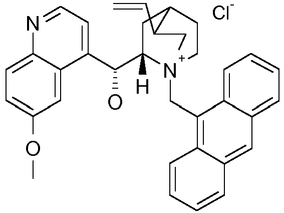

A pre-cooled solution of 5M sodium hydroxide (0.25 ml) was added to a solution of hydroxylamine (50% in water, 37 mg) at 5 °C (ice bath). The solution was stirred for 15 min at 5 °C then added to a vigorously stirred solution of 1, 4-[(E)-3-(3,5-dichloro-phenyl)-4,4,4-trifluoro-but-2-enoyl]-2-methyl-N-(cis-1-oxide-thietan-3-yl)-benzamide (100 mg) and anthracenyl-methyl quininium chloride (24 mg) (catalyst preparation Example 1) in 1,2-dichloroethane (1.5 ml) cooled in an ice bath. The mixture was stirred rapidly at ca 0 °C for 1 hour. The reaction mixture was diluted with dichloromethane, passed through an isolute phase separating cartridge and concentrated *in vacuo* to leave a yellow oil. This residue was purified by chromatography on silica gel (eluent: ethyl acetate / ethanol 5%) to give the title compound (90 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (S, 3F). The product was analyzed by chiral HPLC (method J) and compared to reference samples of ISOMERS A and B of the title compound (chiral separation Example): the product contained ISOMER B (retention time 17.6 min) as the major product (37%ee).

### Example 22

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

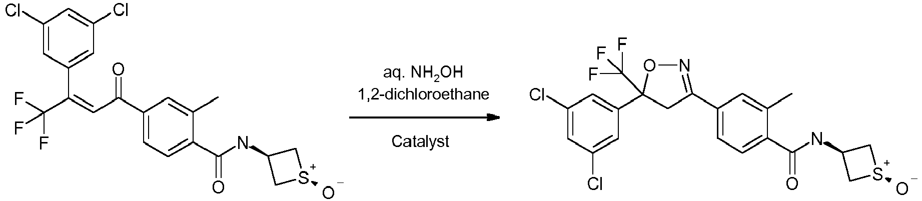

### Catalyst:

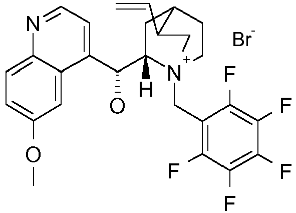

A pre-cooled solution of 5M sodium hydroxide (0.25 ml) was added to a solution of hydroxylamine (50% in water, 37 mg) at 5°C (ice bath). The solution was stirred for 15 min at 5°C then added to a vigorously stirred solution of 1, 4-[(E)-3-(3,5-dichloro-phenyl)-4,4,4-trifluoro-but-2-enoyl]-2-methyl-N-(cis-1-oxide-thietan-3-yl)-benzamide (100 mg) and 2,3,4,5,6-pentafluorophenyl-methyl quininium bromide (24 mg) (catalyst preparation Example 3) in 1,2-dichloroethane (1.5 ml) cooled in an ice bath. The mixture was stirred rapidly at ca 0 °C for 1 hour. The reaction mixture was diluted with dichloromethane, passed through an isolute phase separating cartridge and concentrated *in vacuo* to leave a yellow oil. This residue was purified by chromatography on silica gel (eluent: ethyl acetate / ethanol 5%) to give the title compound (34 mg) as a white solid. ¹H NMR (400 MHz, CDCl₃) 7.48 (m, 6H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.1 min) as the major product (87%ee).

### Example 23

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

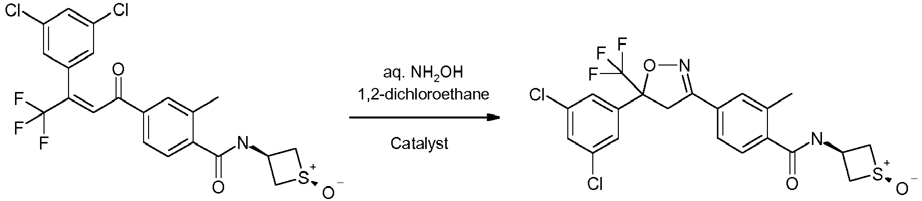

### Catalyst:

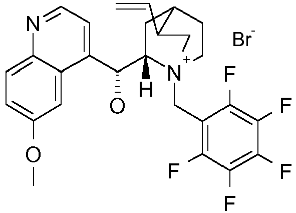

A pre-cooled solution of 5M sodium hydroxide (0.45 ml) was added to a solution of hydroxylamine (50% in water, 67 mg) at 5°C (ice bath). The solution was stirred for 15 min at 5°C then added to a vigorously stirred solution of 1, 4-[(E)-3-(3,5-dichloro-phenyl)-4,4,4-trifluoro-but-2-enoyl]-2-methyl-N-(cis-1-oxide-thietan-3-yl)-benzamide (500 mg) and 2,3,4,5,6-pentafluorophenyl-methyl quininium bromide (120 mg) (catalyst preparation Example 3) in 1,2-dichloroethane (7 ml) cooled in an ice bath. The mixture was stirred rapidly at ca 0 °C for 2 hour. The reaction mixture was diluted with dichloromethane, passed through an isolute phase separating cartridge and concentrated *in vacuo* to leave a yellow oil. This residue was purified by chromatography on silica gel (eluent: ethyl acetate / ethanol 5%) to give the title compound (450 mg) as a white solid. ¹H NMR (400 MHz, CDCl₃) 7.48 (m, 6H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.0 minutes) as the major product (90%ee). Optical rotation was measured on a 3.1 mg sample dissolved in 1ml ethanol using a 1 dm path length. [α_{D}] +58.7°.

### Example 24

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

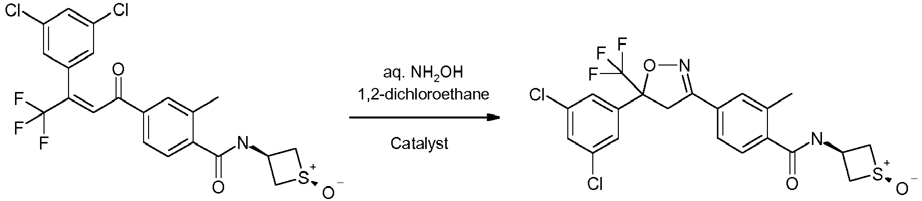

### Catalyst:

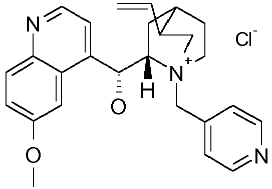

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 1, 4-[(E)-3-(3,5-dichloro-phenyl)-4,4,4-trifluoro-but-2-enoyl]-2-methyl-N-(cis-1-oxide-thietan-3-yl)-benzamide (100 mg) and 4-pyridine-methyl-quininium chloride (15 mg) (catalyst preparation Example 7) in 1,2-dichloroethane (1.5 ml) cooled in an ice bath. The mixture was stirred rapidly at ca 0°C for 1 hour. The reaction mixture was diluted with dichloromethane, passed through an isolute phase separating cartridge and concentrated *in vacuo* to leave yellow oil. This residue was purified by chromatography on silica gel (eluent: ethyl acetate / ethanol 5%) to give the title compound (100 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.14 min) as the major product (59%ee).

### Example 25

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

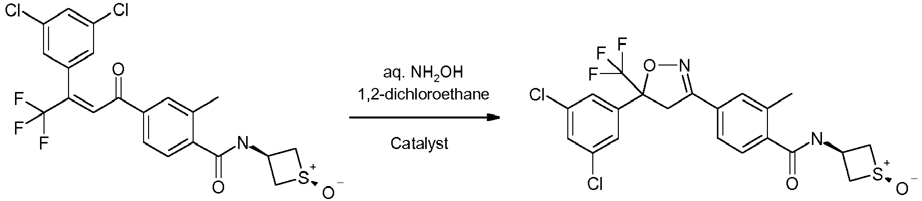

### Catalyst:

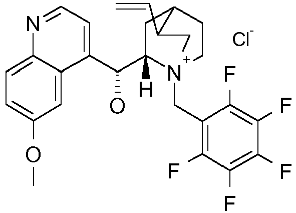

A pre-cooled solution of 5M sodium hydroxide (0.09 ml) was added to a solution of hydroxylamine (50% in water, 0.026 ml) at 5°C (ice bath). The solution was stirred for 15 min at 5°C then added to a vigorously stirred solution of 1, 4-[(E)-3-(3,5-dichloro-phenyl)-4,4,4-trifluoro-but-2-enoyl]-2-methyl-N-(*cis*-1-oxide-thietan-3-yl)-benzamide (100 mg) and 2,3,4,5,6-pentafluorophenyl-methyl quininium chloride (21 mg) (catalyst preparation Example 4) in 1,2-dichloroethane (1.5 ml) cooled in an ice bath. The mixture was stirred rapidly at ca 0°C for 3 hour. The reaction mixture was diluted with dichloromethane, passed through an isolute phase separating cartridge and concentrated *in vacuo* to leave yellow oil. This residue was purified by chromatography on silica gel (eluent: ethyl acetate / ethanol 5%) to give the title compound (85 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.05 min) as the major product (73%ee).

### Example 26

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

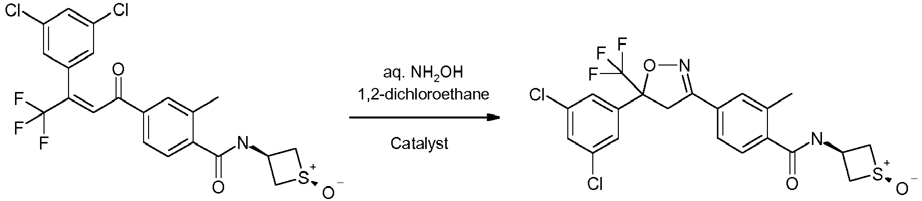

### Catalyst:

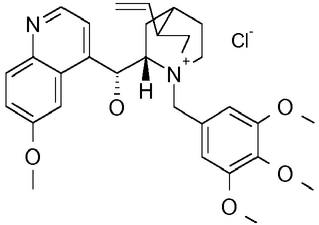

A pre-cooled solution of 5M sodium hydroxide (0.09 ml) was added to a solution of hydroxylamine (50% in water, 0.026 mL) at 5°C (ice bath). The solution was stirred for 15 min at 5°C then added to a vigorously stirred solution of 1, 4-[(E)-3-(3,5-dichloro-phenyl)-4,4,4-trifluoro-but-2-enoyl]-2-methyl-N-(*cis*-1-oxide-thietan-3-yl)-benzamide (100 mg) and 3,4,5 -trimethoxyphenyl-methyl quininium chloride (22 mg) (catalyst preparation Example 6) in 1,2-dichloroethane (1.5 ml) cooled in an ice bath. The mixture was stirred rapidly at ca 0°C for 3 hour. The reaction mixture was diluted with dichloromethane, passed through an isolute phase separating cartridge and concentrated *in vacuo* to leave yellow oil. This residue was purified by chromatography on silica gel (eluent: ethyl acetate / ethanol 5%) to give the title compound (90 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.36 min) as the major product (70%ee).

### Example 27: (not according to the invention)

### 4-[5-(3,5-Dichloro-phenyl)-5-trifluoromethyl-4,5-dihydro-isoxazol-3-yl]-2-methyl-N-(1,1-dioxo-thietan-3-yl)-benzamide

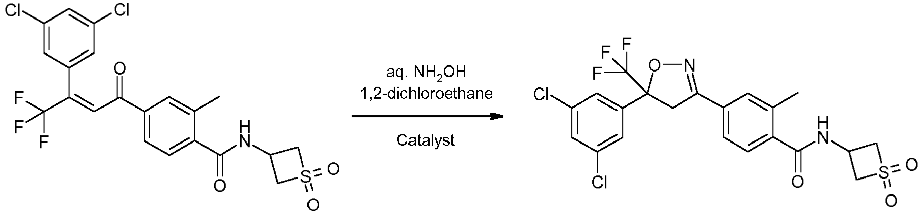

### Catalyst:

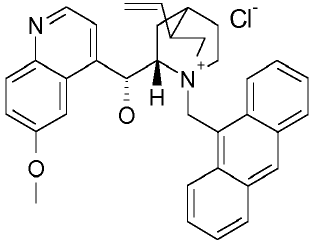

Sodium hydroxide (35 mg, 0.88 mmol) was dissolved in water (0.20 ml), the mixture was cooled to 0 °C in an ice bath and a solution of hydroxyl amine (50% in water, 0.49 ml) was added dropwise. The mixture was stirred for 5-10 min, then added dropwise to a stirred solution of 1, 4-[(E)-3-(3,5-dichloro-phenyl)-4,4,4-trifluoro-but-2-enoyl]-2-methyl-N-(1,1-dioxide-thietan-3-yl)-benzamide (0.202g, 0.40 mmol) and anthracenyl-methyl quininium chloride (66 mg) (catalyst preparation Example 1) in 1,2-dichloroethane (4 ml) at -15°C, the mixture was stirred overnight. LC shows consumption of starting material and formation of product. The mixture was diluted with dichloromethane and quenched with dilute hydrochloric acid (2M, 1mL). The mixture was passed through an isolute phase separating cartridge and concentrated *in vacuo,* the residue was purified by chromatography on silica gel (eluent: cyclohexane / ethyl acetate 50%) to give the title compound (208 mg) as a colourless solid. ¹H NMR (400 MHz, CDCl₃) 7.52 (m, 4H), 7.42 (m, 2H), 6.79 (br. d, 1H), 4.85 (m, 1H), 4.58 (m, 2H), 4.05 (m, 3H), 3.71 (d, 1H), 2.44 (s, 3H). The product was analysed by chiral HPLC (method J) and compared to reference samples of ISOMERS A and B of the title compound (chiral separation Example): the product contained ISOMER B (retention time 21.5 minutes) as the major product (32.5 %ee).

### Example 28

### 4-[5-(3,5-Dichloro-phenyl)-5-trifluoromethyl-4,5-dihydro-isoxazol-3-yl]-2-methyl-N-(1,1-dioxo-thietan-3-yl)-benzamide

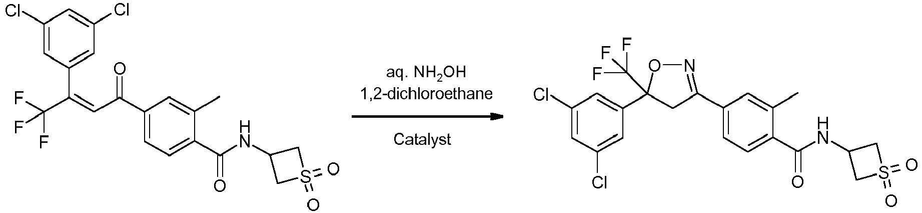

### Catalyst:

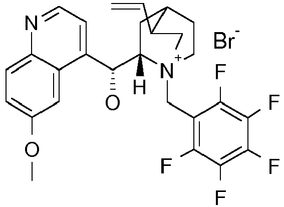

A solution of hydroxylamine (50% in water, 0.024 ml) was added to a pre-cooled (ice-water bath) solution of sodium hydroxide (5M, 0.088 ml), the mixture was stirred for one minute then added to a pre-cooled (ice-water bath)solution of 1, 4-[(E)-3-(3,5-dichloro-phenyl)-4,4,4-trifluoro-but-2-enoyl]-2-methyl-N-(*1*,1-dioxo-thietan-3-yl)-benzamide and 2,3,4,5,6-pentafluorophenyl-methyl quininium bromide (catalyst preparation Example 3) in 1,2-dichloroethane (2 ml). The mixture was stirred for 3 hr at 0-5 °C. A small sample was taken and ¹⁹F NMR (376 MHz, CDCl₃) shows formation of product and consumption of starting material. The mixture was diluted with dichloromethane and quenched with dilute hydrochloric acid (2M, 1 ml). The mixture was passed through an isolute phase separating cartridge and concentrated *in vacuo,* the residue was dissolved in cyclohexane, filtered and concentrated *in vacuo.* The residue was purified by chromatography on silica gel (eluent: cyclohexane / ethyl acetate 15-50%) to give the title compound (55 mg) as an off white solid. ¹H NMR (400 MHz, CDCl₃) 7.52 (m, 4H), 7.42 (m, 2H), 6.75 (br. d, 1H), 4.88 (m, 1H), 4.62 (m, 2H), 4.05 (m, 3H), 3.70 (d, 1H), 2.47 (s, 3H); ¹⁹F NMR (376 MHz, CDCl₃) 79.52 (s, 3F) :The product was analysed by chiral HPLC (method J) and compared to reference samples of ISOMERS A and B of the title compound (chiral separation Example): the product contained ISOMER B (retention time 21.6 minutes) as the major product (67 % ee).

### Example 29

### 4-[5-(3,5-Dichloro-phenyl)-5-trifluoromethyl-4,5-dihydro-isoxazo1-3-yl]-2-methyl-N-(1,1-dioxo-thietan-3-yl)-benzamide

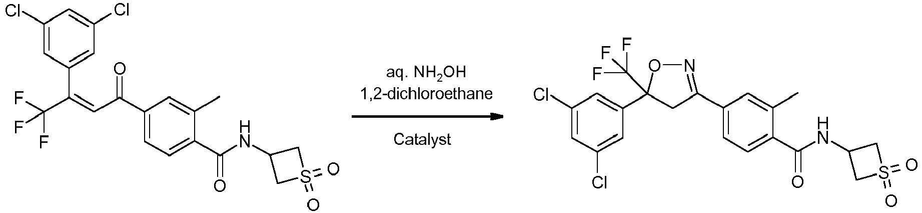

### Catalyst:

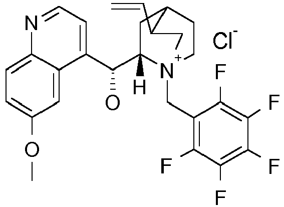

A pre-cooled solution of 5M sodium hydroxide (0.09 ml) was added to a solution of hydroxylamine (50% in water, 0.026 ml) at 5°C (ice bath). The solution was stirred for 15 min at 5°C then added to a vigorously stirred solution of 1, 4-[(E)-3-(3,5-dichloro-phenyl)-4,4,4-trifluoro-but-2-enoyl]-2-methyl-N-(*cis*-1-oxide-thietan-3-yl)-benzamide (100 mg) and 2,3,4,5,6-pentafluorophenyl-methyl quininium chloride (21 mg) (catalyst preparation Example 4) in 1,2-dichloroethane (1.5 ml) cooled in an ice bath. The mixture was stirred rapidly at ca 0°C for 3 hour. The reaction mixture was diluted with dichloromethane, passed through an isolute phase separating cartridge and concentrated *in vacuo* to leave yellow oil. This residue was purified by chromatography on silica gel (eluent: *n*-heptane / ethyl acetate 40%) to give the title compound (58 mg) as a white solid. ¹H NMR (400 MHz, CDCl₃) 7.52 (m, 4H), 7.42 (m, 2H), 6.75 (br. d, 1H), 4.88 (m, 1H), 4.62 (m, 2H), 4.05 (m, 3H), 3.70 (d, 1H), 2.47 (s, 3H); ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 21.45 min) as the major product (65%ee).

### Example 30

### 4-[5-(3,5-Dichloro-phenyl)-5-trifluoromethyl-4,5-dihydro-isoxazol-3-yl]-2-methyl-N-(1,1-dioxo-thietan-3-yl)-benzamide

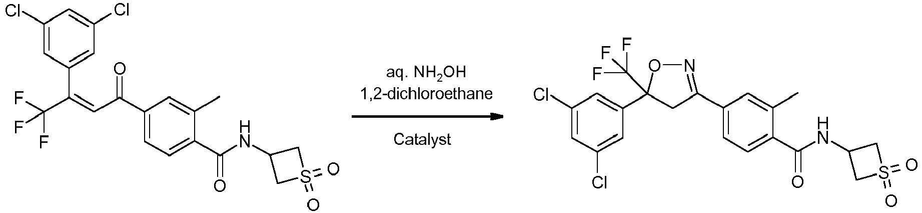

### Catalyst:

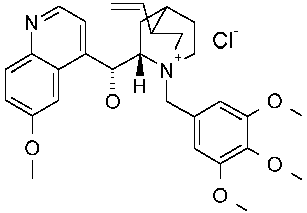

A pre-cooled solution of 5M sodium hydroxide (0.09 mL) was added to a solution of hydroxylamine (50% in water, 0.026 ml) at 5°C (ice bath). The solution was stirred for 15 min at 5°C then added to a vigorously stirred solution of 1, 4-[(E)-3-(3,5-dichloro-phenyl)-4,4,4-trifluoro-but-2-enoyl]-2-methyl-N-(*cis*-1-oxide-thietan-3-yl)-benzamide (100 mg) and 3,4,5 -trimethoxyphenyl-methyl quininium chloride (catalyst preparation Example 6) (22 mg) in 1,2-dichloroethane (1.5 ml) cooled in an ice bath. The mixture was stirred rapidly at ca 0°C for 3 hour. The reaction mixture was diluted with dichloromethane, passed through an isolute phase separating cartridge and concentrated *in vacuo* to leave yellow oil. This residue was purified by chromatography on silica gel (eluent: *n*-heptane / ethyl acetate 40%) to give the title compound (62 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 21.67 min) as the major product (68%ee).

### Example 31

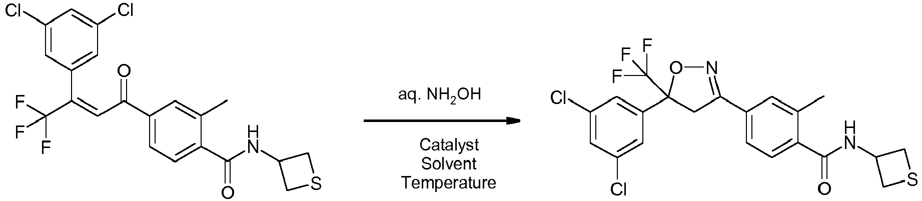

Following the general procedure described in Example 13, different catalysts, solvents and reaction conditions were screened for the preparation of 4-[5-(3,5-dichloro-phenyl)-5-trifluoromethyl-4,5-dihydro-isoxazol-3-yl]-2-methyl-N-(thietan-3-yl)-benzamide. The following results were obtained:

| Example | Catalyst | NH₂OH (eq.) | NaOH (eq.) | Catalyst (mol%) | Solvent | T°C | Yield | ee% |
|---|---|---|---|---|---|---|---|---|
| 31.1 (not according to the invention) | 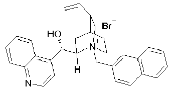 | 5 | 6 | 20mol% | DCE | -15°C | 62% | 5% |
| 31.2 (not according to the invention) | 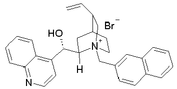 | 5 | 6 | 20mol% | DCE | 0°C | 73% | 6% |
| 31.3 (not according to the invention) | 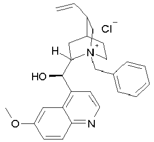 | 5 | 6 | 20mol% | DCE | -30°C | 66% | 20% |
| 31.4 (not according to the invention) | 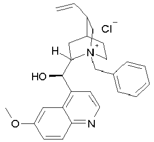 | 2 | 2.2 | 20mol% | DCE | 0°C | 68% | 21% |
| 31.5 (not according to the invention) | 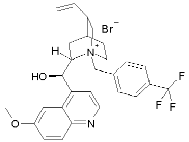 | 5 | 6 | 20mol% | DCE | -25°C | 78% | 15% |
| 31.6 (not according to the invention) | 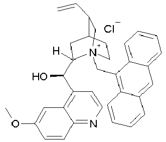 | 5 | 6 | 20mol% | DCE | -25°C | 88% | 40% |
| 31.7 | 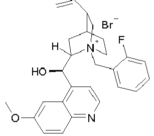 | 5 | 6 | 20mol% | DCE | -25°C | -25°C - | 25% |
| 31.8(not according to the invention) | 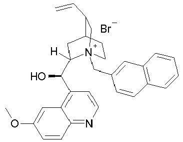 | 5 | 6 | 20mol% | DCE | -25°C | 92% | 20% |
| 31.9 (not according to the invention) | 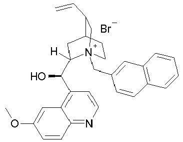 | 5 | 6 | 20mol% | DCE | 0°C | 97% | 20% |
| 31.10 | 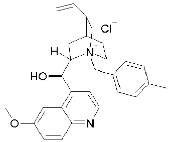 | 5 | 6 | 20mol% | DCE | -25°C | 77% | 20% |
| 31.11 (not according to the invention) | 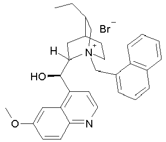 | 5 | 6 | 20mol% | DCE | -25°C | 97% | 20% |
| 31.12 (not according to the invention) | 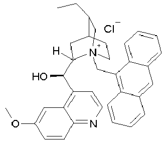 | 5 | 6 | 20mol% | DCE | -25°C | 83% | 40% |
| 31.13 (not according to the invention) | 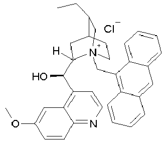 | 2 | 2.2 | 20mol% | DCE | 0°C | 68% | 41% |
| 31.14 (not according to the invention) | 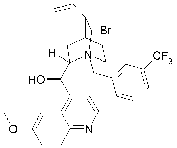 | 5 | 6 | 20mol% | DCE | -15°C | 77% | 23% |
| 31.15 (not according to the invention) | 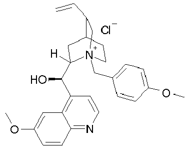 | 5 | 6 | 20mol% | DCE | -15°C | 72% | 27% |
| 31.16 | 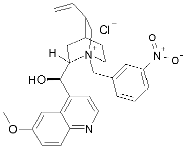 | 5 | 6 | 20mol% | DCE | -15°C | 74% | 15% |
| 31.17 | 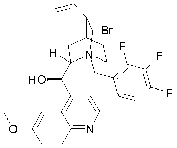 | 5 | 6 | 20mol% | DCE | -15°C | 81% | 30% |
| 31.18 (not according to the invention) | 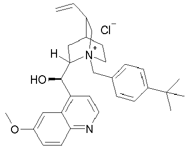 | 5 | 6 | 20mol% | DCE | -15°C | 87% | 20% |
| 31.19 | 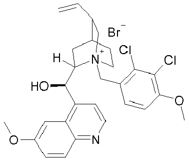 | 5 | 6 | 20mol% | DCE | -15°C | - | 46% |
| 31.20 | 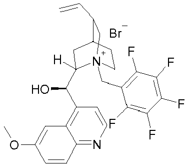 | 5 | 6 | 20mol% | DCE | -15°C | 98% | 63% |
| 31.21 (not according to the invention) | 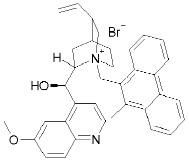 | 5 | 6 | 20mol% | DCE | -15°C | 97% | 30% |
| 31.22 | 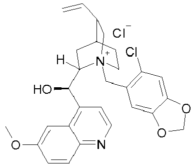 | 5 | 6 | 20mol% | DCE | -15°C | - | 51% |
| 31.23 | 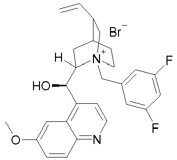 | 5 | 6 | 20mol% | DCE | -15°C | 97% | 38% |
| 31.24 | 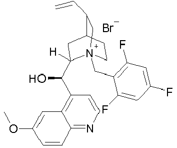 | 5 | 6 | 20mol% | DCE | -15°C | 97% | 49% |
| 31.25 | 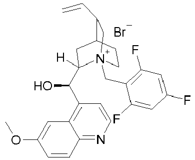 | 2 | 2.2 | 20mol% | DCE | 0°C | 97% | 57% |
| 31.26 | 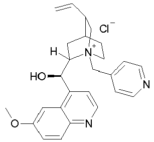 | 5 | 6 | 20mol% | DCE | -15°C | - | 51% |
| 31.27 (not according to the invention) | 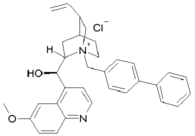 | 5 | 6 | 20mol% | DCE | -15°C | 74% | 25% |
| 31.28 | 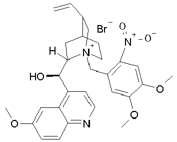 | 5 | 6 | 20mol% | DCE | -15°C | 34% | 44% |
| 31.29 | 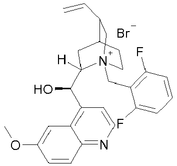 | 5 | 6 | 20mol% | DCE | -15°C | 63% | 47% |
| 31.30 (not according to the invention) | 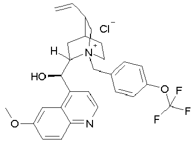 | 5 | 6 | 20mol% | DCE | -15°C | - | 22% |
| 31.31 | 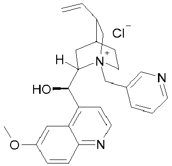 | 5 | 6 | 20mol% | DCE | -15°C | - | 46% |
| 31.32 (not according to the invention) | 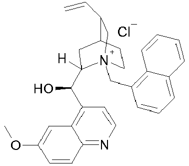 | 5 | 6 | 20mol% | DCE | -15°C | 100% | 33% |
| 31.33 | 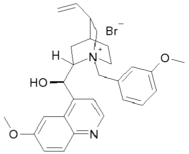 | 5 | 6 | 20mol% | DCE | -15°C | 100% | 33% |
| 31.34 | 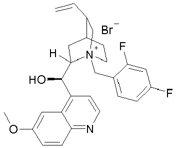 | 5 | 6 | 20mol% | DCE | -15°C | 100% | 37% |
| 31.35 | 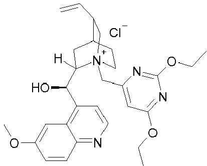 | 5 | 6 | 20mol% | DCE | 0°C | - | 47% |
| 31.36 | 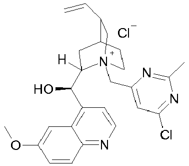 | 5 | 6 | 20mol% | DCE | 0°C | - | 26% |
| 31.37 | 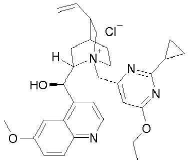 | 5 | 6 | 20mol% | DCE | 0°C | - | 29% |
| 31.38 (not according to the invention) | 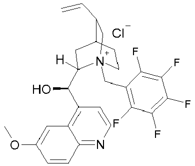 | 5 | 6 | 20mol% | DCE | 0°C | 73% | 75% |
| 31.39 (not according to the invention) | 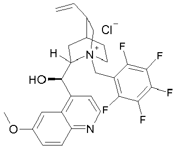 | 2 | 2.2 | 20mol% | DCE | 0°C | 68% | 68% |
| 31.40 | 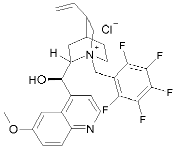 | 5 | 6 | 20mo1% | fluorob enzene | 0°C | 78% | 65% |
| 31.41 | 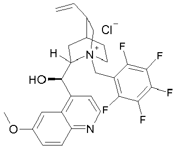 | 5 | 6 | 20mol% | α,α,α-trifluor otoluen e | 0°C | 63% | 46% |
| 31.42 | 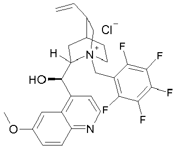 | 5 | 6 | 20mol% | t-butyl-acetate | 0°C | 81% | 62% |
| 31.43 | 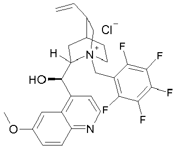 | 5 | 6 | 20mol% | dioxan | 0°C | 64% | 53% |
| 31.44 | 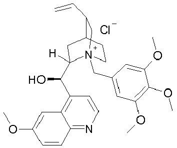 | 5 | 6 | 20mol% | DCE | 0°C | 65% | 77% |
| 31.45 | 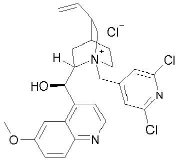 | 5 | 6 | 20mol% | DCE | 0°C | 68% | 66% |
| 31.46 | 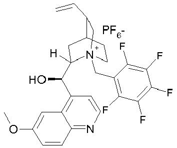 | 5 | 6 | 20mol% | DCE | 0°C | 68% | 60% |
| 31.47 | 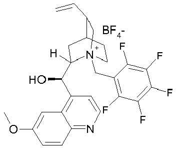 | 5 | 6 | 20mol% | DCE | 0°C | 38% | 83% |
| 31.48 | 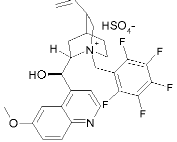 | 5 | 6 | 20mol% | DCE | 0°C | 72% | 58% |
| 31.49 | 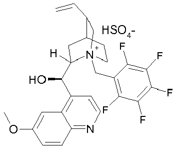 | 5 | 6 | 5mol% | DCE | 0°C | 100% | 59% |
| 31.50 | 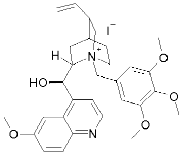 | 5 | 6 | 20mol% | DCE | 0°C | 63% | 77% |
| 31.51 | 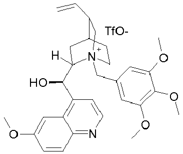 | 5 | 6 | 20mol% | DCE | 0°C | 63% | 77% |
| 31.52 | 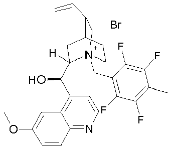 | 5 | 6 | 20mol% | DCE | 0°C | 94% | 51% |

### Example 32

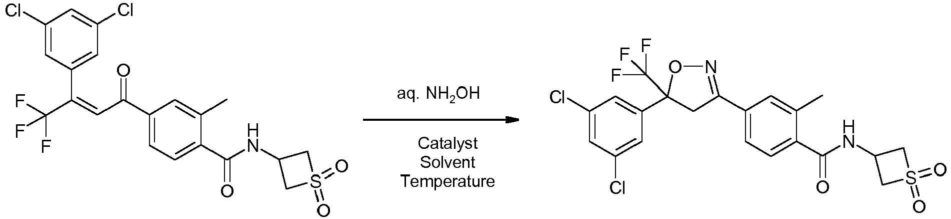

Following the general procedure described in Example 27, different catalysts, solvents and reaction conditions were screened for the preparation of 4-[5-(3,5-dichloro-phenyl)-5-trifluoromethyl-4,5-dihydro-isoxazol-3-yl]-2-methyl-N-(1,1-dioxo-thietan-3-yl)-benzamide. The following results were obtained:

| Example | Catalyst | NH₂OH (eq.) | NaOH (eq.) | Catalyst (mol%) | Solvent | T°C | Yield | ee% |
|---|---|---|---|---|---|---|---|---|
| 32.1 | 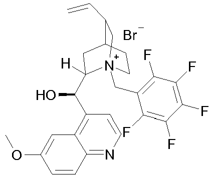 | 2 | 2.2 | 20mol% | DCE | 0°C | 58% | 61% |
| 32.2 (not according to the invention) | 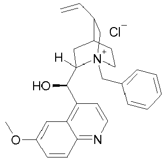 | 2 | 2.2 | 20mol% | DCE | 0°C | 28% | 21% |
| 32.3 (not according to the invention) | 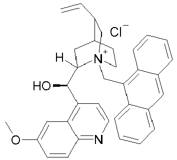 | 2 | 2.2 | 20mol% | DCE | 0°C | 46% | 33% |
| 32.4 | 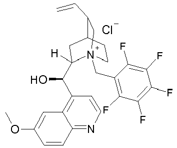 | 2 | 2.2 | 20mol% | DCE | 0°C | 56% | 65% |
| 32.5 | 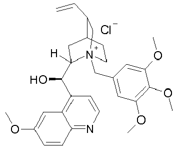 | 2 | 2.2 | 20mol% | DCE | 0°C | 60% | 68% |
| 32.6 | 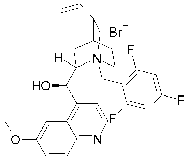 | 2 | 2.2 | 20mol% | DCE | 0°C | 48% | 52% |

### Example 33

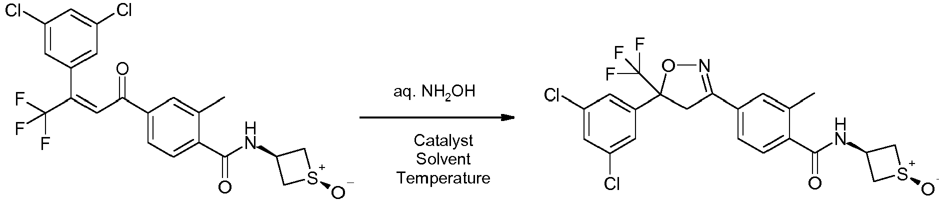

Following the general procedure described in Example 21, different catalysts, solvents and reaction conditions were screened for the preparation of 4-[5-(3,5-dichloro-phenyl)-5-trifluoromethyl-4,5-dihydro-isoxazol-3-yl]-2-methyl-N-(cis-1-oxo-thietan-3-yl)-benzamide. The following results were obtained:

| Example | Catalyst | NH₂OH (eq.) | NaOH (eq.) | Catalyst (mol%) | Solvent | T°C | Yield | ee% |
|---|---|---|---|---|---|---|---|---|
| 33.1 | 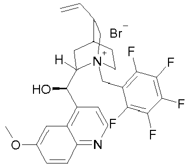 | 2 | 2.2 | 20mol% | DCE | 0°C | 88% | 90% |
| 33.2 | 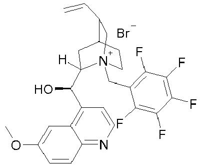 | 2 | 2.2 | 3mol% | DCE | 0°C | 100% | 73% |
| 33.3 (not according to the invention) | 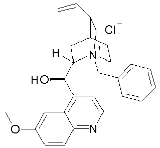 | 2 | 2.2 | 20mol% | DCE | 0°C | 92% | 20% |
| 33.4 (not according to the invention) | 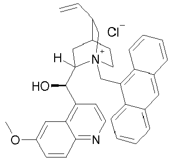 | 2 | 2.2 | 20mol% | DCE | 0°C | 68% | 37% |
| 33.5 | 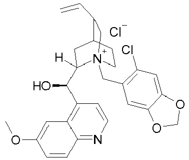 | 2 | 2.2 | 20mol% | DCE | 0°C | 40% | 16% |
| 33.6 | 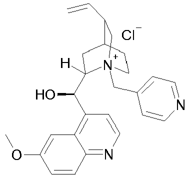 | 2 | 2.2 | 20mol% | DCE | 0°C | 100% | 60% |
| 33.7 | 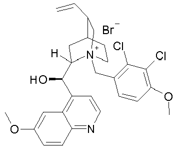 | 2 | 2.2 | 20mol% | DCE | 0°C | 100% | 39% |
| 33.8 | 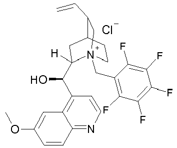 | 2 | 2.2 | 20mol% | DCE | 0°C | 87% | 73% |
| 33.9 | 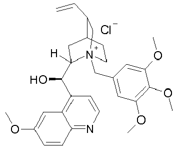 | 2 | 2.2 | 20mol% | DCE | 0°C | 83% | 70% |
| 33.10 | 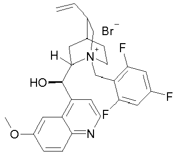 | 2 | 2.2 | 20mol% | DCE | 0°C | 63% | 64% |
| 33.11 | 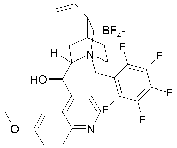 | 5 | 6 | 20mol% | DCE | 0°C | 53% | 50% |
| 33.12 | 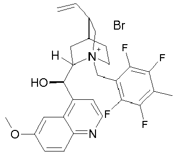 | 2 | 2.2 | 20mol% | DCE | -10°C | 88% | 79.5 % |

### Example 34:

Influence of the E/Z ratio of (4-[3-(3,5-dichloro-phenyl)-4,4,4-trifluoro-but-2-enoyl]-2-methyl-N-thietan-3-yl-benzamide) on enantiomeric excess

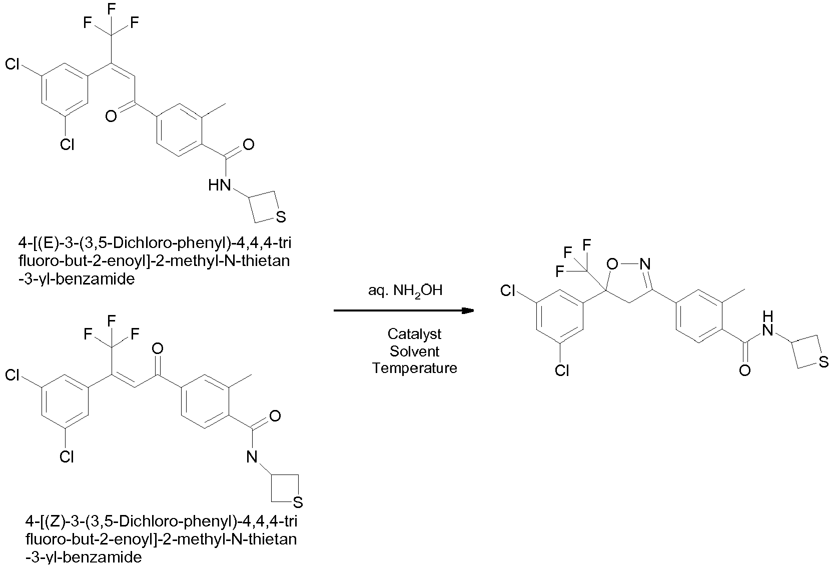

### Catalyst:

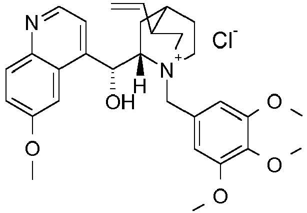

Using the procedure described in Example 10, Step E, different E/Z ratios of the intermediate 4-[3-(3,5-dichloro-phenyl)-4,4,4-trifluoro-but-2-enoyl]-2-methyl-N-thietan-3-yl-benzamide could be obtained by collecting different fractions from column chromatography (heptanes / ethyl acetate 5/1). Thus, from 4-acetyl-2-methyl-N-thietan-3-yl-benzamide (3.97 g), 3.2 g of a 75/25 E/Z mixture of 4-[3-(3,5-dichloro-phenyl)-4,4,4-trifluoro-but-2-enoyl]-2-methyl-N-thietan-3-yl-benzamide was obtained by collecting specific fractions from the chromatography; similarly, from 4-acetyl-2-methyl-N-thietan-3-yl-benzamide (3.97 g), 3.2 g of a 75/25 E/Z mixture of 4-[3-(3,5-dichloro-phenyl)-4,4,4-trifluoro-but-2-enoyl]-2-methyl-N-thietan-3-yl-benzamide were obtained. Similarly, in another experience, from 4-acetyl-2-methyl-N-thietan-3-yl-benzamide (10 g), 3.2 g of a 75/25 E/Z mixture of 4-[3-(3,5-dichloro-phenyl)-4,4,4-trifluoro-but-2-enoyl]-2-methyl-N-thietan-3-yl-benzamide was obtained; similarly, from 4-acetyl-2-methyl-N-thietan-3-yl-benzamide (3.97 g), 16 g of a 91/9 E/Z mixture of 4-[3-(3,5-dichloro-phenyl)-4,4,4-trifluoro-but-2-enoyl]-2-methyl-N-thietan-3-yl-benzamide were obtained by collecting all fractions from the flash chromatography.

A pre-cooled solution of 5M sodium hydroxide (0.093 ml) was added to a solution of hydroxylamine (50% in water, 0.026 mL) at 5°C (ice bath). The solution was stirred for 15 min at 5°C then added to a vigorously stirred solution of an isomeric mixture of 4-[(E)-3-(3,5-dichloro-phenyl)-4,4,4-trifluoro-but-2-enoyl]-2-methyl-N-thietan-3-yl-benzamide and 4-[(Z)-3-(3,5-dichloro-phenyl)-4,4,4-trifluoro-but-2-enoyl]-2-methyl-N-thietan-3-yl-benzamide (100 mg, ratio described in table below) and 3,4,5-trimethoxyphenyl-methyl quininium chloride (21 mg) (catalyst preparation Example 6) in 1,2-dichloroethane (1.5 ml) cooled in an ice bath. The mixture was stirred rapidly at ca 0°C for 2 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound. The product was analysed by chiral HPLC (method J) and compared to reference samples of ISOMERS A and B of the title compound (chiral separation Example): the product contained ISOMER B as the major product (enantiomeric excess in table below)

| Example | **E/Z ratio** (4-[3-(3,5-dichloro-phenyl)-4,4,4-trifluoro-but-2-enoyl]-2-methyl-N-thietan-3-yl-benzamide) | Yield | ee% |
|---|---|---|---|
| 34.1 | 75/25 | 85% | 73% |
| 34.2 | 91/9 | 68% | 77% |

### Example 35: Biological examples

This Example illustrates the pesticidal/insecticidal properties of compounds of formula (I). The absolute configuration of isomers A and B has been confirmed by X-ray diffraction. Isomer B corresponds to the compound of formula IB. Tests were performed as follows:
*Spodoptera littoralis* (Egyptian cotton leafworm):
   Cotton leaf discs were placed on agar in a 24-well microtiter plate and sprayed with test solutions at an application rate of 50 ppm. After drying, the leaf discs were infested with 5 L1 larvae. The samples were checked for mortality, feeding behavior, and growth regulation 3 days after treatment (DAT).

The following results were obtained:

| Compound (obtained as described in Example 9) | ISOMER | Control of *Spodoptera littoralis* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 50% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 50% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Heliothis virescens (Tobacco budworm):

Eggs (0-24 h old) were placed in 24-well microtiter plate on artificial diet and treated with test solutions at an application rate of 50 ppm (concentration in well 18 ppm) by pipetting. After an incubation period of 4 days, samples were checked for egg mortality, larval mortality, and growth regulation.

The following results were obtained:

| Compound (obtained as described in Example 9) | ISOMER | Control of *Heliothis virescens* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 50% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 0% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Plutella xylostella (Diamond back moth):

24-well microtiter plate (MTP) with artificial diet was treated with test solutions at an application rate of 50 ppm (concentration in well 4.5 ppm) by pipetting. After drying, the MTP's were infested with L2 larvae (7-12 per well). After an incubation period of 6 days, samples were checked for larval mortality and growth regulation.

The following results were obtained:

| Compound (obtained as described in Example 9) | ISOMER | Control of *Plutella xylostella* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 0% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 0% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Diabrotica balteata (Corn root worm):

A 24-well microtiter plate (MTP) with artificial diet was treated with test solutions at an application rate of 50 ppm (concentration in well 4.5 ppm) by pipetting. After drying, the MTP's were infested with L2 larvae (6-10 per well). After an incubation period of 5 days, samples were checked for larval mortality and growth regulation.

The following results were obtained:

| Compound (obtained as described in Example 9) | ISOMER | Control of *Diabrotica balteata* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 0% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 0% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Thrips tabaci (Onion thrips):

Sunflower leaf discs were placed on agar in a 24-well microtiter plate and sprayed with test solutions at an application rate of 50 ppm. After drying, the leaf discs were infested with an aphid population of mixed ages. After an incubation period of 7 days, samples were checked for mortality.

The following results were obtained:

| Compound (obtained as described in Example 9) | ISOMER | Control of *Thrips tabaci* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 0% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 0% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Tetranychus urticae (Two-spotted spider mite):

Bean leaf discs on agar in 24-well microtiter plates were sprayed with test solutions at an application rate of 50 ppm. After drying, the leaf discs are infested with mite populations of mixed ages. 8 days later, discs are checked for egg mortality, larval mortality, and adult mortality.

The following results were obtained:

| Compound (obtained as described in Example 9) | ISOMER | Control of *Tetranychus urticae* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 0% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 80% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Example 5: Catalyst preparation: 6-chloropiperonyl quininium chloride

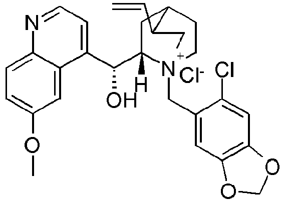

A solution of 6-chloropiperonyl chloride (0.41 g) and quinine (0.50 g) in toluene (9 ml) was heated at 80°C for 18 hours. The reaction mixture was poured in diethyl ether and then filtrate to afford the title product as a pale yellow solid (0.75 g). LCMS (method H) 1.12 min, M⁺ 494; ¹H NMR (400 MHz, CDCl₃) 8.83 (d, 1H), 8.11 (d, 1H), 7.86 (m, 2H), 7.41 (m, 2H), 7.09 (d, 1H), 6.98 (s, 1H), 6.72 (m, 2H), 6.10 (s, 2H), 5.61 (m, 1H), 5.12 (d, 1H), 5.05 (d, 1H), 4.62 (d, 1H), 3.98 (s, 3H), 3.67 (m, 1H), 3.38 (dd, 1H), 3.19 (m, 2H), 2.58 (m, 2H), 2.33 (m, 1H), 2.10 (m, 1H), 1.83 (m, 1H), 1.48 (m, 1H).

### Example 6: Catalyst preparation: 3,4,5-trimethoxybenzyl quininium chloride

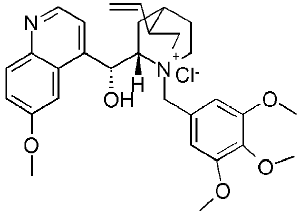

A solution of 3,4,5-trimethoxybenzyl chloride (0.42 g) and quinine (0.50 g) in toluene (9 ml) was heated at 80°C for 18 hours. The reaction mixture was poured in diethyl ether and then filtrate to afford the title product as a white solid (0.848g). LCMS (method G) 1.06 min, M⁺ 505; ¹H NMR (400 MHz, CDCl₃) 8.75 (d, 1H), 8.05 (d, 1H), 7.71 (d, 1H), 7.37 (m, 2H), 7.08 (s, 2H), 6.67 (d, 1H), 6.11 (d, 1H), 5.61 (m, 1H), 5.12 (m, 2H), 4.61 (d, 1H), 3.98 (s, 3H), 3.87 (s, 3H), 3.81 (s, 6H), 3.70 (t, 1H), 3.15 (m, 2H), 2.65 (m, 1H), 2.37 (m, 2H), 2.09 (m, 1H), 1.79 (m, 3H), 1.58 (t, 1H).

### Example 7: Catalyst preparation: 4-methylpyridine quininium chloride

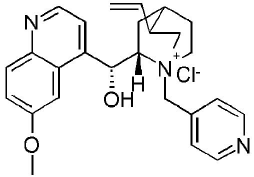

A solution of 4-chloromethyl-pyridine hydrochloride (0.41 g) and potassium bicarbonate (0.22 g) in toluene (9 ml) was stirred for 1 hour. Then quinine (0.50 g) was added to the suspension and the reaction mixture was heated at 80°C for 18 hours. The reaction mixture was poured in a mixture of water and diethyl ether, then filtrate, dried *in vacuo* at 35°C to afford the title product as a red solid (0.30 g). LCMS (method G) 0.76 min, M⁺ 516; ¹H NMR (400 MHz, CDCl₃) 8.71 (d, 1H), 8.65 (d, 2H), 7.95 (d, 1H), 7.74 (m, 3H), 7.29 (m, 1H), 6.58 (m, 1H), 6.28 (d, 1H), 5.55 (m, 1H), 5.10 (m, 3H), 3.94 (s, 3H), 3.54 (m, 1H), 3.38 (m, 2H), 3.00 (m, 2H), 2.61 (m, 1H), 2.31 (m, 1H), 2.05 (m, 2H), 1.74 (m, 2H), 1.41 (m, 1H).

### Example 8: Catalyst preparation: 2,6-dichloro-4-methyl-pyridine quininium chloride

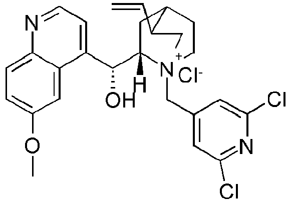

A solution of 2,6-dichloro-4-chloromethyl-pyridine (0.14 g) and quinine (0.18 g) in toluene (3 ml) was heated at 80°C for 18 hours. The reaction mixture was poured in diethyl ether and then filtrate to afford the title product as a white solid (0.10 g). LCMS (method G) 1.07 min, M⁺ 485; ¹H NMR (400 MHz, DMSO-*d6*) 8.81 (d, 1H), 8.05 (s, 2H), 8.03 (d, 1H), 7.73 (d, 1H), 7.48 (dd, 1H), 7.21 (d, 1H), 6.48 (s, 1H), 5.75 (m, 1H), 5.58 (m, 1H), 5.15 (d, 1H), 5.03 (d, 1H), 4.72 (d, 1H), 4.42 (m, 1H), 4.01 (s, 3H), 3.78 (m, 1H), 3.68 (m, 1H), 3.51 (t, 1H), 3.27 (m, 1H), 2.63 (m, 1H), 2.18 (m, 2H), 2.05 (m, 1H), 1.78 (m, 1H), 1.45 (m, 1H).

### Example 9: Chiral separation

### 4-[5-(3,5-Dichloro-phenyl)-5-trifluoromethyl-4,5-dihydro-isoxazol-3-yl]-2-methyl-N-thietan-3-yl-benzamide

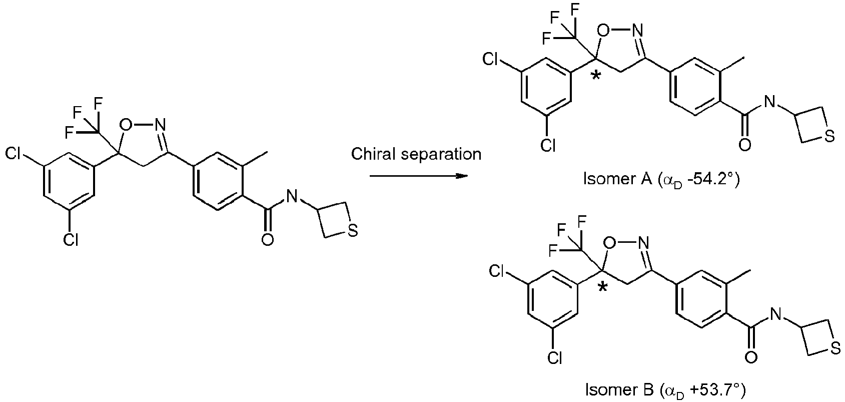

4-[5-(3,5-Dichloro-phenyl)-5-trifluoromethyl-4,5-dihydro-isoxazol-3-yl]-2-methyl-N-thietan-3-yl-benzamide (0.97 g; prepared as described in WO09080250) was separated through chiral phase preparative HPLC (Column: CHIRALPAK® IC 5 µm; Mobile Phase: 90/10 Carbon Dioxide/Ethanol; Flow Rate: 120 ml/min; Detection: 230 nm; Temperature: 25°C; Outlet Pressure: 150 bars) to afford 0.38 g of 4-[5-(3,5-dichloro-phenyl)-5-trifluoromethyl-4,5-dihydro-isoxazol-3-yl]-2-methyl-N-thietan-3-yl-benzamide ISOMER A (α_{D} -54.2°), and 0.35 g 4-[5-(3,5-dichloro-phenyl)-5-trifluoromethyl-4,5-dihydro-isoxazol-3-yl]-2-methyl-N-thietan-3-yl-benzamide ISOMER B (α_{D} +53.7°).
Similarly 4-[5-(3,5-dichloro-phenyl)-5-trifluoromethyl-4,5-dihydro-isoxazol-3-yl]-2-methyl-N-(cis-1-oxide-thietan-3-yl)-benzamide (1.98 g; prepared as described in WO09080250) was resolved to afford 0.81 g of 4-[5-(3,5-dichloro-phenyl)-5-trifluoromethyl-4,5-dihydro-isoxazol-3-yl]-2-methyl-N-(cis-1-oxide-thietan-3-yl)-benzamide ISOMER A (α_{D} -46.2°) and 0.83 g of ISOMER B (α_{D} +48.4°). Similarly 4-[5-(3,5-diehloro-phenyl)-5-trifluoromethyl-4,5-dihydro-isoxazol-3-yl]-2-methyl-N-(1,1-dioxo-thietan-3-yl)-benzamide (0.94 g ; prepared as described in WO09080250) was resolved to afford 0.44 g of 4-[5-(3,5-dichloro-phenyl)-5-trifluoromethyl-4,5-dihydro-isoxazol-3-yl]-2-methyl-N-(1,1-dioxo-thietan-3-yl)-benzamide ISOMER A (α_{D} -50.2°); and 0.45g of ISOMER B (α_{D} +51.1°).

### Example 10: Intermediate preparation

### 4-[(E)-3-(3,5-dichloro-phenyl)-4,4,4-trifluoro-but-2-enoyl]-2-methyl-N-thietan-3-yl-benzamide

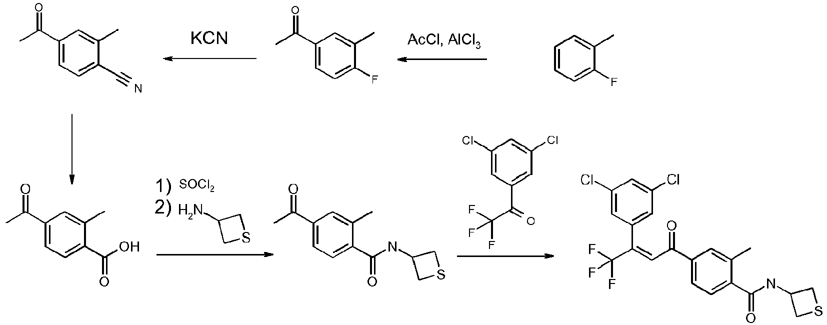

### Step A: 1-(4-Fluoro-3-methyl-phenyl)-ethanone

To a solution of 2-fluorotoluene (10.0 g, 90.8 mmol) and acetyl chloride (6.64 ml, 93.4 mmol) in dry CH₂Cl₂ (50 ml) was added portion-wise AlCl₃ (15.3 g, 115 mmol) (caution: vigorous exothermic reaction). The reaction was stirred at room temperature for 24 h. To complete the reaction, the mixture was heated to reflux for 2 h. The reaction mixture was quenched with saturated aqueous Na₂CO₃, diluted with Et₂O and filtered through a pad of Celite®. The organic phase was then separated, washed with saturated aqueous NaCl, dried over Na₂SO₄ and concentrated in vacuo. Purification by flash chromatography (SiO₂, Heptane: Ethylacetate 97:3 to 90:10) gave the title product as a pale brown oil (13.6 g). ¹H NMR (400 MHz, CDCl₃) 7.83 (d, 1H), 7.77-7.81 (m, 1H), 7.06 (t, 1H), 2.58 (s, 3H), 2.32 (d, 3H).

### Step B: 1-(4-Cyano-3-methyl-phenyl)-ethanone

1-(4-Fluoro-3-methyl-phenyl)-ethanone (2.02 g, 13.3 mmol) was dissolved in DMSO (4.5 ml) and then NaCN (0.81g, 16.6 mmol) was added. The resulting mixture was heated for 12h at 120 °C. The cooled reaction mixture was then diluted with CH₂Cl₂ and washed with water. The water phase was back-extracted with CH₂Cl₂ (3x). The combined organics were washed with H₂O (3x), saturated aqueous NaCl (1x), dried over Na₂SO₄, filtrated and concentrated under reduced pressure to afford an orange oil (2.1 g). ¹H NMR (400 MHz, CDCl₃): 7.89 (s, 1H), 7.83 (d, 1H), 7.71 (d, 1H), 2.63 (s, 3H), 2.62 (s, 3H).

### Step C: 4-Acetyl-2-methyl-benzoic acid

A pale yellow solution of 1-(4-Cyano-3-methyl-phenyl)-ethanone (4.54 g, 28.5 mmol) in AcOH (23 ml), H₂O (23 ml) and concentrated H₂SO₄ (23 ml) was refluxed for 5 h and stirred at room temperature overnight. The resulting orange slurry was adjusted to pH≈10 with 175ml NaOH 30%. The resulting orange solution was washed with AcOEt (2x250mL). The aqueous layer was acidified with concentrated HCI (100 ml) to pH=1 and then extracted with AcOEt (2x 200 ml). The organic phase was washed with H₂O (1x200mL), dried over Na₂SO₄, filtrated and evaporated under reduced pressure to give an orange solid (4.94g). The orange solid was dissolved in CH₂Cl₂ (150 ml) and water (100 ml) followed by 30% NaOH (50 ml) were successively added. The water phase was washed with CH₂Cl₂ (2x50mL). To the aqueous phase HCl conc. (90 ml) was added. The resulting precipitate was then filtered off and washed with water. The residue was then dissolved in CH₂Cl₂. The organic phase was dried over Na₂SO₄, filtrated and concentrated under reduced pressure to give a pale orange solid (3.3 g). LCMS (method F) RT = 1.21min, [M+H⁺]= 179; ¹H NMR (400 MHz, DMSO-d₆):13.2 (bs, 1H), 7.82-7.91 (m, 3H), 2.61 (s, 3H), 2.58 (s, 3H).

### Step D: 4-Acetyl-2-methyl-N-thietan-3-yl-benzamide

4-Acetyl-2-methyl-benzoic acid (70 g, 39.3 mmol) was dissolved in CH₂Cl₂ (700 ml) and oxalyl chloride (109 g, 86 mmol) DMF (1 mL) were successively added. The reaction mixture was stirred overnight at room temperature. The mixture was then concentrated in vacuo to afford a brown oil (82 g). The acid chloride was redissolved in CH₂Cl₂ (500 ml) and cooled to 0 °C. A solution of hydro thietane-3-yl amine trifluoroacetate (prepared as described in WO09080250, 92 g, 34 mmol) and Et₃N (200 g, 200 mmol) in CH₂Cl₂ (400mL) was then added dropwise. The resulting mixture was stirred overnight at room temperature. NaOH 10% was added. The mixture was washed successively with water and with 2N HCl (300 ml). The organic layer was dried over Na₂SO₄ and evaporated in vacuo to afford brown oil (132 g). Purification by silica gel chromatography (CH₂Cl₂: Ethyl acetate 2:1) afforded the title compound as a yellow solid (34 g). LCMS (method F) RT = 1.21min, [M+H⁺]= 250. ¹H NMR (400 MHz, CDCl₃): 7.82 (s, 1H), 7.79 (d, 1H), 7.43 (d, 1H), 6.21 (bs, 1H), 5.43 (q, 1H), 3.50 (t, 2H), 3.39 (t, 2H), 2.61 (s, 3H), 2.49 (s, 3H).

### Step E: 4-[(E)-3-(3,5-dichloro-phenyl)-4,4,4-trifluoro-but-2-enoyl]-2-methyl-N-thietan-3-yl-benzamide

4-Acetyl-2-methyl-N-thietan-3-yl-benzamide (0.25 g, 1 mmol), 3,5 dichloro 2,2,2 trifluoroacetophenone (0.24 g, 1 mmol) potassium carbonate (138 mg, 1 mmol) and triethylamine (10 mg, 0.1 mmol) were dissolved in 1,2 dichloroethane (2.5 ml). The resulting mixture was refluxed for 3h. The reaction mixture was cooled to room temperature and saturated aqueous NH₄Cl was added. The mixture was extracted with CH₂Cl₂ (3x50 ml). The combined organic extracts were dried over Na₂SO₄ and concentrated in vacuo to afford a brown solid (0.51 g). Purification by silica gel chromatography (Heptane: Ethyl acetate 2:1) afforded the title compound as a yellow solid (0.33 g). LCMS (method F) RT = 2.09 min, [M+H⁺]= 474, 476, 477. ¹H NMR (400 MHz, CDCl₃): 7.65-7.67 (m, 2H), 7.40-7.43 (m, 1H), 7.36-7.37 (m, 1H), 7.34 (t, 1H), 7.15 (s, 2H), 6.17 (d, 1H), 5.42 (q, 1H), 3.50 (t, 2H), 3.38 (t, 2H), 2.46 (s, 3H).

### Example 11: Intermediate preparation

### 4-[(E)-3-(3,5-dichloro-phenyl)-4,4,4-trifluoro-but-2-enoyl]-2-methyl-N-(cis-1-oxide-thietan-3-yl)-benzamide

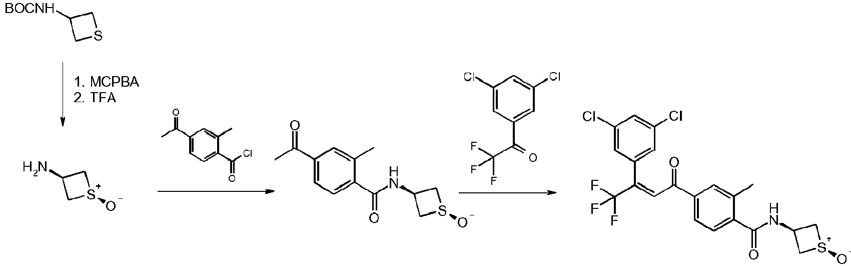

### Step A: cis-1-oxide-thietan-3ylamine

Thietan-3-yl-carbamic acid tert-butyl ester (prepared as described in WO09080250, 5.0 g, 26.4 mmol) was dissolved in dichloromethane (400 ml), followed by the addition of NaHCO₃ as a solution in water (150 ml). The reaction mixture was cooled to 0°C and under strong stirring, 3-chloroperbenzoic acid (6.5 g, 264.4 mmol) was added dropwise as a solution in dichloromethane (100 ml) within 30 minutes. The reaction mixture temperature did not go over 4°C during the addition. Dichloromethane (50 ml) was added for washing, and the reaction mixture was stirred at around 2-3 °C during 2 hours. Immiscible phases were separated, and the aqueous phase was extracted with dichloromethane (3x). The combined organic phases were collected, dried over Na₂SO₄, filtered and concentrated in vacuo to give a white solid (5.3 g). This crude product was purified by silica gel chromatography (ethyl acetate / heptane / methanol 75:25:0 - 100:0:0 - 80:0:20) and first afforded *cis*-1-oxide-thietan-3-yl carbamic acid tert-butyl ester (2.09 g), then a mixture of both *cis*-1-oxide-thietan-3-yl carbamic acid tert-butyl ester and *trans*-1-oxide-thietan-3yl carbamic acid tert-butyl ester (1.15 g), and finally 1,1-dioxide-thietan-3-yl carbamic acid tert-butyl ester (0.92 g).

To a solution of *cis*-1-oxide-thietan-3-yl carbamic acid tert-butyl ester (10 g, 5.3 mmol) at 0 °C was added trifluoroacetic acid (6.5 g, 5.7 mmol). The resulting mixture was stirred at 0 °C overnight and was then concentrated in vacuo to afford the title salt as a yellow oil (12.1 g), which was used without further purification in the next step.¹H NMR (400 MHz, DMSO-d₆): 12.1 (bs, 1H), 8.73 (bs, 2H), 4.50-4.57 (m, 2H), 4.35-4.43 (m, 2H), 4.07-4.16 (m, 1H).

### Step B: 4-Acetyl-2-methyl-N-(cis-1-oxide-thietan-3-yl)-benzamide

To a solution of *cis*-1-oxide-thietan-3ylamine (2.2 g, 10 mmol) and 4-Acetyl-2-methylbenzoic acid chloride (2 g, 10 mmol) in CH₂Cl₂ (21 ml) was added triethylamine (5.05 g, 50 mmol) at 0 °C. The resulting mixture was stirred at room temperature overnight. The mixture was quenched with saturated aqueous NH₄Cl. The aqueous phase was extracted with CH₂Cl₂ (3x50mL). The combined organic phases were washed with saturated aqueous Na₂CO₃, dried over Na₂SO₄ and concentrated in vacuo to afford the title compound as an amber solid (2.2 g). ¹H NMR (400 MHz, CDCl₃): 7.80 (s, 1H), 7.77 (d, 1H), 7.44 (d, 1H), 6.66 (d, 1H), 4.69 (q, 1H), 4.17-4.32 (m, 2H), 3.29 (dt, 2H), 2.61 (s, 3H), 2.48 (s,3H).

### Step C: 4-[(E)-3-(3,5-dichloro-phenyl)-4,4,4-trifluoro-but-2-enoyl]-2-methyl-N-(cis-1-oxide-thietan-3-yl-benzamide

4-Acetyl-2-methyl-N-(cis-1-dioxide-thietan-3-yl)-benzamide (1.0 g, 3.7 mmol), (3,5 dichloro 2,2,2 trifluoroacetophenone (0.9 g, 3.7 mmol) potassium carbonate (0.52 g, 3.7 mmol) and triethylamine (40 mg, 0.37 mmol) were dissolved in 1,2 dichloroethane (11 ml). The resulting mixture was refluxed overnight. The reaction mixture was cooled to room temperature and was diluted with warm (40°C) ethyl acetate, water was added, and the separated aqueous layer was extacted with warm EtOAc, the organic layer was washed brine with dried with Na₂SO₄ and concentrated in vacuo to afford an off white solid (1.8 g). LCMS (method F) RT = 1.89 min, [M+H⁺]= 490, 492. ¹H NMR (400 MHz, CDCl₃): 7.66-7.68 (m, 2H), 7.42-7.45 (m, 2H), 7.36-7.38 (m, 1H), 7.34 (t, 1H), 7.15 (d, 2H), 6.54 (d, 1H), 4.64-4.75 (m, 1H), 4.17-4.24 (m, 2H), 3.25 (dt, 2H), 2.46 (s, 3H).

### Example 12: Intermediate preparation

### 4-[(E)-3-(3,5-dichloro-phenyl)-4,4,4-trifluoro-but-2-enoyl]-2-methyl-N-(1,1-dioxide-thietan-3-yl)-benzamide

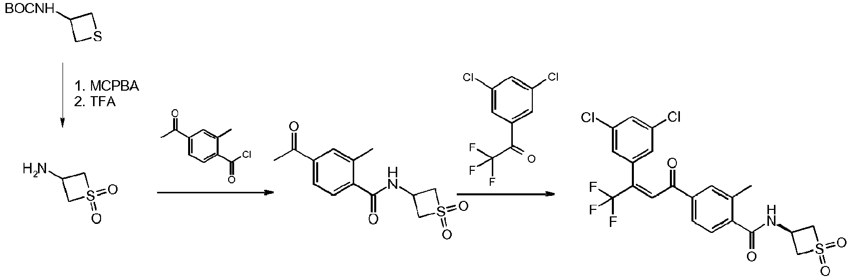

### Step A: 1,1-dioxide-thietan-3ylamine

Thietan-3-yl-carbamic acid tert-butyl ester (prepared as described in WO09080250, 5.0 g, 26.4 mmol) was dissolved in dichloromethane (400 ml), followed by the addition of NaHCO₃ as a solution in water (150 ml). The reaction mixture was cooled to 0°C and under a strong stirring, 3-chloroperbenzoic acid (6.5 g, 264.4 mmol) was added dropwise as a solution in dichloromethane (100 ml) within 30 minutes. The reaction mixture temperature did not go over 4°C during the addition. Dichloromethane (50 ml) was added for washing, and the reaction mixture was stirred at around 2-3 °C during 2 hours. Immiscible phases were separated, and the aqueous phase was extracted with dichloromethane (3x). The combined organic phases were collected, dried over Na₂SO₄, filtered and concentrated in vacuo to give a white solid (5.3 g). This crude product was purified by silica gel chromatography (ethyl acetate / heptane / methanol 75:25:0 - 100:0:0 - 80:0:20) and first afforded *cis*-1-oxide-thietan-3-yl carbamic acid tert-butyl ester (2.09 g), then a mixture of both *cis*-1-oxide-thietan-3-yl carbamic acid tert-butyl ester and *trans*-1-oxide-thietan-3yl carbamic acid tert-butyl ester (1.15 g), and finally 1,1-dioxide-thietan-3-yl carbamic acid tert-butyl ester (0.92 g).

To a solution of 1,1-dioxide-thietan-3-yl carbamic acid tert-butyl ester (1.0 g, 4.5 mmol) was added trifluoroacetic acid (1 ml, 12.9 mmol). The resulting mixture was stirred for 2h at room temperature. Another injection of trifluoroacetic acid (3 ml) was performed after 2h followed by a last addition of trifluoroacetic acid (5 ml) after 5h. Solvents were then removed in vacuo. The resulting gum was washed with Et₂O (3x) and the resulting solid was dried in vacuo to afford the title compound as a white powder (0.92 g). ¹H NMR (400 MHz, DMSO-d₆): 8.31 (bs, 2H), 4.01 (t, 2H), 3.70-3.81 (bs, 1H), 3.27 (t, 2H).

### Step B: 4-Acetyl-2-methyl-N-(1,1-dioxide-thietan-3-yl)-benzamide

To a solution of 4-acetyl-2-methyl benzoylchloride (4.6 g, 23 mmol) and Et₃N (2.53 g, 25mmol) in CH₂Cl₂ (100 ml) cooled to 0 °C was added hydro thietane-3-yl amine 1,1-dioxide trifluoroacetate (5.0 g, 21 mmol). The resulting mixture was stirred overnight at room temperature. The mixture was washed successively with water and extracted with CH₂Cl₂ (2x100 ml). The combined organic phases were washed with 2N HCl. The organic layer was dried over Na₂SO₄ and evaporated in vacuo to afford yellow oil (5.9 g). LCMS (method F) RT = 1.05 min, [M+H⁺]=282. ¹H NMR (400 MHz, CDCl₃): 7.82 (s, 1H), 7.78 (d, 1H), 7.46 (d, 1H), 6.63 (d, 1H), 4.86-4.94 (m, 1H), 4.59-4.67 (m, 2H), 4.03-4.08 (m, 2H), 2.62 (s, 3H), 2.50 (s, 3H).

### Step C: 4-[(E)-3-(3,5-dichloro-phenyl)-4,4,4-trifluoro-but-2-enoyl]-2-methyl-N-(1,1-dioxide-thietan-3-yl-benzamide

4-Acetyl-2-methyl-N-(1,1-dioxide-thietan-3-yl)-benzamide (0.28 g, 1 mmol), (3,5 dichloro 2,2,2 trifuloroacetophenone (0.24 g, 1 mmol) potassium carbonate (138 mg, 1 mmol) and triethylamine (10 mg, 0.1 mmol) were dissolved in 1,2 dichloroethane (2.5 ml). The resulting mixture was refluxed for 3h. The reaction mixture was cooled to room temperature and saturated aqueous NH₄Cl was added. The mixture was extracted with CH₂Cl₂ (3x50 ml). The combined organic extracts were dried over Na₂SO₄ and concentrated in vacuo to afford a brown solid (0.52 g). Purification by silica gel chromatography (heptane: Ethyl acetate 2:1) afforded the title compound as a yellow solid (0.33 g). The solid was suspended in heptane (10 ml) and stirred for 1h at 40 °C. It was then cooled to 0 °C, filtered, washed with 1 ml cold TBME. After being dried under vacuum the title compound was obtained as a white solid (0.50 g). ¹H NMR (400 MHz, CDCl₃): 7.65-7.69 (m, 2H), 7.45-7.47 (m, 2H), 7.43-7.45 (m, 2H), 7.36-7.37 (m, 1H), 7.35 (t, 1H), 7.15 (d, 2H), 6.49 (d, 1H), 4.86-4.94 (m, 1H), 4.59-4.67 (m, 2H), 3.99-4.05 (m, 2H), 2.48 (s, 3H).

### Example 13:

### 4-[5-(3,5-Dichloro-phenyl)-5-trifluoromethyl-4,5-dihydro-isoxazol-3-yl]-2-methyl-N-thietan-3-yl-benzamide

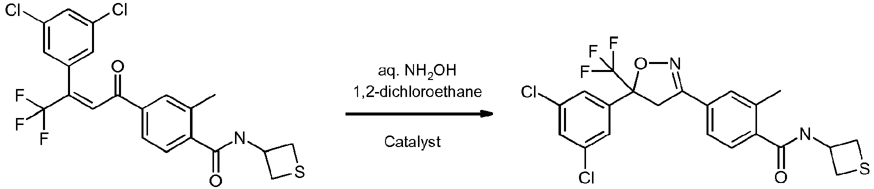

### Catalyst:

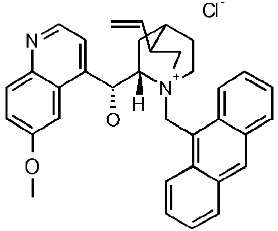

A pre-cooled solution of 5M sodium hydroxide (0.25 ml) was added to a solution of hydroxylamine (50% in water, 37 mg) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and anthracenyl-methyl quininium chloride (20 mg) (catalyst preparation Example 1) in 1,2-dichloroethane (1.5 ml) cooled in an ice-acetone bath. The mixture was stirred rapidly at ca -25 °C for 5 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 5%) to give the title compound (90 mg) as a white solid. ¹H NMR (400 MHz, CDCl₃) 7.52 (m, 4H), 7.42 (m, 2H), 6.18 (d, 1H), 5.41 (q, 1H), 4.09 (d, 1H), 3.67 (d, 1H), 3.50 (t, 2H), 3.39 (t, 2H), 2.47 (s, 3H). ¹⁹F NMR (376 MHz, CDCl₃) -79.51 (s, 3F). The product was analysed by chiral HPLC (method I) and compared to reference samples of ISOMERS A and B of the title compound (chiral separation Example): the product contained ISOMER B (retention time 8.60 minutes) as the major product (40%ee).

### Example 14

### 4-[5-(3,5-Dichloro-phenyl)-5-trifluoromethyl-4,5-dihydro-isoxazol-3-yl]-2-methyl-N-thietan-3-yl-benzamide

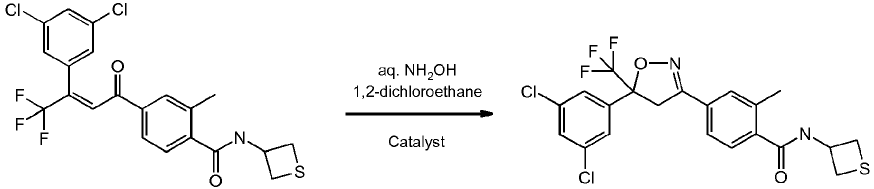

### Catalyst:

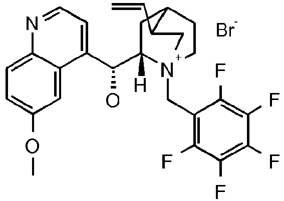

A pre-cooled solution of 5M sodium hydroxide (0.25 ml) was added to a solution of hydroxylamine (50% in water, 37 mg) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 2,3,4,5,6-pentafluorophenyl-methyl quininium bromide (20 mg) (catalyst preparation Example 3) in 1,2-dichloroethane (1.5 ml) cooled by a cryostat in an acetone bath. The mixture was stirred rapidly at ca -15 °C for 3 hours. The reaction mixture was diluted with dichloromethane, passed through an isolute phase separating cartridge and concentrated *in vacuo* to leave a yellow oil. This residue was purified by chromatography on silica gel (eluent: heptane / ethyl acetate 20%) to give the title compound (100 mg) as a white solid. ¹H NMR (400 MHz, CDCl₃) 7.52 (m, 4H), 7.42 (m, 2H), 6.18 (d, 1H), 5.41 (q, 1H), 4.09 (d, 1H), 3.67 (d, 1H), 3.50 (t, 2H), 3.39 (t, 2H), 2.47 (s, 3H). ¹⁹F NMR (376 MHz, CDCl₃)-79.51 (s, 3F). The product was analysed by chiral HPLC (method I) and compared to reference samples of ISOMERS A and B of the title compound (Chiral separation Example): the product contained ISOMER B (retention time 8.80 min) as the major product (63%ee).

### Example 15

### 4-[5-(3,5-Dichloro-phenyl)-5-trifluoromethyl-4,5-dihydro-isoxazol-3-yl]-2-methyl-N-thietan-3-yl-benzamide

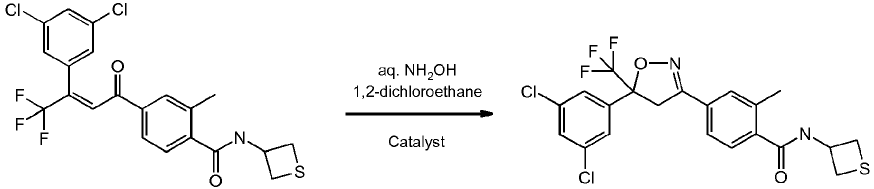

### Catalyst:

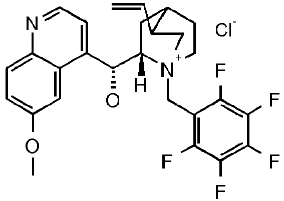

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 2,3,4,5,6-pentafluorophenyl-methyl quininium chloride (21 mg) (catalyst preparation Example 4) in 1,2-dichloroethane (1.5 ml) cooled in an ice bath. The mixture was stirred rapidly at ca 0°C for 2 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (75 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (75%ee).

### Example 16

### 4-[5-(3,5-Dichloro-phenyl)-5-trifluoromethyl-4,5-dihydro-isoxazol-3-yl]-2-methyl-N-thietan-3-yl-benzamide

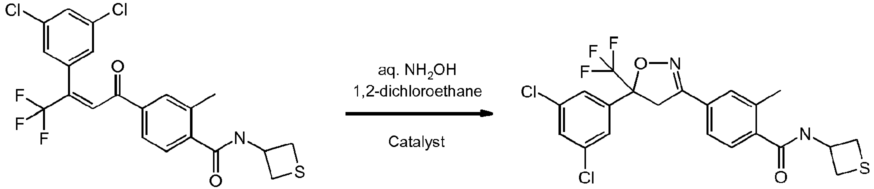

### Catalyst:

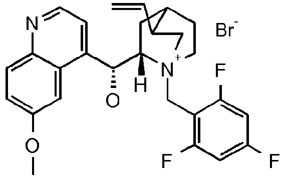

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 2,4,6-trifluorophenyl-methyl quininium bromide (19 mg) (catalyst preparation Example 2) in 1,2-dichloroethane (1.5 ml) cooled by a cryostat in an acetone bath. The mixture was stirred rapidly at ca -15°C for 3 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (100 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃)-79.51 (s, 3F).The product was analysed by chiral HPLC (method I) and compared to reference samples of ISOMERS A and B of the title compound (chiral separation Example): the product contained ISOMER B (retention time 8.40 min) as the major product (49%ee).

### Example 17

### 4-[5-(3,5-Dichloro-phenyl)-5-trifluoromethyl-4,5-dihydro-isoxazol-3-yl]-2-methyl-N-thietan-3-yl-benzamide

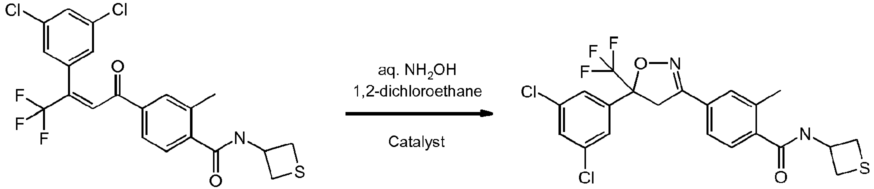

### Catalyst:

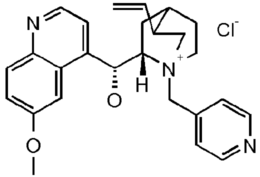

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 4-pyridine-methyl-quininium chloride (15 mg) (catalyst preparation Example 7) in 1,2-dichloroethane (1.5 mL) cooled by a cryostat in an acetone bath. The mixture was stirred rapidly at ca - 15°C for 3 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (54 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (51%ee).

### Example 18

### 4-[5-(3,5-Dichloro-phenyl)-5-trifluoromethyl-4,5-dihydro-isoxazol-3-yl]-2-methyl-N-thietan-3-yl-benzamide

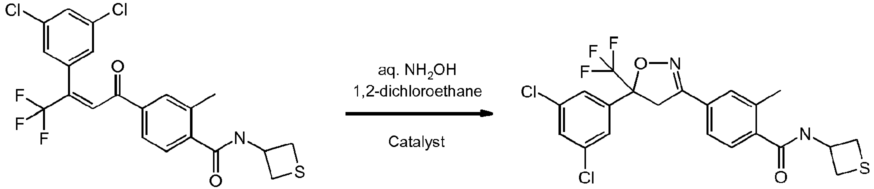

### Catalyst:

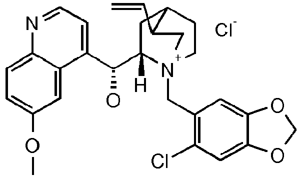

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 6-piperonyl quininium chloride (18 mg) (catalyst preparation Example 5) in 1,2-dichloroethane (1.5 ml) cooled by a cryostat in an acetone bath. The mixture was stirred rapidly at ca -15°C for 3 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (100 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (51%ee).

### Example 19

### 4-[5-(3,5-Dichloro-phenyl)-5-trifluoromethyl-4,5-dihydro-isoxazol-3-yl]-2-methyl-N-thietan-3-yl-benzamide

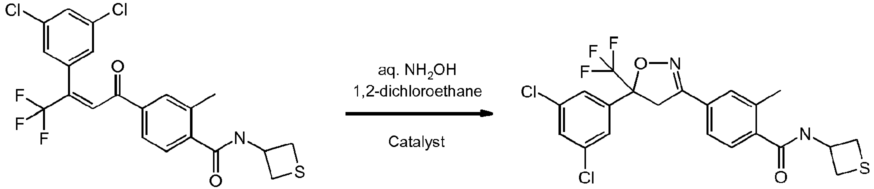

### Catalyst:

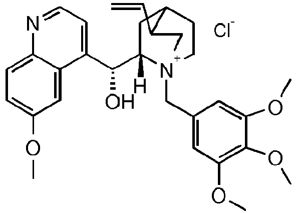

A pre-cooled solution of 5M sodium hydroxide (0.25 ml) was added to a solution of hydroxylamine (50% in water, 0.075 mL) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 3,4,5-trimethoxyphenyl-methyl quininium chloride (22 mg) (catalyst preparation Example 6) in 1,2-dichloroethane (1.5 ml) cooled in an ice bath. The mixture was stirred rapidly at ca 0°C for 2 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (67 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (77%ee).

### Example 20

### 4-[5-(3,5-Dichloro-phenyl)-5-trifluoromethyl-4,5-dihydro-isoxazol-3-yl]-2-methyl-N-thietan-3-yl-benzamide

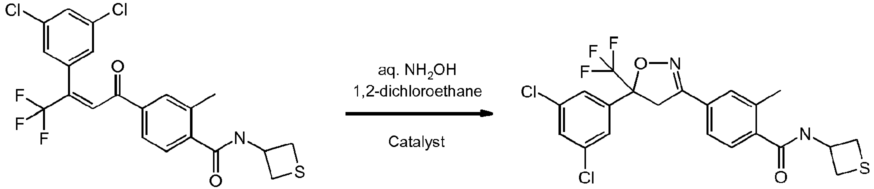

### Catalyst:

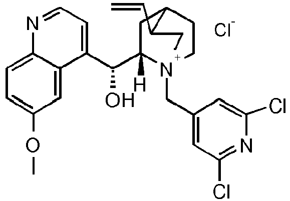

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 4-[(E)-3-(3,5-dichloro-phenyl)-4,4,4-trifluoro-but-2-enoyl]-2-methyl-N-thietan-3-yl-benzamide (100 mg) and 2,6-dichloropyridine-methyl quininium chloride (21 mg) (catalyst preparation Example 8) in 1,2-dichloroethane (1.5 ml) cooled in an ice bath. The mixture was stirred rapidly at ca 0°C for 2 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound (70 mg) as a white solid. ¹H NMR (400 MHz, CDCl₃) 7.52 (m, 4H), 7.42 (m, 2H), 6.18 (d, 10H), 5.41 (q, 1H), 4.09 (d, 1H), 3.67 (d, 1H), 3.50 (t, 1H), 3.39 (t, 1H), 2.47 (s, 3H). ¹⁹F NMR (376 MHz, CDCl₃) -79.51 (s, 3F).The product was analysed by chiral HPLC (method I) and compared to reference samples of ISOMERS A and B of the title compound (chiral separation Example): the product contained ISOMER B (retention time 8.80 min) as the major product (66%ee).

### Example 21

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

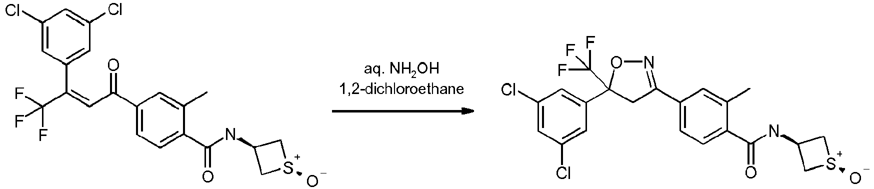

### Catalyst:

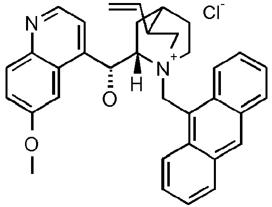

A pre-cooled solution of 5M sodium hydroxide (0.25 ml) was added to a solution of hydroxylamine (50% in water, 37 mg) at 5 °C (ice bath). The solution was stirred for 15 min at 5 °C then added to a vigorously stirred solution of 1, 4-[(E)-3-(3,5-dichloro-phenyl)-4,4,4-trifluoro-but-2-enoyl]-2-methyl-N-(cis-1-oxide-thietan-3-yl)-benzamide (100 mg) and anthracenyl-methyl quininium chloride (24 mg) (catalyst preparation Example 1) in 1,2-dichloroethane (1.5 ml) cooled in an ice bath. The mixture was stirred rapidly at ca 0 °C for 1 hour. The reaction mixture was diluted with dichloromethane, passed through an isolute phase separating cartridge and concentrated *in vacuo* to leave a yellow oil. This residue was purified by chromatography on silica gel (eluent: ethyl acetate / ethanol 5%) to give the title compound (90 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (S, 3F). The product was analyzed by chiral HPLC (method J) and compared to reference samples of ISOMERS A and B of the title compound (chiral separation Example): the product contained ISOMER B (retention time 17.6 min) as the major product (37%ee).

### Example 22

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

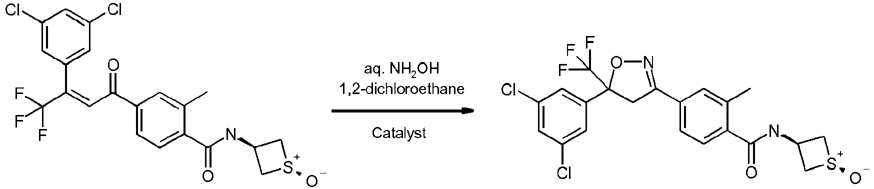

### Catalyst:

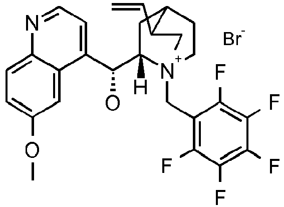

A pre-cooled solution of 5M sodium hydroxide (0.25 ml) was added to a solution of hydroxylamine (50% in water, 37 mg) at 5°C (ice bath). The solution was stirred for 15 min at 5°C then added to a vigorously stirred solution of 1, 4-[(E)-3-(3,5-dichlorophenyl)-4,4,4-trifluoro-but-2-enoyl]-2-methyl-N-(*cis*-1-oxide-thietan-3-yl)-benzamide (100 mg) and 2,3,4,5,6-pentafluorophenyl-methyl quininium bromide (24 mg) (catalyst preparation Example 3) in 1,2-dichloroethane (1.5 ml) cooled in an ice bath. The mixture was stirred rapidly at ca 0 °C for 1 hour. The reaction mixture was diluted with dichloromethane, passed through an isolute phase separating cartridge and concentrated *in vacuo* to leave a yellow oil. This residue was purified by chromatography on silica gel (eluent: ethyl acetate / ethanol 5%) to give the title compound (34 mg) as a white solid. ¹H NMR (400 MHz, CDCl₃) 7.48 (m, 6H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.1 min) as the major product (87%ee).

### Example 23

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

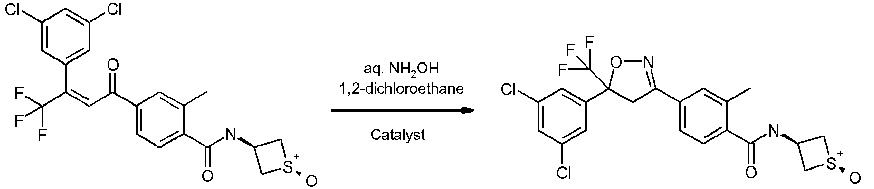

### Catalyst:

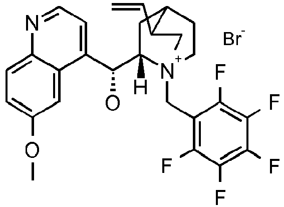

A pre-cooled solution of 5M sodium hydroxide (0.45 ml) was added to a solution of hydroxylamine (50% in water, 67 mg) at 5°C (ice bath). The solution was stirred for 15 min at 5°C then added to a vigorously stirred solution of 1, 4-[(E)-3-(3,5-dichlorophenyl)-4,4,4-trifluoro-but-2-enoyl]-2-methyl-N-(*cis*-1-oxide-thietan-3-yl)-benzamide (500 mg) and 2,3,4,5,6-pentafluorophenyl-methyl quininium bromide (120 mg) (catalyst preparation Example 3) in 1,2-dichloroethane (7 ml) cooled in an ice bath. The mixture was stirred rapidly at ca 0 °C for 2 hour. The reaction mixture was diluted with dichloromethane, passed through an isolute phase separating cartridge and concentrated *in vacuo* to leave a yellow oil. This residue was purified by chromatography on silica gel (eluent: ethyl acetate / ethanol 5%) to give the title compound (450 mg) as a white solid. ¹H NMR (400 MHz, CDCl₃) 7.48 (m, 6H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.0 minutes) as the major product (90%ee). Optical rotation was measured on a 3.1 mg sample dissolved in 1ml ethanol using a 1 dm path length. [α_{D}] +58.7°.

### Example 24

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

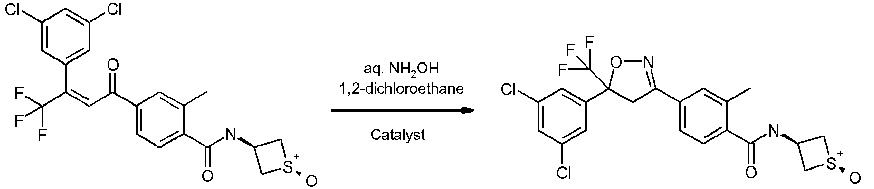

### Catalyst:

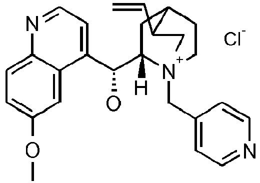

A pre-cooled solution of 5M sodium hydroxide (0.25 ml) was added to a solution of hydroxylamine (50% in water, 0.075 ml) at 5°C (ice bath). The solution was stirred for 15 min at 5°C then added to a vigorously stirred solution of 1, 4-[(E)-3-(3,5-dichlorophenyl)-4,4,4-trifluoro-but-2-enoyl]-2-methyl-N-(*cis*-1-oxide-thietan-3-yl)-benzamide (100 mg) and 4-pyridine-methyl-quininium chloride (15 mg) (catalyst preparation Example 7) in 1,2-dichloroethane (1.5 ml) cooled in an ice bath. The mixture was stirred rapidly at ca 0°C for 1 hour. The reaction mixture was diluted with dichloromethane, passed through an isolute phase separating cartridge and concentrated *in vacuo* to leave yellow oil. This residue was purified by chromatography on silica gel (eluent: ethyl acetate / ethanol 5%) to give the title compound (100 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.14 min) as the major product (59%ee).

### Example 25

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

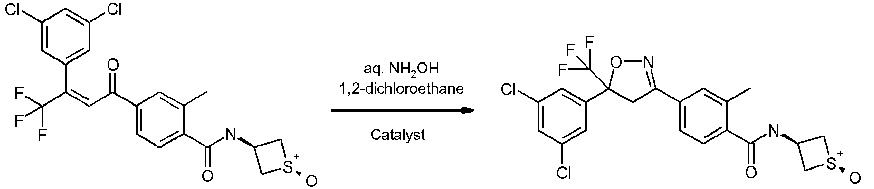

### Catalyst:

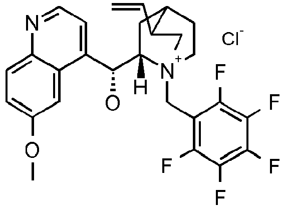

A pre-cooled solution of 5M sodium hydroxide (0.09 ml) was added to a solution of hydroxylamine (50% in water, 0.026 ml) at 5°C (ice bath). The solution was stirred for 15 min at 5°C then added to a vigorously stirred solution of 1, 4-[(E)-3-(3,5-dichlorophenyl)-4,4,4-trifluoro-but-2-enoyl]-2-methyl-N-(*cis*-1-oxide-thietan-3-yl)-benzamide (100 mg) and 2,3,4,5,6-pentafluorophenyl-methyl quininium chloride (21 mg) (catalyst preparation Example 4) in 1,2-dichloroethane (1.5 ml) cooled in an ice bath. The mixture was stirred rapidly at ca 0°C for 3 hour. The reaction mixture was diluted with dichloromethane, passed through an isolute phase separating cartridge and concentrated *in vacuo* to leave yellow oil. This residue was purified by chromatography on silica gel (eluent: ethyl acetate / ethanol 5%) to give the title compound (85 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.05 min) as the major product (73%ee).

### Example 26

### 4-[5-(3,5-Dichloro-phenyl)-5-trifluoromethyl-4,5-dihydro-isoxazol-3-yl]-2-methyl-N-(cis-1-oxide-thietan-3-yl)-benzamide

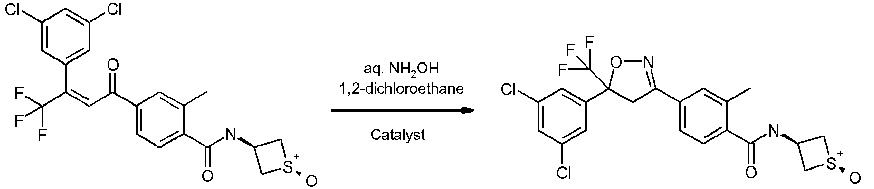

### Catalyst:

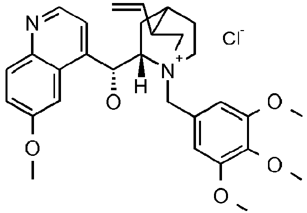

A pre-cooled solution of 5M sodium hydroxide (0.09 ml) was added to a solution of hydroxylamine (50% in water, 0.026 mL) at 5°C (ice bath). The solution was stirred for 15 min at 5°C then added to a vigorously stirred solution of 1, 4-[(E)-3-(3,5-dichloro-phenyl)-4,4,4-trifluoro-but-2-enoyl]-2-methyl-N-(cis-1-oxide-thietan-3-yl)-benzamide (100 mg) and 3,4,5 -trimethoxyphenyl-methyl quininium chloride (22 mg) (catalyst preparation Example 6) in 1,2-dichloroethane (1.5 ml) cooled in an ice bath. The mixture was stirred rapidly at ca 0°C for 3 hour. The reaction mixture was diluted with dichloromethane, passed through an isolute phase separating cartridge and concentrated *in vacuo* to leave yellow oil. This residue was purified by chromatography on silica gel (eluent: ethyl acetate / ethanol 5%) to give the title compound (90 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 17.36 min) as the major product (70%ee).

### Example 27

### 4-[5-(3,5-Dichloro-phenyl)-5-trifluoromethyl-4,5-dihydro-isoxazol-3-yl]-2-methyl-N-(1,1-dioxo-thietan-3-yl)-benzamide

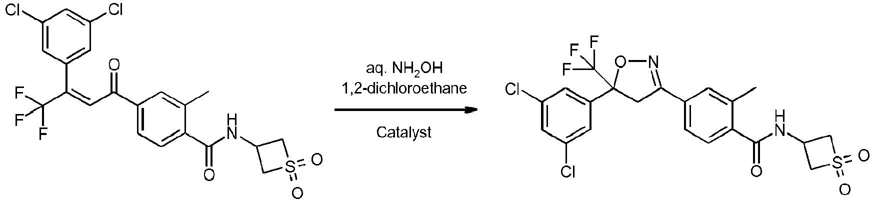

### Catalyst:

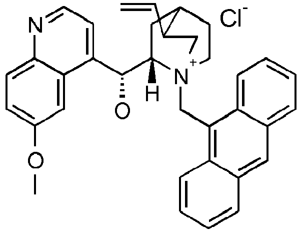

Sodium hydroxide (35 mg, 0.88 mmol) was dissolved in water (0.20 ml), the mixture was cooled to 0 °C in an ice bath and a solution of hydroxyl amine (50% in water, 0.49 ml) was added dropwise. The mixture was stirred for 5-10 min, then added dropwise to a stirred solution of 1, 4-[(E)-3-(3,5-dichloro-phenyl)-4,4,4-trifluoro-but-2-enoyl]-2-methyl-N-(1,1-dioxide-thietan-3-yl)-benzamide (0.202g, 0.40 mmol) and anthracenyl-methyl quininium chloride (66 mg) (catalyst preparation Example 1) in 1,2-dichloroethane (4 ml) at -15°C, the mixture was stirred overnight. LC shows consumption of starting material and formation of product. The mixture was diluted with dichloromethane and quenched with dilute hydrochloric acid (2M, 1mL). The mixture was passed through an isolute phase separating cartridge and concentrated *in vacuo,* the residue was purified by chromatography on silica gel (eluent: cyclohexane / ethyl acetate 50%) to give the title compound (208 mg) as a colourless solid. ¹H NMR (400 MHz, CDCl₃) 7.52 (m, 4H), 7.42 (m, 2H), 6.79 (br. d, 1H), 4.85 (m, 1H), 4.58 (m, 2H), 4.05 (m, 3H), 3.71 (d, 1H), 2.44 (s, 3H). The product was analysed by chiral HPLC (method J) and compared to reference samples of ISOMERS A and B of the title compound (chiral separation Example): the product contained ISOMER B (retention time 21.5 minutes) as the major product (32.5 %ee).

### Example 28

### 4-[5-(3,5-Dichloro-phenyl)-5-trifluoromethyl-4,5-dihydro-isoxazol-3-yl]-2-methyl-N-(1,1-dioxo-thietan-3-yl)-benzamide

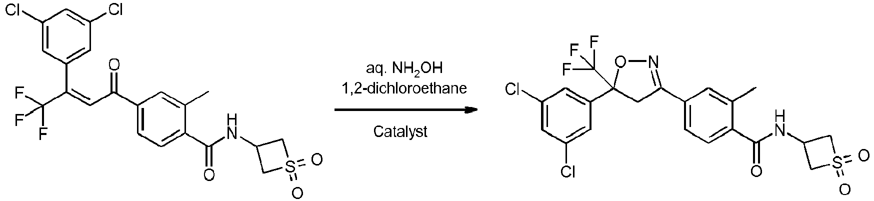

### Catalyst:

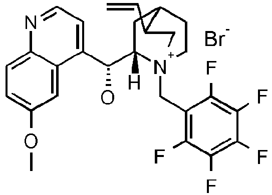

A solution of hydroxylamine (50% in water, 0.024 ml) was added to a pre-cooled (ice-water bath) solution of sodium hydroxide (5M, 0.088 ml), the mixture was stirred for one minute then added to a pre-cooled (ice-water bath)solution of 1, 4-[(E)-3-(3,5-dichlorophenyl)-4,4,4-trifluoro-but-2-enoyl]-2-methyl-N-(*1*,1-dioxo-thietan-3-yl)-benzamide and 2,3,4,5,6-pentafluorophenyl-methyl quininium bromide (catalyst preparation Example 3) in 1,2-dichloroethane (2 ml). The mixture was stirred for 3 hr at 0-5 °C. A small sample was taken and ¹⁹F NMR (376 MHz, CDCl₃) shows formation of product and consumption of starting material. The mixture was diluted with dichloromethane and quenched with dilute hydrochloric acid (2M, 1 ml). The mixture was passed through an isolute phase separating cartridge and concentrated *in vacuo,* the residue was dissolved in cyclohexane, filtered and *concentrated in vacuo.* The residue was purified by chromatography on silica gel (eluent: cyclohexane / ethyl acetate 15-50%) to give the title compound (55 mg) as an off white solid. ¹H NMR (400 MHz, CDCl₃) 7.52 (m, 4H), 7.42 (m, 2H), 6.75 (br. d, 1H), 4.88 (m, 1H), 4.62 (m, 2H), 4.05 (m, 3H), 3.70 (d, 1H), 2.47 (s, 3H); ¹⁹F NMR (376 MHz, CDCl₃) 79.52 (s, 3F) :The product was analysed by chiral HPLC (method J) and compared to reference samples of ISOMERS A and B of the title compound (chiral separation Example): the product contained ISOMER B (retention time 21.6 minutes) as the major product (67 % ee).

### Example 29

### 4-[5-(3,5-Dichloro-phenyl)-5-trifluoromethyl-4,5-dihydro-isoxazol-3-yl]-2-methyl-N-(1,1-dioxo-thietan-3-yl)-benzamide

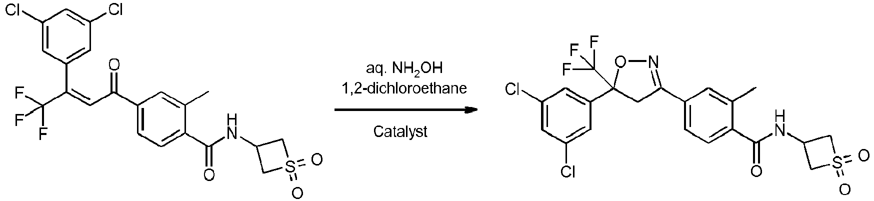

### Catalyst:

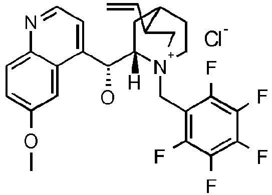

A pre-cooled solution of 5M sodium hydroxide (0.09 ml) was added to a solution of hydroxylamine (50% in water, 0.026 ml) at 5°C (ice bath). The solution was stirred for 15 min at 5°C then added to a vigorously stirred solution of 1, 4-[(E)-3-(3,5-dichlorophenyl)-4,4,4-trifluoro-but-2-enoyl]-2-methyl-N-(*cis*-1-oxide-thietan-3-yl)-benzamide (100 mg) and 2,3,4,5,6-pentafluorophenyl-methyl quininium chloride (21 mg) (catalyst preparation Example 4) in 1,2-dichloroethane (1.5 ml) cooled in an ice bath. The mixture was stirred rapidly at ca 0°C for 3 hour. The reaction mixture was diluted with dichloromethane, passed through an isolute phase separating cartridge and concentrated *in vacuo* to leave yellow oil. This residue was purified by chromatography on silica gel (eluent: *n*-heptane / ethyl acetate 40%) to give the title compound (58 mg) as a white solid. ¹H NMR (400 MHz, CDCl₃) 7.52 (m, 4H), 7.42 (m, 2H), 6.75 (br. d, 1H), 4.88 (m, 1H), 4.62 (m, 2H), 4.05 (m, 3H), 3.70 (d, 1H), 2.47 (s, 3H); ₁₉F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 21.45 min) as the major product (65%ee).

### Example 30

### 4-[5-(3,5-Dichloro-phenyl)-5-trifluoromethyl-4,5-dihydro-isoxazol-3-yl]-2-methyl-N-(1,1-dioxo-thietan-3-yl)-benzamide

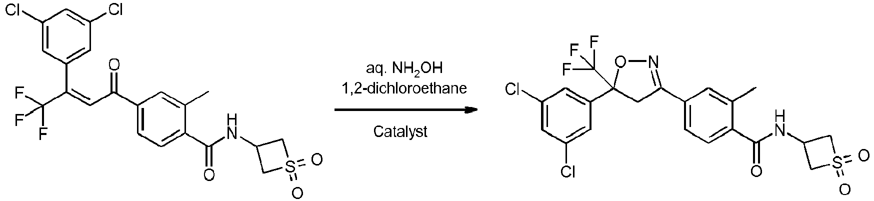

### Catalyst:

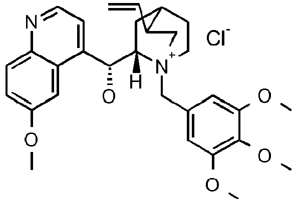

A pre-cooled solution of 5M sodium hydroxide (0.09 mL) was added to a solution of hydroxylamine (50% in water, 0.026 ml) at 5°C (ice bath). The solution was stirred for 15 min at 5°C then added to a vigorously stirred solution of 1, 4-[(E)-3-(3,5-dichlorophenyl)-4,4,4-trifluoro-but-2-enoyl]-2-methyl-N-(*cis*-1-oxide-thietan-3-yl)-benzamide (100 mg) and 3,4,5 -trimethoxyphenyl-methyl quininium chloride (catalyst preparation Example 6) (22 mg) in 1,2-dichloroethane (1.5 ml) cooled in an ice bath. The mixture was stirred rapidly at ca 0°C for 3 hour. The reaction mixture was diluted with dichloromethane, passed through an isolute phase separating cartridge and concentrated *in vacuo* to leave yellow oil. This residue was purified by chromatography on silica gel (eluent: *n*-heptane / ethyl acetate 40%) to give the title compound (62 mg) as a white solid. ¹H NMR (400 MHz, CDCl₃) 7.48 (m, 5H), 7.15 (d, 1H), 4.70 (m, 1H), 4.15 (m, 2H), 4.10 (d, 1H), 3.73 (d, 1H), 3.47 (m, 2H), 2.42 (s, 3H). ¹⁹F NMR (376 MHz, CDCl₃) 79.52 (s, 3F). The product was analyzed by chiral HPLC (method J) and compared to reference samples of ISOMERS A and B of the title compound (chiral separation Example): the product contained ISOMER B (retention time 21.67 min) as the major product (68%ee).

### Example 31

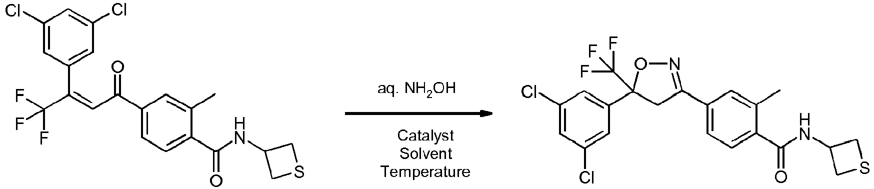

Following the general procedure described in Example 13, different catalysts, solvents and reaction conditions were screened for the preparation of 4-[5-(3,5-dichloro-phenyl)-5-trifluoromethyl-4,5-dihydro-isoxazol-3-yl]-2-methyl-N-(thietan-3-yl)-benzamide. The following results were obtained:

| Example | Catalyst | NH₂OH (eq.) | NaOH (eq.) | Catalyst (mol%) | Solvent | T°C | Yield | ee% |
|---|---|---|---|---|---|---|---|---|
| 31.1 | 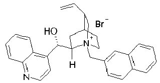 | 5 | 6 | 20mol% | DCE | -15°C | 62% | 5% |
| 31.2 | 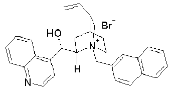 | 5 | 6 | 20mol% | DCE | 0°C | 73% | 6% |
| 31.3 | 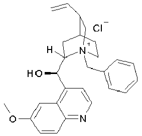 | 5 | 6 | 20mol% | DCE | -30°C | 66% | 20% |
| 31.4 | 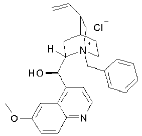 | 2 | 2.2 | 20mol% | DCE | 0°C | 68% | 21% |
| 31.5 | 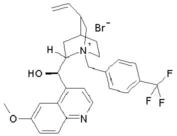 | 5 | 6 | 20mol% | DCE | -25°C | 78% | 15% |
| 31.6 | 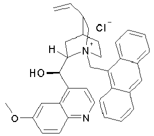 | 5 | 6 | 20mol% | DCE | -25°C | 88% | 40% |
| 31.7 | 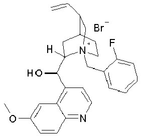 | 5 | 6 | 20mol% | DCE | -25°C | - | 25% |
| 31.8 | 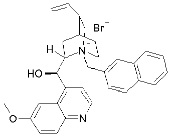 | 5 | 6 | 20mol% | DCE | -25°C | 92% | 20% |
| 31.9 | 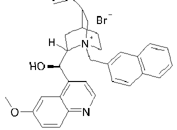 | 5 | 6 | 20mol% | DCE | 0°C | 97% | 20% |
| 31.10 | 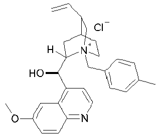 | 5 | 6 | 20mol% | DCE | -25°C | 77% | 20% |
| 31.11 | 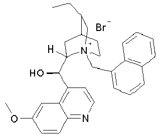 | 5 | 6 | 20mol% | DCE | -25°C | 97% | 20% |
| 31.12 | 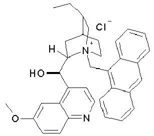 | 5 | 6 | 20mol% | DCE | -25°C | 83% | 40% |
| 31.13 | 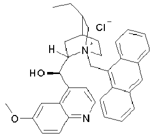 | 2 | 2.2 | 20mol% | DCE | 0°C | 68% | 41% |
| 31.14 | 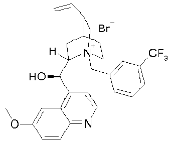 | 5 | 6 | 20mol% | DCE | -15°C | 77% | 23% |
| 31.15 | 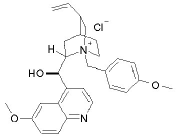 | 5 | 6 | 20mol% | DCE | -15°C | 72% | 27% |
| 31.16 | 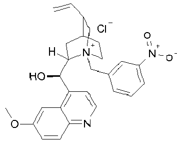 | 5 | 6 | 20mol% | DCE | -15°C | 74% | 15% |
| 31.17 | 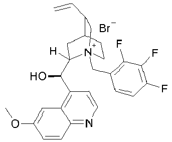 | 5 | 6 | 20mol% | DCE | -15°C | 81% | 30% |
| 31.18 | 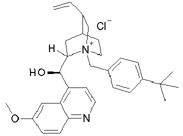 | 5 | 6 | 20mol% | DCE | -15°C | 87% | 20% |
| 31.19 | 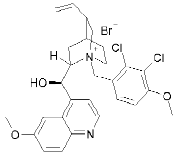 | 5 | 6 | 20mol% | DCE | -15°C | - | 46% |
| 31.20 | 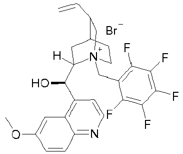 | 5 | 6 | 20mol% | DCE | -15°C | 98% | 63% |
| 31.21 | 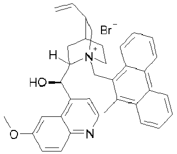 | 5 | 6 | 20mol% | DCE | -15°C | 97% | 30% |
| 31.22 | 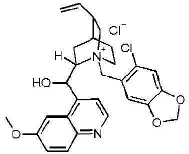 | 5 | 6 | 20mol% | DCE | -15°C | - | 51% |
| 31.23 | 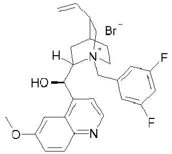 | 5 | 6 | 20mol% | DCE | -15°C | 97% | 38% |
| 31.24 | 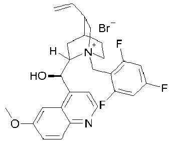 | 5 | 6 | 20mol% | DCE | -15°C | 97% | 49% |
| 31.25 | 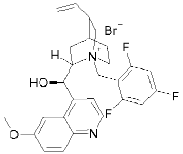 | 2 | 2.2 | 20mol% | DCE | 0°C | 97% | 57% |
| 31.26 | 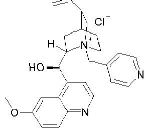 | 5 | 6 | 20mol% | DCE | -15°C | - | 51% |
| 31.27 | 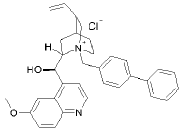 | 5 | 6 | 20mol% | DCE | -15°C | 74% | 25% |
| 31.28 | 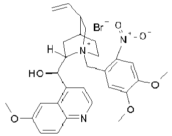 | 5 | 6 | 20mol% | DCE | -15°C | 34% | 44% |
| 31.29 | 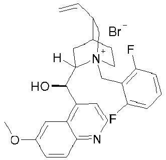 | 5 | 6 | 20mol% | DCE | -15°C | 63% | 47% |
| 31.30 | 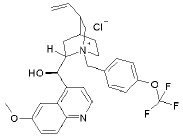 | 5 | 6 | 20mol% | DCE | -15°C | - | 22% |
| 31.31 | 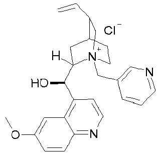 | 5 | 6 | 20mol% | DCE | -15°C | - | 46% |
| 31.32 | 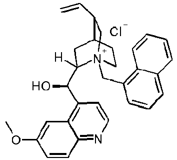 | 5 | 6 | 20mol% | DCE | -15°C | 100% | 33% |
| 31.33 | 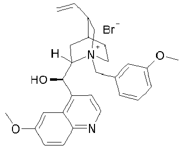 | 5 | 6 | 20mol% | DCE | -15°C | 100% | 33% |
| 31.34 | 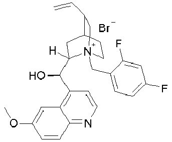 | 5 | 6 | 20mol% | DCE | -15°C | 100% | 37% |
| 31.35 | 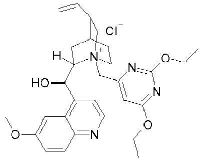 | 5 | 6 | 20mol% | DCE | 0°C | - | 47% |
| 31.36 | 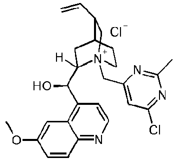 | 5 | 6 | 20mol% | DCE | 0°C | - | 26% |
| 31.37 | 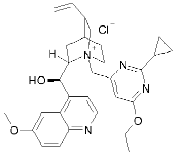 | 5 | 6 | 20mol% | DCE | 0°C | - | 29% |
| 31.38 | 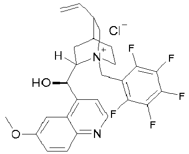 | 5 | 6 | 20mol% | DCE | 0°C | 73% | 75% |
| 31.39 | 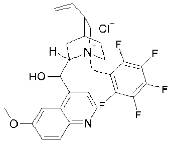 | 2 | 2.2 | 20mol% | DCE | 0°C | 68% | 68% |
| 31.40 | 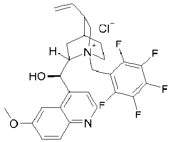 | 5 | 6 | 20mol% | fluorob enzene | 0°C | 78% | 65% |
| 31.41 | 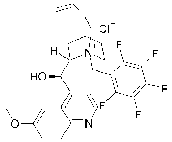 | 5 | 6 | 20mol% | α,α,α-trifluor otoluen e | 0°C | 63% | 46% |
| 31.42 | 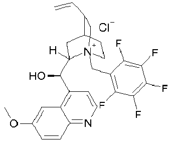 | 5 | 6 | 20mol% | t-butyl-acetate | 0°C | 81% | 62% |
| 31.43 | 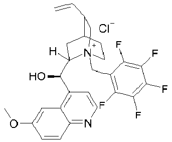 | 5 | 6 | 20mol% | dioxan | 0°C | 64% | 53% |
| 31.44 | 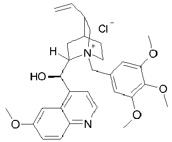 | 5 | 6 | 20mol% | DCE | 0°C | 65% | 77% |
| 31.45 | 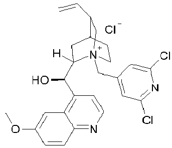 | 5 | 6 | 20mol% | DCE | 0°C | 68% | 66% |
| 31.46 | 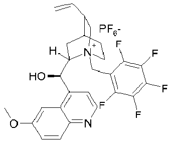 | 5 | 6 | 20mol% | DCE | 0°C | 68% | 60% |
| 31.47 | 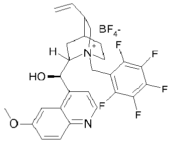 | 5 | 6 | 20mol% | DCE | 0°C | 38% | 83% |
| 31.48 | 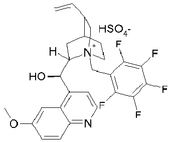 | 5 | 6 | 20mol% | DCE | 0°C | 72% | 58% |
| 31.49 | 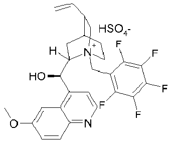 | 5 | 6 | 5mol% | DCE | 0°C | 100% | 59% |
| 31.50 | 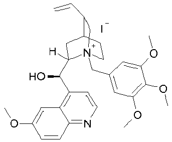 | 5 | 6 | 20mol% | DCE | 0°C | 63% | 77% |
| 31.51 | 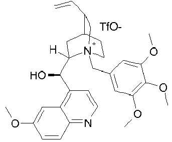 | 5 | 6 | 20mol% | DCE | 0°C | 63% | 77% |
| 31.52 | 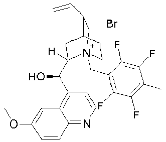 | 5 | 6 | 20mol% | DCE | 0°C | 94% | 51% |

### Example 32

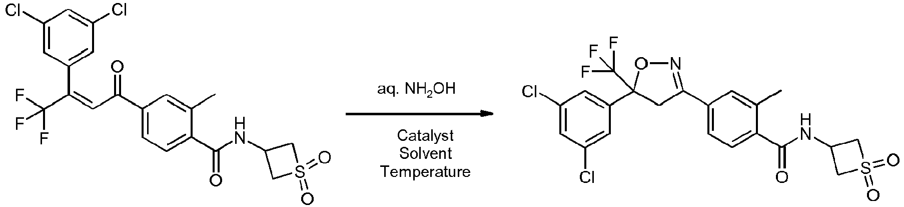

Following the general procedure described in Example 27, different catalysts, solvents and reaction conditions were screened for the preparation of 4-[5-(3,5-dichloro-phenyl)-5-trifluoromethyl-4,5-dihydro-isoxazol-3-yl]-2-methyl-N-(1,1-dioxo-thietan-3-yl)-benzamide. The following results were obtained:

| Example | Catalyst | NH₂OH (eq.) | NaOH (eq.) | Catalyst (mol%) | Solvent | T°C | Yield | ee% |
|---|---|---|---|---|---|---|---|---|
| 32.1 | 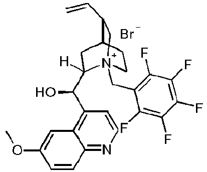 | 2 | 2.2 | 20mol% | DCE | 0°C | 58% | 61% |
| 32.2 | 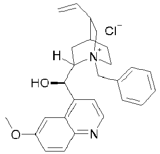 | 2 | 2.2 | 20mol% | DCE | 0°C | 28% | 21% |
| 32.3 | 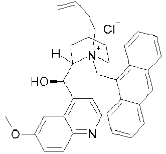 | 2 | 2.2 | 20mol% | DCE | 0°C | 46% | 33% |
| 32.4 | 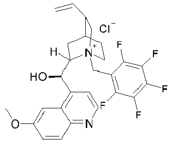 | 2 | 2.2 | 20mol% | DCE | 0°C | 56% | 65% |
| 32.5 | 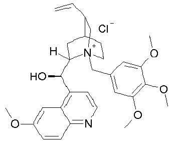 | 2 | 2.2 | 20mol% | DCE | o_{°}c | 60% | 68% |
| 32.6 | 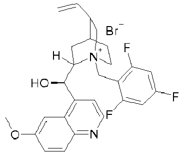 | 2 | 2.2 | 20mot% | DCE | 0°C | 48% | 52% |

### Example 33

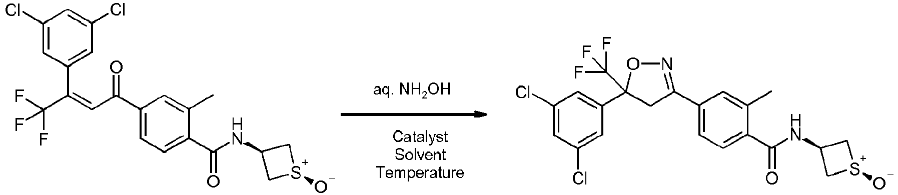

Following the general procedure described in Example 21, different catalysts, solvents and reaction conditions were screened for the preparation of 4-[5-(3,5-dichloro-phenyl)-5-trifluoromethyl-4,5-dihydro-isoxazol-3-yl]-2-methyl-N-(cis-1-oxo-thietan-3-yl)-benzamide. The following results were obtained:

| Example | Catalyst | NH₂OH (eq.) | NaOH (eq.) | Catalyst (mol%) | Solvent | T°C | Yield | ee% |
|---|---|---|---|---|---|---|---|---|
| 33.1 | 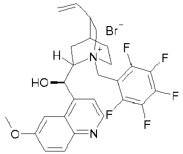 | 2 | 2.2 | 20mol% | DCE | 0°C | 88% | 90% |
| 33.2 | 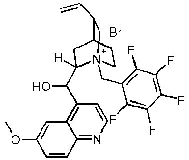 | 2 | 2.2 | 3mol% | DCE | 0°C | 100% | 73% |
| 33.3 | 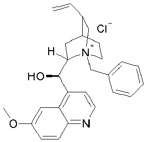 | 2 | 2.2 | 20mol% | DCE | 0°C | 92% | 20% |
| 33.4 | 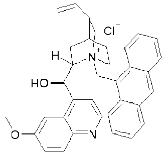 | 2 | 2.2 | 20mol% | DCE | 0°C | 68% | 37% |
| 33.5 | 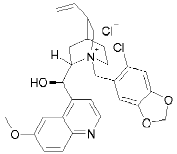 | 2 | 2.2 | 20mol% | DCE | 0°C | 40% | 16% |
| 33.6 | 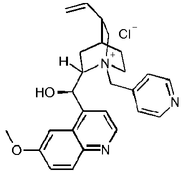 | 2 | 2.2 | 20mol% | DCE | 0°C | 100% | 60% |
| 33.7 | 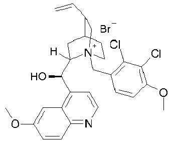 | 2 | 2.2 | 20mol% | DCE | 0°C | 100% | 39% |
| 33.8 | 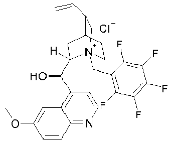 | 2 | 2.2 | 20mol% | DCE | 0°C | 87% | 73% |
| 33.9 | 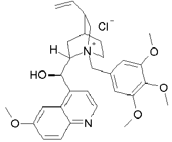 | 2 | 2.2 | 20mol% | DCE | 0°C | 83% | 70% |
| 33.10 | 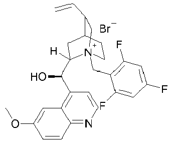 | 2 | 2.2 | 20mol% | DCE | 0°C | 63% | 64% |
| 33.11 | 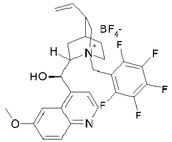 | 5 | 6 | 20mol% | DCE | 0°C | 53% | 50% |
| 33.12 | 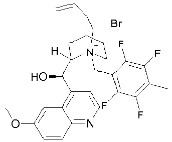 | 2 | 2.2 | 20mol% | DCE | -10°C | 88% | 79.5 % |

### Example 34:

Influence of the E/Z ratio of_(4-[3-(3,5-dichloro-phenyl)-4,4,4-trifluoro-but-2-enoyl]-2-methyl-N-thietan-3-yl-benzamide) on enantiomeric excess

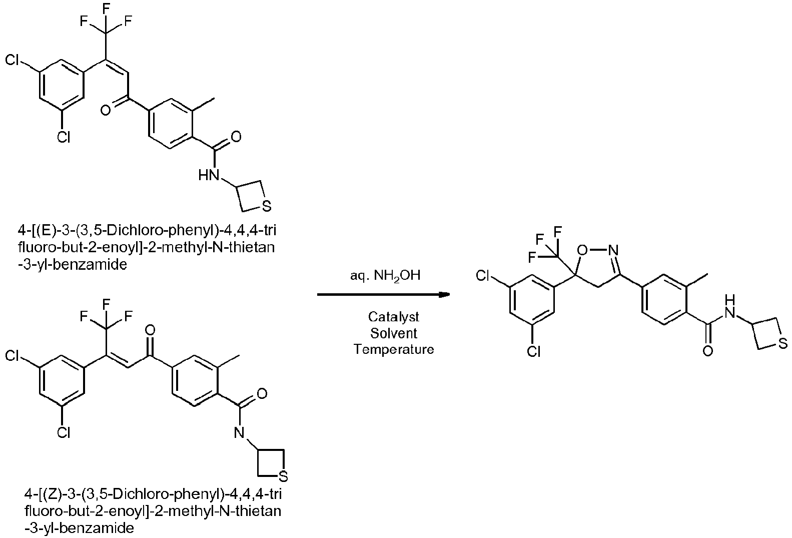

### Catalyst:

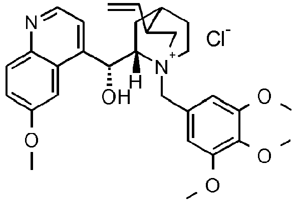

Using the procedure described in Example 10, Step E, different E/Z ratios of the intermediate 4-[3-(3,5-dichloro-phenyl)-4,4,4-trifluoro-but-2-enoyl]-2-methyl-N-thietan-3-yl-benzamide could be obtained by collecting different fractions from column chromatography (heptanes / ethyl acetate 5/1). Thus, from 4-acetyl-2-methyl-N-thietan-3-yl-benzamide (3.97 g), 3.2 g of a 75/25 E/Z mixture of 4-[3-(3,5-dichloro-phenyl)-4,4,4-trifluoro-but-2-enoyl]-2-methyl-N-thietan-3-yl-benzamide was obtained by collecting specific fractions from the chromatography; similarly, from 4-acetyl-2-methyl-N-thietan-3-yl-benzamide (3.97 g), 3.2 g of a 75/25 E/Z mixture of 4-[3-(3,5-dichloro-phenyl)-4,4,4-trifluoro-but-2-enoyl]-2-methyl-N-thietan-3-yl-benzamide were obtained. Similarly, in another experience, from 4-acetyl-2-methyl-N-thietan-3-yl-benzamide (10 g), 3.2 g of a 75/25 E/Z mixture of 4-[3-(3,5-dichloro-phenyl)-4,4,4-trifluoro-but-2-enoyl]-2-methyl-N-thietan-3-yl-benzamide was obtained; similarly, from 4-acetyl-2-methyl-N-thietan-3-yl-benzamide (3.97 g), 16 g of a 91/9 E/Z mixture of 4-[3-(3,5-dichloro-phenyl)-4,4,4-trifluoro-but-2-enoyl]-2-methyl-N-thietan-3-yl-benzamide were obtained by collecting all fractions from the flash chromatography.

A pre-cooled solution of 5M sodium hydroxide (0.093 ml) was added to a solution of hydroxylamine (50% in water, 0.026 mL) at 5°C (ice bath). The solution was stirred for 15 min at 5°C then added to a vigorously stirred solution of an isomeric mixture of 4-[(E)-3-(3,5-dichloro-phenyl)-4,4,4-trifluoro-but-2-enoyl]-2-methyl-N-thietan-3-yl-benzamide and 4-[(Z)-3-(3,5-dichloro-phenyl)-4,4,4-trifluoro-but-2-enoyl]-2-methyl-N-thietan-3-yl-benzamide (100 mg, ratio described in table below) and 3,4,5-trimethoxyphenyl-methyl quininium chloride (21 mg) (catalyst preparation Example 6) in 1,2-dichloroethane (1.5 ml) cooled in an ice bath. The mixture was stirred rapidly at ca 0°C for 2 hours. The reaction mixture was diluted with dichloromethane, passed through an isolute phase separating cartridge and concentrated *in vacuo* to leave yellow oil. This residue was purified by chromatography on silica gel (eluent: heptane / ethyl acetate 20%) to give the title compound. The product was analysed by chiral HPLC (method J) and compared to reference samples of ISOMERS A and B of the title compound (chiral separation Example): the product contained ISOMER B as the major product (enantiomeric excess in table below)

| Example | **E/Z ratio** (4-[3-(3,5-dichloro-phenyl)-4,4,4-trifluoro-but-2-enoyl]-2-methyl-N-thietan-3-yl-benzamide) | Yield | ee% |
|---|---|---|---|
| 34.1 | 75/25 | 85% | 73% |
| 34.2 | 91/9 | 68% | 77% |

### Example 35: Biological examples

This Example illustrates the pesticidal/insecticidal properties of compounds of formula (I). The absolute configuration of isomers A and B has been confirmed by X-ray diffraction. Isomer B corresponds to the compound of formula IB. Tests were performed as follows:

### Spodoptera littoralis (Egyptian cotton leafworm):

Cotton leaf discs were placed on agar in a 24-well microtiter plate and sprayed with test solutions at an application rate of 50 ppm. After drying, the leaf discs were infested with 5 L1 larvae. The samples were checked for mortality, feeding behavior, and growth regulation 3 days after treatment (DAT).

The following results were obtained:

| Compound (obtained as described in Example 9) | ISOMER | Control of *Spodoptera littoralis* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 50% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 50% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Heliothis virescens (Tobacco budworm):

Eggs (0-24 h old) were placed in 24-well microtiter plate on artificial diet and treated with test solutions at an application rate of 50 ppm (concentration in well 18 ppm) by pipetting. After an incubation period of 4 days, samples were checked for egg mortality, larval mortality, and growth regulation.

The following results were obtained:

| Compound (obtained as described in Example 9) | ISOMER | Control of *Heliothis virescens* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 50% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 0% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Plutella xylostella (Diamond back moth):

24-well microtiter plate (MTP) with artificial diet was treated with test solutions at an application rate of 50 ppm (concentration in well 4.5 ppm) by pipetting. After drying, the MTP's were infested with L2 larvae (7-12 per well). After an incubation period of 6 days, samples were checked for larval mortality and growth regulation.

The following results were obtained:

| Compound (obtained as described in Example 9) | ISOMER | Control of *Plutella xylostella* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 0% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 0% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Diabrotica balteata (Corn root worm):

A 24-well microtiter plate (MTP) with artificial diet was treated with test solutions at an application rate of 50 ppm (concentration in well 4.5 ppm) by pipetting. After drying, the MTP's were infested with L2 larvae (6-10 per well). After an incubation period of 5 days, samples were checked for larval mortality and growth regulation.

The following results were obtained:

| Compound (obtained as described in Example 9) | ISOMER | Control of *Diabrotica balteata* |
|---|---|---|
| 4-[5-(3,5-Dichloro-phenyl)-5-trifluoromethyl-4,5-dihydro-isoxazol-3-yl]-2-methyl-N-thictan-3-yl-benzamide | A | 0% |
| 4-[5-(3,5-Dichloro-phenyl)-5-trifluoromethyl-4,5-dihydro-isoxazol-3-yl]-2-methyl-N-thictan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 0% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Thrips tabaci (Onion thrips):

Sunflower leaf discs were placed on agar in a 24-well microtiter plate and sprayed with test solutions at an application rate of 50 ppm. After drying, the leaf discs were infested with an aphid population of mixed ages. After an incubation period of 7 days, samples were checked for mortality.

The following results were obtained:

| Compound (obtained as described in Example 9) | ISOMER | Control of *Thrips tabaci* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 0% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 0% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

### Tetranychus urticae (Two-spotted spider mite):

Bean leaf discs on agar in 24-well microtiter plates were sprayed with test solutions at an application rate of 50 ppm. After drying, the leaf discs are infested with mite populations of mixed ages. 8 days later, discs are checked for egg mortality, larval mortality, and adult mortality.

The following results were obtained:

| Compound (obtained as described in Example 9) | ISOMER | Control of *Tetranychus urticae* |
|---|---|---|
| 4-[5-(3,5-Dichloro-phenyl)-5-trifluoromethyl-4,5-dihydro-isoxazol-3-yl]-2-methyl-N-thietan-3-yl-benzamide | A | 0% |
| 4-[5-(3,5-Dichloro-phenyl)-5-trifluoromethyl-4,5-dihydro-isoxazol-3-yl]-2-methyl-N-thietan-3-yl-benzamide | B | 100% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | A | 0% |
| 4-[5-(3,5-dichloro-phenyl)-5-trifluoromethyl-4,5-dihydro-isoxazol-3-yl]-2-methyl-N-(cis-1-oxide-thietan-3-yl)-benzamide | B | 100% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | A | 80% |
| of 4-[5-(3,5-dichloro-phenyl)-5-trifluoromethyl-4,5-dihydro-isoxazol-3-yl]-2-methyl-N-(1,1-dioxo-thietan-3-yl)-benzamide | B | 100% |

## Claims

1. A process for the preparation of a compound of formula (IB) 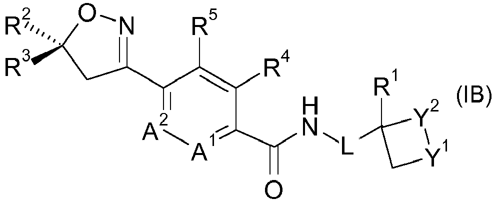 wherein
one of Y¹ and Y² is S, SO or SO₂ and the other is CH₂;
L is a direct bond or methylene;
A¹ and A² are C-H, or one of A¹ and A² is C-H and the other is N;
R¹ is hydrogen or methyl;
R² is chlorodifluoromethyl or trifluoromethyl;
R³ is 3,5-dibromo-phenyl, 3,5-dichloro-phenyl, 3,4-dichloro-phenyl, or 3,4,5-trichloro-phenyl; R⁴ is methyl;
R⁵ is hydrogen;
or R⁴ and R⁵ together form a bridging 1,3-butadiene group;
comprising reacting a compound of formula (II) 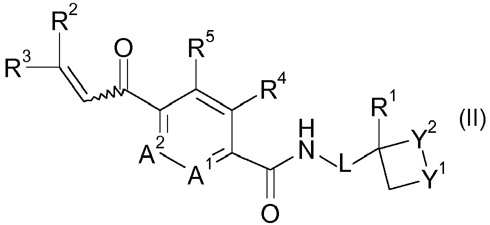 wherein y¹, Y², L, A¹, A², R¹, R², R³, R⁴ and R⁵ are as defined for the compound of formula (IB);
with hydroxylamine in the presence of water, a base and a chiral phase transfer catalyst, which chiral phase transfer catalyst is a quinine derivative of formula (IIIa) 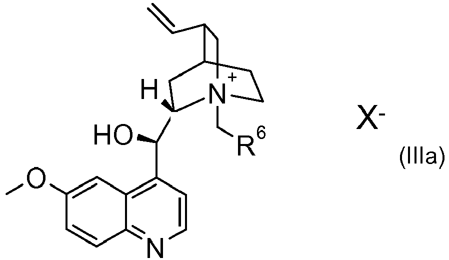 wherein X is an anion;
R⁶ is phenyl substituted by one to five R⁷, pyridyl or pyridyl substituted by one to four R⁷, pyrimidyl or pyrimidinyl substituted by one to three R⁷, or group A 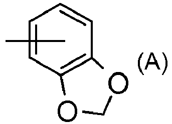 or group A substituted by one to four R⁷, and each R⁷ is independently halogen, nitro, C₁-C₄ alkyl or C₁-C₄ alkoxy.

2. A process according to claim 1, wherein R⁶ is phenyl substituted by one to five substituents independently selected from halogen, methyl and methoxy, pyridyl or pyridyl substituted by one to four halogen atoms.

3. A process according to claim 1, wherein R⁶ is phenyl substituted by three to five substituents independently selected from fluorine, methyl and methoxy, or is pyridyl or pyridyl substituted by two to four halogen atoms.

4. A process according to claim 1, wherein R⁶ is phenyl substituted by three to five substituents independently selected from fluorine, methyl and methoxy.

5. A process according to claim 1, wherein the compound of formula IIIa is a compound of formula IIIB, IIIC, IIIE, IIIF, IIIG, IIIH, IIIJ, IIIK, IIIL, IIIM, IIIN or IIIO 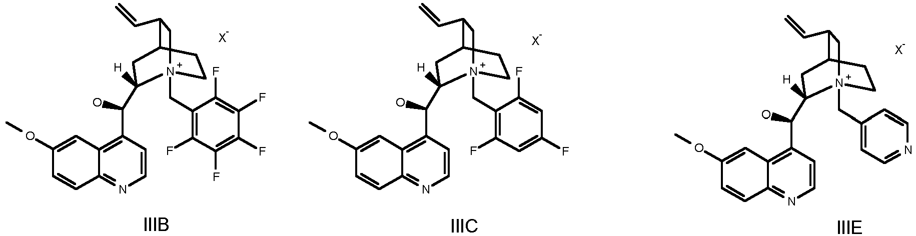 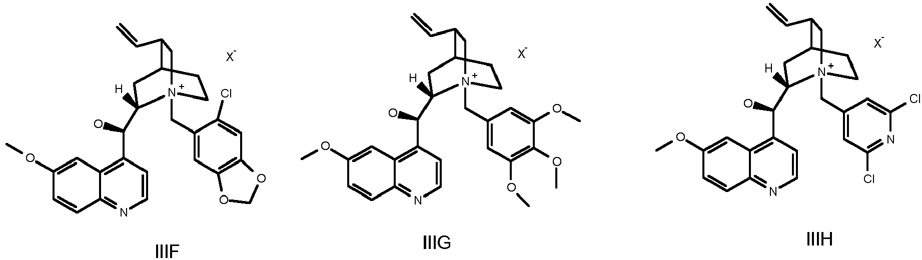 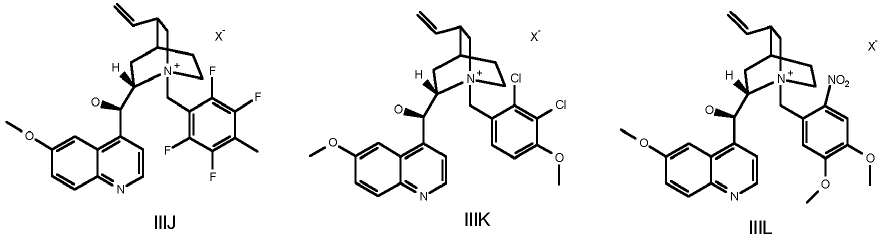 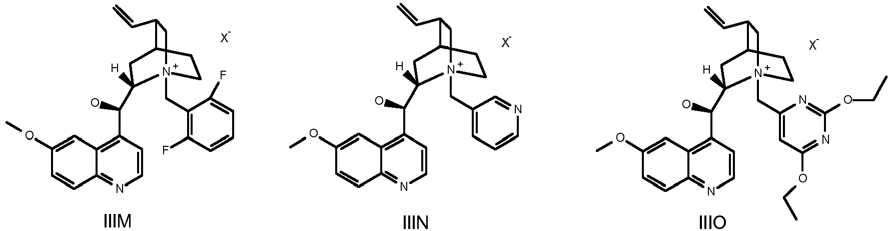 wherein X is an anion.

6. A process according to claim 1, wherein the compound of formula IIIa is a compound of formula IIIB, IIIC, or IIIG.

7. A process according to any one of claims 1 to 6, wherein X is halogen.

8. A process according to any one of claims 1 to 7, wherein when L is a direct bond Y² is CH₂ and Y¹ is S, SO or SO₂, and wherein when L is methylene Y² is S, SO or SO₂ and Y¹ is CH₂.

9. A compound of formula (III*) 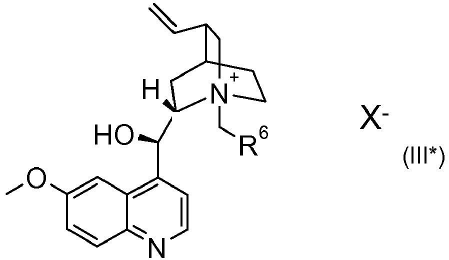 wherein R⁶ is 2,4,6-trifluorophenyl, phenyl substituted by three to five groups independently selected from methyl and fluorine, providing that the phenyl is substituted by at least one methyl and one fluorine; or pyridyl or pyridyl substituted by two to four halogen.

10. A compound according to claim 9, wherein the compound of formula III* is a compound of formula IIIC, IIIE, IIIH, IIIJ, IIIN as defined in claim 5.

## Patentansprüche

1. Verfahren zur Herstellung einer Verbindung der Formel (IB) 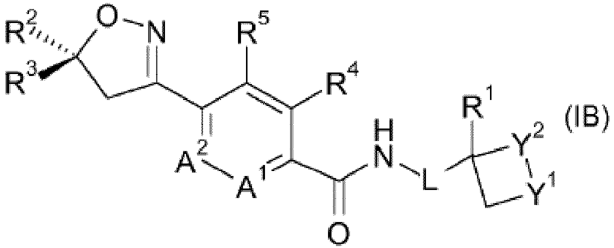 worin
eines von Y¹ und Y² S, SO oder SO₂ bedeutet und das andere CH₂ bedeutet;
L eine direkte Bindung oder Methylen bedeutet;
A¹ und A² C-H bedeuten, oder eines von A¹ und A² C-H bedeutet und das andere N bedeutet;
R¹ Wasserstoff oder Methyl bedeutet;
R² Chlordifluormethyl oder Trifluormethyl bedeutet;
R³ 3,5-Dibromphenyl, 3,5-Dichlorphenyl, 3,4-Dichlorphenyl oder 3,4,5-Trichlorphenyl bedeutet;
R⁴ Methyl bedeutet;
R⁵ Wasserstoff bedeutet;
oder R⁴ und R⁵ gemeinsam eine 1,3-Butadien-Verbrückungsgruppe bilden;
bei dem man eine Verbindung der Formel (II), 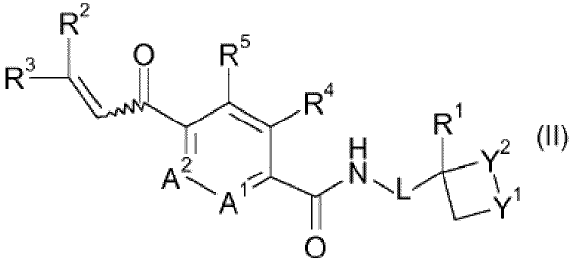 worin Y¹, Y², L, A¹, A², R¹, R², R³, R⁴ und R⁵ wie für die Verbindung der Formel (IB) definiert sind,
in Gegenwart von Wasser, einer Base und einem chiralen Phasentransferkatalysator mit Hydroxylamin umsetzt,
wobei es sich bei dem chiralen Phasentransferkatalysator um ein Chininderivat der Formel (IIIa) handelt, 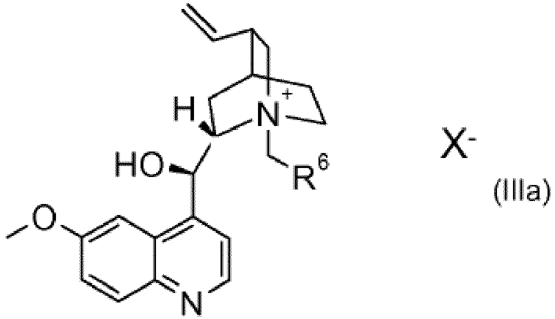 worin X ein Anion bedeutet;
R⁶ Phenyl, substituiert durch ein bis fünf R⁷, Pyridyl oder Pyridyl, substituiert durch ein bis vier R⁷, Pyrimidyl oder Pyrimidinyl, substituiert durch ein bis drei R⁷, oder Gruppe A 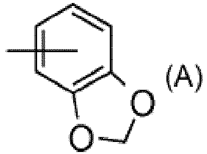 oder Gruppe A, substituiert durch ein bis vier R⁷, und jedes R⁷ unabhängig Halogen, Nitro, C₁-C₄-Alkyl oder C₁-C₄-Alkoxy ist, bedeutet.

2. Verfahren nach Anspruch 1, wobei R⁶ Phenyl, substituiert durch ein bis fünf Substituenten, unabhängig ausgewählt aus Halogen, Methyl und Methoxy, Pyridyl oder Pyridyl, substituiert durch ein bis vier Halogenatome, bedeutet.

3. Verfahren nach Anspruch 1, wobei R⁶ Phenyl, substituiert durch drei bis fünf Substituenten, unabhängig ausgewählt aus Fluor, Methyl und Methoxy, bedeutet, oder Pyridyl oder Pyridyl, substituiert durch zwei bis vier Halogenatome, bedeutet.

4. Verfahren nach Anspruch 1, wobei R⁶ Phenyl, substituiert durch drei bis fünf Substituenten, unabhängig ausgewählt aus Fluor, Methyl und Methoxy, bedeutet.

5. Verfahren nach Anspruch 1, wobei es sich bei der Verbindung der Formel IIIa um eine Verbindung der Formel IIIB, IIIC, IIIE, IIIF, IIIG, IIIH, IIIJ, IIIK, IIIL, IIIM, IIIN oder IIIO 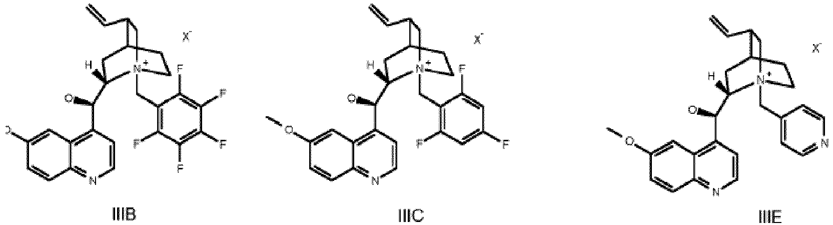 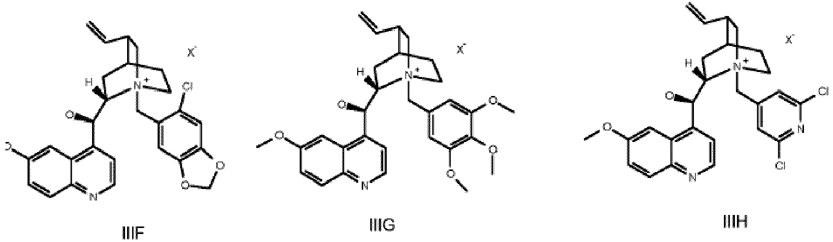 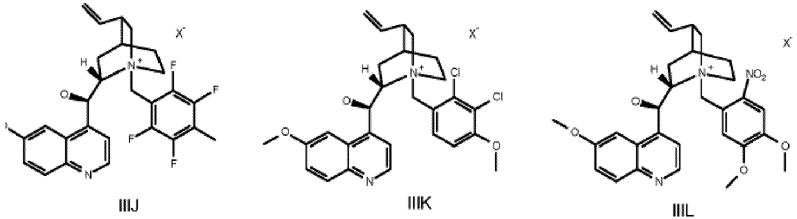 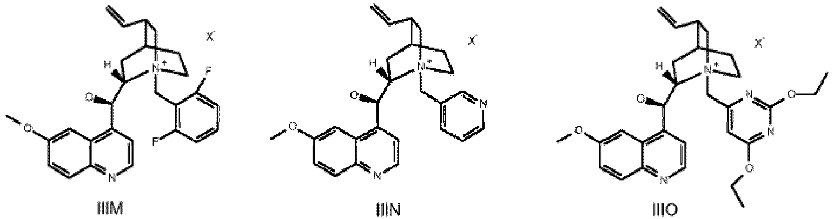 worin X ein Anion bedeutet, handelt.

6. Verfahren nach Anspruch 1, wobei es sich bei der Verbindung der Formel IIIa um eine Verbindung der Formel IIIB, IIIC oder IIIG handelt.

7. Verfahren nach einem der Ansprüche 1 bis 6, wobei X Halogen bedeutet.

8. Verfahren nach einem der Ansprüche 1 bis 7, wobei, wenn L eine direkte Bindung ist, dann Y² CH₂ bedeutet und Y¹ S, SO oder SO₂ bedeutet, und wobei, wenn L Methylen ist, dann Y² S, SO oder SO₂ bedeutet und Y¹ CH₂ bedeutet.

9. Verbindung der Formel (III*) 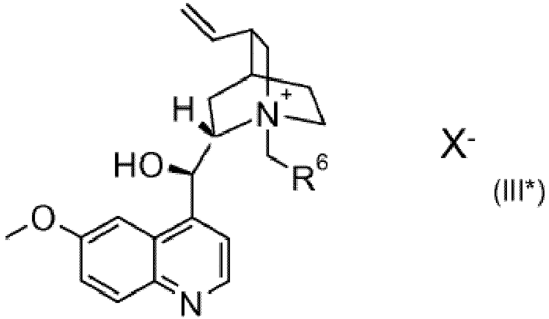 worin R⁶ 2,4,6-Trifluorphenyl, Phenyl, substituiert durch drei bis fünf Gruppen, unabhängig ausgewählt aus Methyl und Fluor, mit der Maßgabe, dass das Phenyl durch mindestens ein Methyl und ein Fluor substituiert ist; oder Pyridyl oder Pyridyl, substituiert durch zwei bis vier Halogen, bedeutet.

10. Verbindung nach Anspruch 9, wobei es sich bei der Verbindung der Formel III* um eine Verbindung der Formel IIIC, IIIE, IIIH, IIIJ, IIIN wie in Anspruch 5 definiert handelt.

## Revendications

1. Procédé pour la préparation d'un composé de formule (IB) 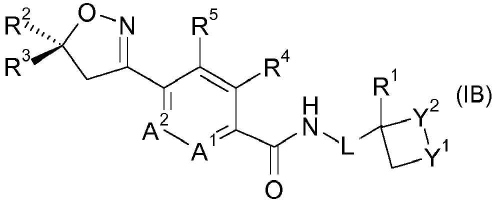 dans laquelle
l'un parmi Y¹ et Y² représente S, SO ou SO₂ et l'autre représente CH₂ ;
L représente une liaison directe ou méthylène ;
A¹ et A² représentent C-H ou l'un parmi A¹ et A² représente C-H et l'autre représente N ;
R¹ représente hydrogène ou méthyle ;
R² représente chlorodifluorométhyle ou trifluorométhyle ;
R³ représente 3,5-dibromophényle, 3,5-dichlorophényle, 3,4-dichlorophényle ou 3,4,5-trichlorophényle ;
R⁴ représente méthyle ;
R⁵ représente hydrogène ; ou
R⁴ et R⁵ forment conjointement un groupe 1,3-butadiène formant un pont ;
comprenant la réaction d'un composé de formule (II) 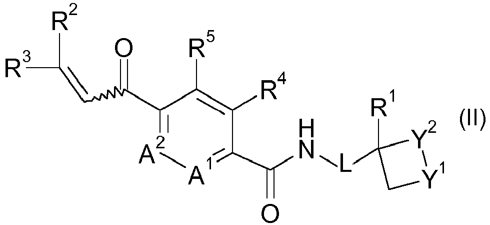 dans laquelle Y¹, Y², L, A¹, A², R¹, R², R³, R⁴ et R⁵ sont tels que définis pour le composé de formule (IB) ;
avec de l'hydroxylamine en présence d'eau, d'une base et d'un catalyseur de transfert à phase chirale, ledit catalyseur de transfert à phase chirale étant un dérivé de quinine de formule (IIIa) 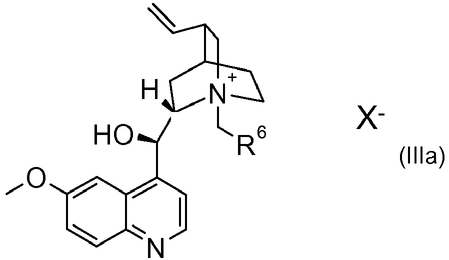 dans laquelle X représente un anion ;
R⁶ représente phényle substitué par un à cinq R⁷, pyridyle ou pyridyle substitué par un à quatre R⁷, pyrimidyle ou pyrimidinyle substitué par un à trois R⁷ ou un groupe A 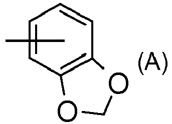 ou un groupe A substitué par un à quatre R⁷, et chaque R⁷ représente, indépendamment, halogène, nitro, C₁-C₄-alkyle ou C₁-C₄-alcoxy.

2. Procédé selon la revendication 1, R⁶ représentant phényle substitué par un à cinq substituants choisis, indépendamment, parmi halogène, méthyle et méthoxy, pyridyle ou pyridyle substitué par un à quatre atomes d'halogène.

3. Procédé selon la revendication 1, R⁶ représentant phényle substitué par trois à cinq substituants choisis, indépendamment, parmi fluor, méthyle et méthoxy ou représente pyridyle ou pyridyle substitué par deux à quatre atomes d'halogène.

4. Procédé selon la revendication 1, R⁶ représentant phényle substitué par trois à cinq substituants choisis, indépendamment, parmi fluor, méthyle et méthoxy.

5. Procédé selon la revendication 1, le composé de formule IIIa étant un composé des formules IIIB, IIIC, IIIE, IIIF, IIIG, IIIH, IIIJ, IIIK, IIIL, IIIM, IIIN ou IIIO 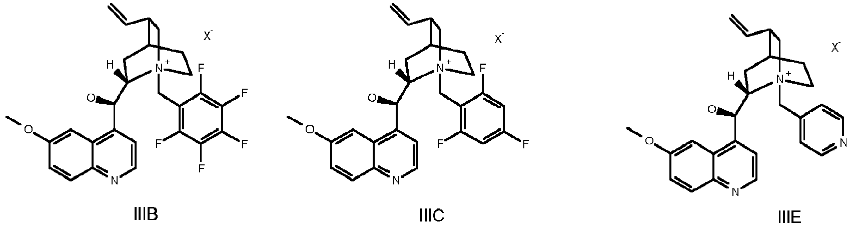 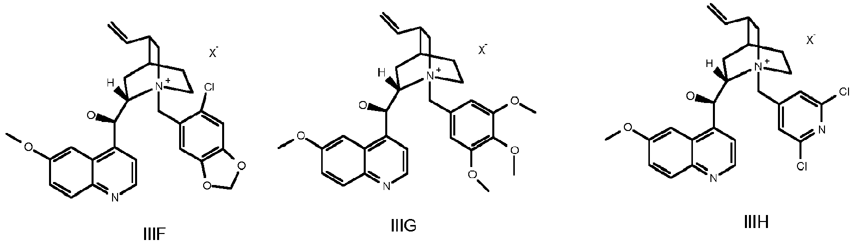 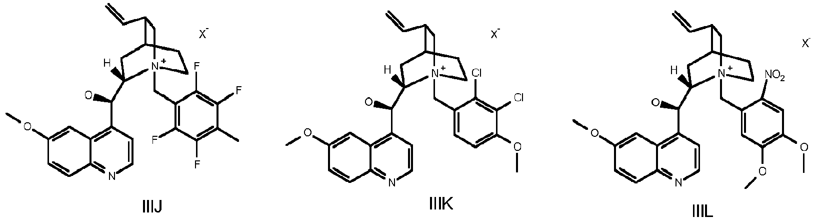 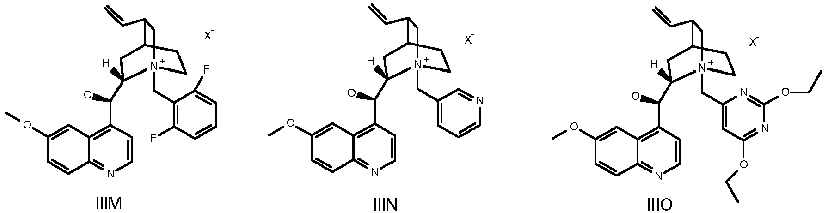 dans lesquelles X représente un anion.

6. Procédé selon la revendication 1, le composé de formule IIIa étant un composé des formules IIIB, IIIC ou IIIG.

7. Procédé selon l'une quelconque des revendications 1 à 6, X représentant halogène.

8. Procédé selon l'une quelconque des revendications 1 à 7, Y² représentant CH₂ et Y¹ représentant S, SO ou SO₂ lorsque L représente une liaison directe et Y² représentant S, SO ou SO₂ et Y¹ représentant CH₂ lorsque L représente méthylène.

9. Composé de formule (III*) 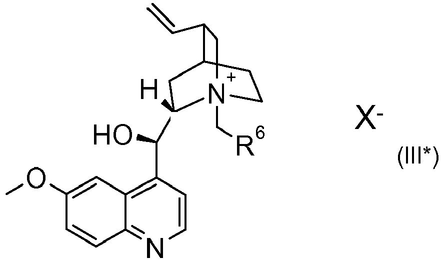 dans laquelle R⁶ représente 2,4,6-trifluorophényle, phényle substitué par trois à cinq groupes choisis, indépendamment, parmi méthyle et fluor, à condition que phényle soit substitué par au moins un méthyle et un fluor ; ou pyridyle ou pyridyle substitué par deux à quatre halogènes.

10. Composé selon la revendication 9, le composé de formule III* étant un composé des formules IIIC, IIIE, IIIH, IIIJ, IIIN telles que définies dans la revendication 5.
